# EUROPEAN PATENT APPLICATION

(11) **EP 1 992 633 A1**
(43) Date of publication of application: **19.11.2008**
(21) Application number: 08001068.9
(22) Date of filing: 02.11.1998
(51) Int. Cl.: C07H 21/00, C07H 21/02, C07H 21/04, A01N 43/04, A61K 31/70

(54) **VEGI, an inhibitor of angiogenesis and tumor growth**

(30) Priority: 03.11.1997 US 963272
(62) Divisional of application: 98958456.0
(71) Applicant: HUMAN GENOME SCIENCES, INC., Rockville, MD 20850 (US); Georgetown University Medical Center, Washington, DC 20007 (US)
(72) Inventor: Li, Lu-Yuan, Gaithersburg, MD 20878 (US); Zhai, Yifan, Rockville, MD 20850 (US); Yu, Guo-Liang, San Mateo, CA 94403 (US); Ni, Jian, Rockville, MD 20853 (US); Lippman, Marc, E., Bethesda, MD 20817 (US); Rosen, Craig, A., Laytonsville, MD 20882 (US)
(74) Representative: Vossius & Partner

(57) **Abstract**

The present invention relates to a vascular endothelial cell inhibitor, VEGI, capable of inhibiting angiogenesis in cellular models and tumourigenesis in animal models, and methods of use.

## Description

### Background of the Invention

Humans or animals undergo angiogenesis, the generation of new blood vessels into a tissue or organ, under normal physiological conditions in very specific restricted situations. For example, angiogenesis is normally observed in wound healing. and embryonal development and formation of the corpus luteum, endometrium and placenta. The term "endothelium" means a thin layer of flat epithelial cells that lines serous cavities, lymph vessels. and blood vessels. The term "anti-angiogenic" or "angiogenic inhibiting activity" means the capability of a molecule to inhibit angiogenesis in general.

Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. The endothelial cells, which line the lumen of blood vessels, then protrude through the basement membrane. Angiogenic stimulants induce the endothelial cells to migrate through the eroded basement membrane. The migrating cells form a "sprout" off the parent blood vessel, where the endothelial cells undergo mitosis and proliferate. The endothelial sprouts merge with each other to form capillary loops, creating the new blood vessel.

Persistent unregulated angiogenesis occurs in a multiplicity of disease states. tumor metastasis and abnormal growth by endothelial cells and supports the pathological damage seen in these conditions. The diverse pathological disease states in which unregulated angiogenesis is present have been grouped together as angiogenic dependent or angiogenic associated diseases.

During tumor growth, endothelial cells proliferate, invade into stroma, migrate toward the source of an angiogenesis stimulus such as cancer cells, interact with perivascular cells and stromal cells, and eventually form capillary vessels linking the tumor tissue to the circulation (J. Folkman (1995) Nat. Med. 1: 27-31). Although the undoubtedly highly complex, mechanism that regulates angiogenesis is yet to be understood, it is becoming clear that the initiation or termination of the process is a result of a balance between positive and negative regulators of angiogenesis. A number of angiogenic factors, often markedly upregulated in tumor tissues, have been described, including several members of the fibroblast growth factor family, such as FGF-1 (G. Gimenez-Gallego et al. (1985) Science 230: 1385), FGF-2 (L. Schweigerer et al. (1987) Nature 325: 257), and those of the vascular endothelial cell growth factor family (VEGF) (D. W. Leung et al. (1989) Science 246: 1306*),* as well as the receptors of these growth factors (L. W. Burrus and B. B. Olwin (1989) J. Biol. Chem. 264:18647; S. Wennstrom et al. (1991) Growth Factors 4: 197; B. Terman et al. (1992) Biochem. Biophys. Res. Comm. 187: 1579; C. de Vries et al, (1992) Science 255: 989). All documents cited herein supra and *infra* are hereby incorporated in their entirety by reference thereto.

Several inhibitors of angiogenesis have also been reported, including thrombospondin *(*D. J. Good et al. (1990) Proc. Natl. Acad. Sci. USA 87:6624), angiostatin (M. S. O'Reilly et al. (1994) Cell 79:315), endostatin (M.S. O'Reilly et al. (1997) Cell 88:277), and platelet factor-4 (E. Maione et al. (199)] Science 247:77). It is apparent that normal angiogenesis is promptly activated when required, and swiftly terminated when no longer needed, whereas pathological angiogenesis, once initiated, is often prolonged and difficult to stop. This indicates that the negative regulation mechanism functioning in a normal angiogenesis process is missing or suppressed in a pathological angiogenesis process. It has been suggested that proteolytic activities that release angiogenesis inhibitors from a number of precursors may account partly for down-regulation of angiogenesis, as indicated by the proteolytic activation of angiostatin from plasminogen and that of endostatin from collagen XVIII (M. S. O'Reilly, (1997) Cell 88:277). Many of the known angiogenesis regulators are pleiotrophic and can act on other cell types as well as the one that produces the regulators, although it is conceivable that endothelial cells may produce autocrine factors to suppress an angiogenesis process or maintain the quiescence of a mature vasculature. No such autocrine regulators of angiogenesis have been previously described. In this application is described a novel autocrine negative regulator of angiogenesis. called Vascular Endothelial Cell Growth Inhibitor (VEGI), specifically expressed by endothelial cells.

Even though the protein described in this application was first described in EP95903521.3, filed November 7, 1994 as TNF-gamma, the angiogenic inhibitory function of the protein was not disclosed therein. The protein was discovered by constructing eight cDNA libraries using RNA derived from various endothelial cells from which 30,000 expressed sequence tags (ESTs) were generated. ESTs unique to endothelial cells were further characterized for sequence homology to known protein families, particularly cytokines. The deduced amino acid sequences of one group of ESTs contained the consensus amino acid sequence of the tumor necrosis factors (TNFs). To obtain the full length cDNA clone, a human umbilical vein endothelial (HUVE) cell library was screened using radio-labeled probe from one of the initial clones. Several positive clones were isolated and sequenced. The longest cDNA clone contained an insert of 4.5 kilobases encoding an open reading frame of 174 amino acids and long untranslated regions at both the 3'- and 5'-ends. An in-frame stop codon upstream of the predicted initiation codon indicated that the translation could not start further upstream. The new protein exhibited 20-30% sequence homology to human TNFα, TNFβ, and the Fas ligand. A protein with a molecular weight of 22 kD was produced in an *in vitro* transcription and translation experiment using the cDNA clone as a template, consistent with the predicted open reading frame. Hydrophobicity analysis of the protein predicted a 12-amino acid hydrophobic region immediately following the N-terminal segment of 14 non-hydrophobic amino acids. This is consistent with the structure of a type II transmembrane protein, similar to TNFs (B. B. Aggarwal and K. Natarajan (1996) Eur. Cytokine News. 7:93*).*

Recent Northern analysis of total RNA preparations from 22 different types of cultured cells of various lineages indicated that transcripts for this protein can only be detected in two lines of endothelial cells: HUVE cells and human venous endothelial cells of an early passage. The mRNA was not detected in human venous endothelial cells of a later passage, nor was it seen in human artery cells. In sharp contrast. the TNF family members are mostly expressed in immune cells (B. B. Aggarwal and K. Natarajan (1996), supra). For instance, TNFα is produced by macrophages, monocytes, neutrophils, and T cells, while TNFβ is predominantly produced by mitogen-stimulated T lymphocytes and leukocytes. Similarly, the ligands for Fas and other TNF family members, CD27, CD30, CD40, OX40, and 4-1 BB are all expressed in cell types in the immune system. Using multiple tissue Northern blots, the VEGI transcript was found to be expressed in placenta, lung, kidney, skeletal muscle, brain, liver, thymus, testis, ovary and peripheral blood lymphocytes.

The present invention identifies and describes the novel function of this protein. A truncated form of the protein described above has been found to inhibit endothelial cell growth. The protein is therefore named Vascular Endothelial Cell Growth Inhibitor (VEGI). A truncated version of VEGI consisting of residues 39-174 corresponding to the putative extracellular domain was expressed in E. *coli.* isolated, and examined in a variety of cellular assays. The truncated form of VEGI was found to specifically inhibit the proliferation of endothelial cells, to inhibit the formation of capillary-like tubes in an *in vitro* model, and to inhibit the growth of human breast cancer xenograft in mammary pads of female athymic nude mice.

### Summary of the Invention

The present invention relates to an inhibitor of endothelial cell proliferation in general and an inhibitor of angiogenesis in particular, and methods of use. The complete nucleotide sequence of VEGI is shown in SEQ ID NO:1, SEQ ID NO:8, SEQ ID NO:26. and the encoded amino acid sequence in shown in SEQ ID NO:2, SEQ ID NO:9, and SEQ ID NO:27, respectively. A truncated form of the protein consisting of residues 39-174 (SEQ ID NO:3) corresponding to the putative extracellular domain was found to specifically inhibit the proliferation of endothelial cells. VEGI was found to inhibit the formation of capillary-like tubes in an *in vitro* angiogenesis model. Finally, VEGI was found to inhibit the growth of xenograft tumors in athymic nude mice.

In another embodiment, the present invention provides an inhibitor of angiogenesis said inhibitor comprising VEGI polynucleotides, a polypeptides of the invention or a modified form of VEGI in a pharmaceutically acceptable diluent, in a pharmaceutically acceptable amount.

In another embodiment, the present invention provides an accelerator of angiogenesis, said accelerator comprising an antibody, an antisense oligonucleotide, an antagonist, a ribozyme, drug or agent which reduces or eliminates VEGI function in a pharmaceutically acceptable diluent, in a pharmaceutically acceptable amount.

In another embodiment, the present invention provides a method for inhibiting angiogenesis which comprises administering to a human or animal a composition comprising a substantially purified VEGI polynucleotides, a polypeptides of the invention or a modified form of VEGI in a dosage sufficient to inhibit angiogenesis.

In another embodiment, the present invention provides a method for accelerating angiogenesis which comprises administering to a human or animal a composition comprising an antibody, an antisense oligonucleotide, an antagonist, a ribozyme, a drug, or agent which reduces or eliminates VEGI function.

In yet another embodiment, the present invention provides a method for the diagnosis of pathological angiogenesis comprising detecting the presence or absence of VEGI polypeptides in a sample said method comprising the steps of:
(i) contacting a sample from an individual suspected of having pathological angiogenesis with antibodies that are specific for VEGI polypeptides of the invention; and
(ii) detecting the presence or absence of a complex formed between VEGI and the antibodies.

In yet another embodiment, the present invention provides a method for the diagnosis of pathological angiogenesis comprising detecting the presence or absence of VEGI polynucleotides (preferably RNA) in a sample said method comprising the steps of:
(i) contacting a sample from an individual suspected of having pathological angiogenesis with oligonucleotides that specifically bind VEGI polynucleotides (e.g., RNA); and
(ii) detecting the presence or absence of a duplex formed between VEG polynucleotides and oligonucleotides specific therefor.

In another embodiment, the present invention provides a method for the diagnosis of pathological angiogenesis using the polymerase chain reaction, said method comprising designing primers using the nucleotide sequence of VEGI as shown in SEQ ID NO:1, SEQ ID NO:8, and SEQ ID NO:26, which specifically amplify a region of VEGI. The primers can be used to amplify VEGI DNA or VEGI RNA, the latter after converting the RNA into complementary DNA (cDNA) by reverse transcription of the RNA. The PCR assay can be made quantitative by comparing the amplified product to a standard which can be generated using methods known in the art.

In yet another embodiment, the present invention provides a method for the detection of VEGI polynucleotides in a sample which comprises assaying for the presence or absence of VEGI RNA or DNA in a sample by hybridization assays.

In a further embodiment, the present invention provides a diagnostic or prognostic kit comprising antibodies that respectfully bind against VEGI polynucleotides or polypeptides of the invention, oligonucleotides which hybridize to VEGI DNA or RNA, and/or PCR primers for amplification of VEGI DNA or RNA, and ancillary reagents suitable for use in detecting the presence of VEGI in a sample. Since VEGI may function as a membrane protein, a naturally existing soluble form of membrane-bound VEGI may function as its antagonist and methods for detecting said soluble form are included in another embodiment of the present invention.

In yet another embodiment, the present invention provides a diagnostic assay comprising detecting the presence of a mutation in VEGI polynucleotides which results in the decrease or increase of VEGI expression or function. Such an assay would include hybridization assay, restriction map polymorphism assays, and gene sequencing, to name a few.

In yet another embodiment, the present invention provides a therapeutic method and composition for the treatment or amelioration of diseases and processes that are mediated by angiogenesis including, but not limited to, hemangioma, solid tumors, leukemia, metastasis, telangiectasia, psoriasis, scleroderma, pyogenic granuloma, myocardial angiogenesis, plagie neovascularization. coronary collaterals, ischemic limb angiogenesis, corneal diseases, rubeosis, neovascular glaucoma, diabetic retinopathy retrolental fibroplasia. arthritis, and diabetic neovascularization; wherein angiogenesis is uncontrolled or excessive and requires inhibition, said method comprising providing to an individual in need of such treatment an effective amount of VEGI polynucleotides or polypeptides of the invention such that angiogenesis is inhibited.

In yet another embodiment, the present invention provides a therapeutic method and composition for the treatment or amelioration of diseases such as macular degeneration, wound healing, peptic ulcer, fractures, keloids, vasculogenesis, hematopoiesis, ovulation, menstruation, and placentation, in which angiogenesis is desired, said method comprising administering to an individual in need of such treatment, an antagonist of VEGI polynucleotides or polypeptides of the invention, antisense oligonucleotides specific for VEGI polynucleotides, or anti-VEGI antibodies, agents, or drugs which reduce or eliminate VEGI function in a pharmaceutically acceptable diluent, in a pharmaceutically acceptable amount.

In another embodiment, the present invention provides a repressor or inhibitor of cancer growth composition comprising substantially purified VEGI polynucleotides or polypeptides of the invention.

In another embodiment, the present invention provides a method for detection and prognosis of cancer by detecting the presence or absence of VEGI polynucleotides or polypeptides of the invention in a sample, or the detection of a mutation in VEGI polynucleotides which alters the expression or function of VEGI.

In yet another embodiment, the present invention provides a method for testing possible agents or drugs for angiogenic inhibitory activity by testing whether or not the drug or agent is capable of upregulating VEGI expression. Since VEGI, like other angiogenic inhibitors. is probably activated by proteases which release the protein from the cell membrane, proteases, as well as other agents that facilitate such activation such as metal ions, would be useful as agents for increasing the expression of VEGI.

In another embodiment, the present invention provides a method for testing possible antitumor agents or drugs by testing whether or not the drug or agent is capable of inhibiting angiogenesis by upregulating VEGI expression.

In still another embodiment, the present invention provides a method for testing possible drugs or agent which promote angiogenesis by testing whether or not the agent or drug can block VEGI function (e.g., inhibition of angiogensis). In this case, inhibition of proteases which activate VEGI as discussed above or agents required for, or agents which facilitate such activation such as metal ions, can be used to down-regulate VEGI. thereby upregulating angiogenesis.

### Brief Description of the Figures

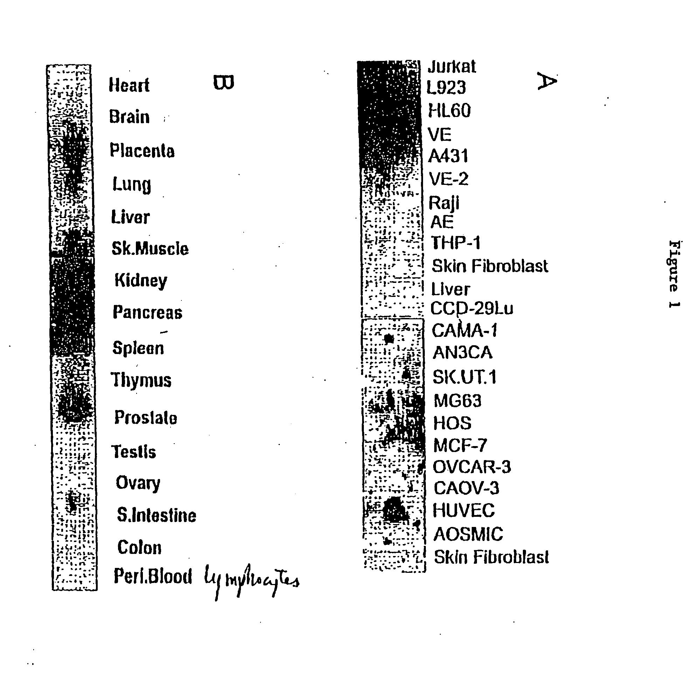

**Figure 1** represents a northern blotting analysis of VEGI transcripts. Panel A, VEGI expression in human cells: Jurkat, human T cell leukemia cell; L923, human embryonic kidney cell; HL60, human promyelocytic leukemia; V.E, human venous endothelial cell (10th passage); A431, human epide rmoid carcinoma; V.E.-2, human venous endothelial cell (20th passage); Raji, human Burkitt's lymphoma; A.E; human artery endothelial cell; THP-1, human monocytic leukemia; CCD-29Lu, human lung emphysema; CAMA1, breast cancer; AN3CA, uterine cancer; SK.UT.1, uterine cancer; MG63, osteoblastoma; HOS, osteoblastoma; MCF7, breast cancer; OVCAR-3, ovarian cancer; CAOV-3. ovarian cancer; HUVEC, human umbilical vein endothelial cell; AOSMIC, smooth muscle. The estimated message size is 6.5 kb. Panel B, VEGI expression in various types of human tissues: Three separate blots were carried out. Positive results from any of the three experiments are shown.

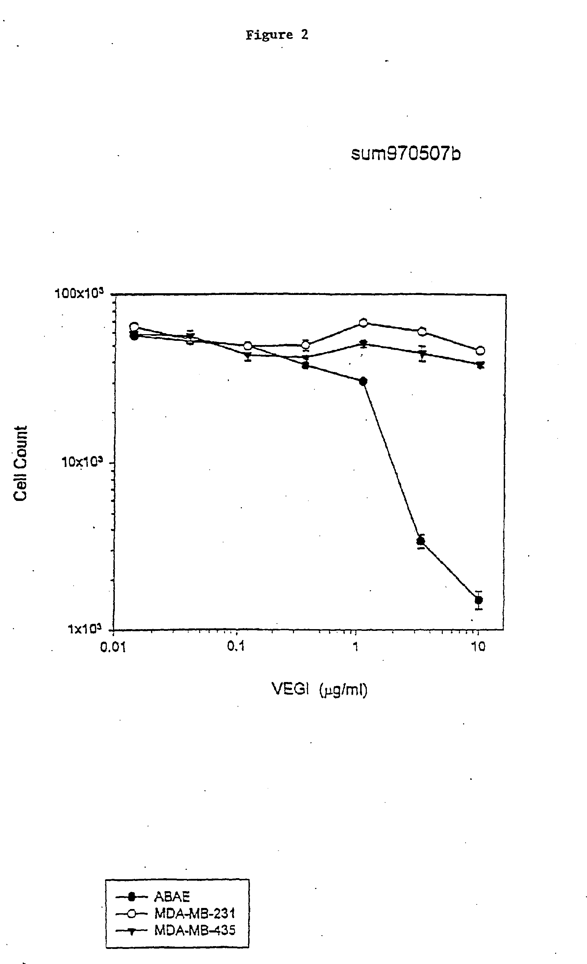

**Figure 2** demonstrates effect of VEGI on the proliferation of endothelial cell and breast cancer cells. The number of cells are plotted against VEGI concentration as indicated. Inhibition of the growth of ABAE cells (open circles) but not that of MDA-MB231 (triangles) or MDA-MB-435 (squares) cells, is shown.

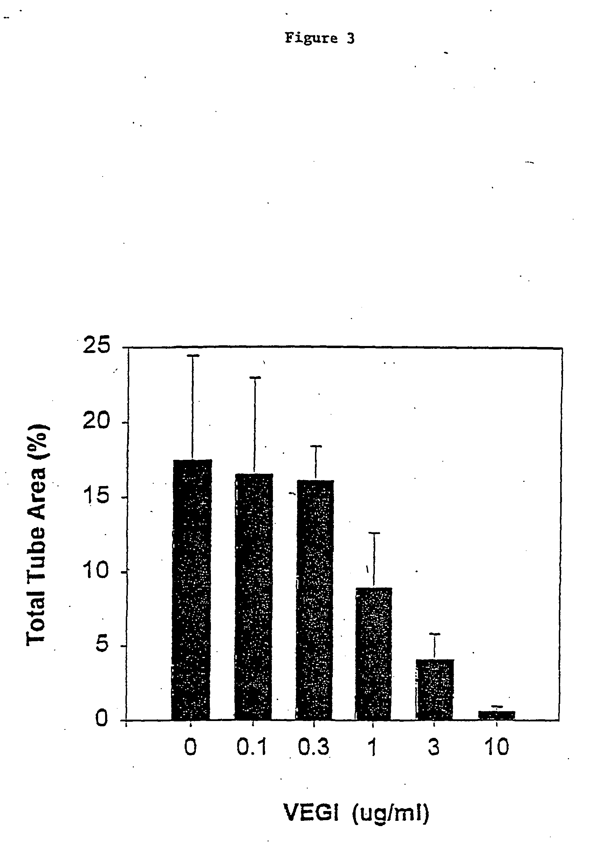

**Figure 3** demonstrates the ability of VEGI to inhibit the formation of capillary- like tubes by ABAE cells on collagen gels is shown. The p-values (t-test) given above the columns are obtained by comparing the extent of the capillary-like tube formation by ABAE cells in the presence of various concentrations of VEGI, as indicated, to that when VEGI is absent from the culture media. The abundance of the capillary-like structures are measured as percentages of the white areas over the total areas measured.

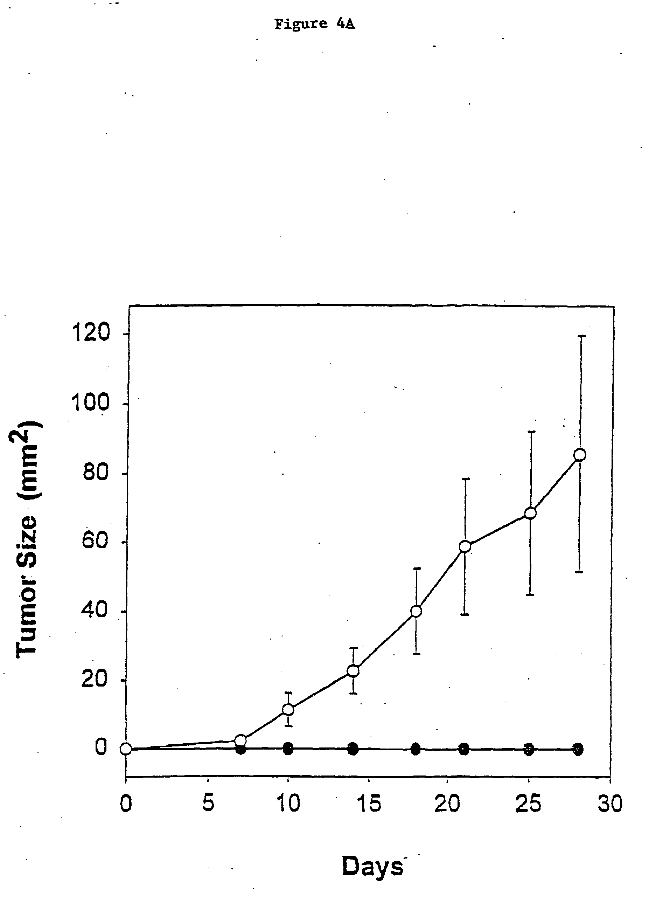

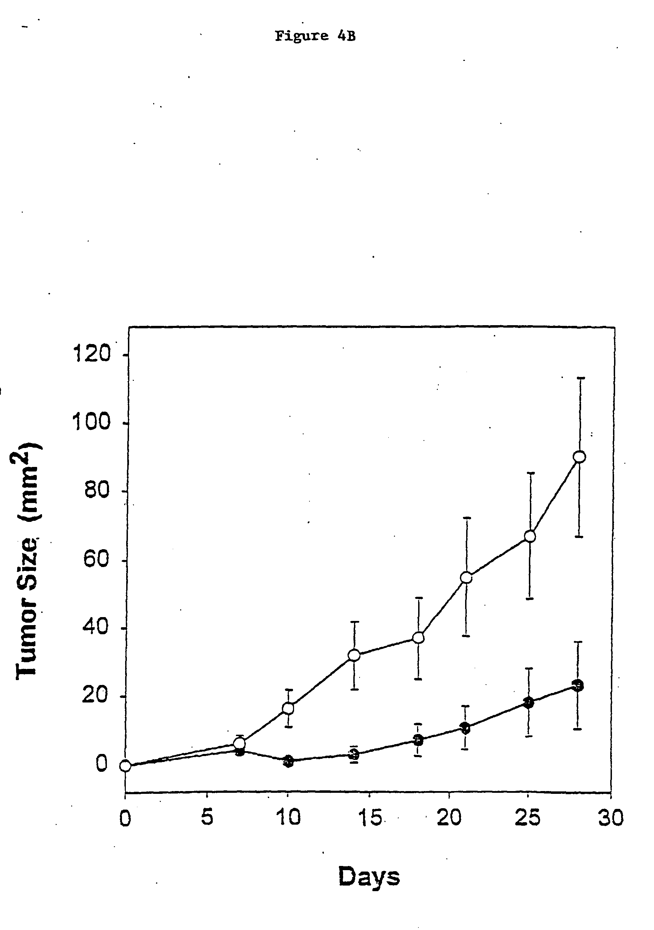

**Figure 4** demonstrates the inhibition of growth of human breast cancer xenograft tumors in athymic nude mice by VEGI. Panel A: Plot of the sizes of the MDA-MB-231 xenograft tumors (mm²) as a function of time post- inoculation (days). Panel B: Plot of the sizes of the im-DA-MB-435 xenograft tumors (mm²) as a function of time post-inoculation (days). Open circles. co-inoculated with vector-transfected CHO cells. Closed circles, co-inoculated with secreted VEGI transfected CHO cells.

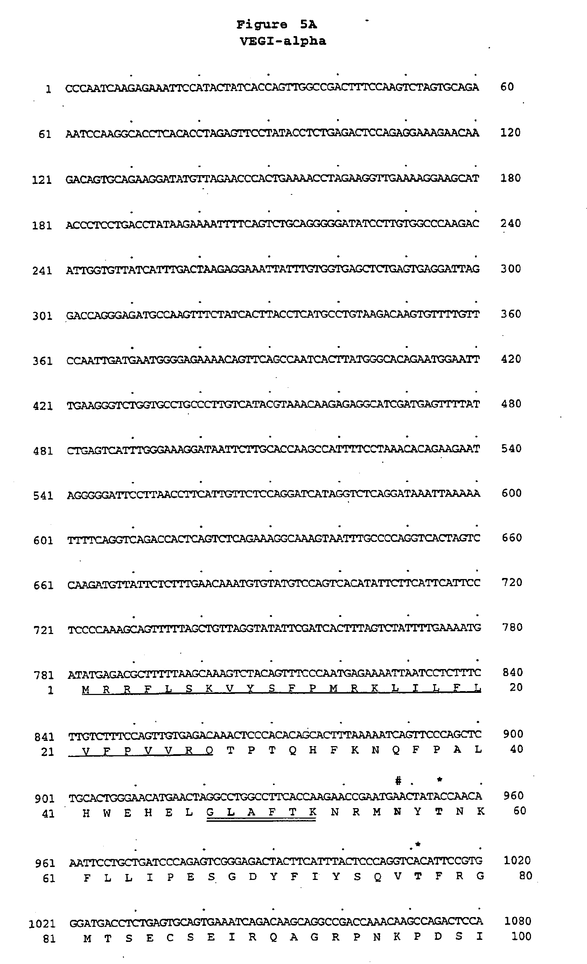

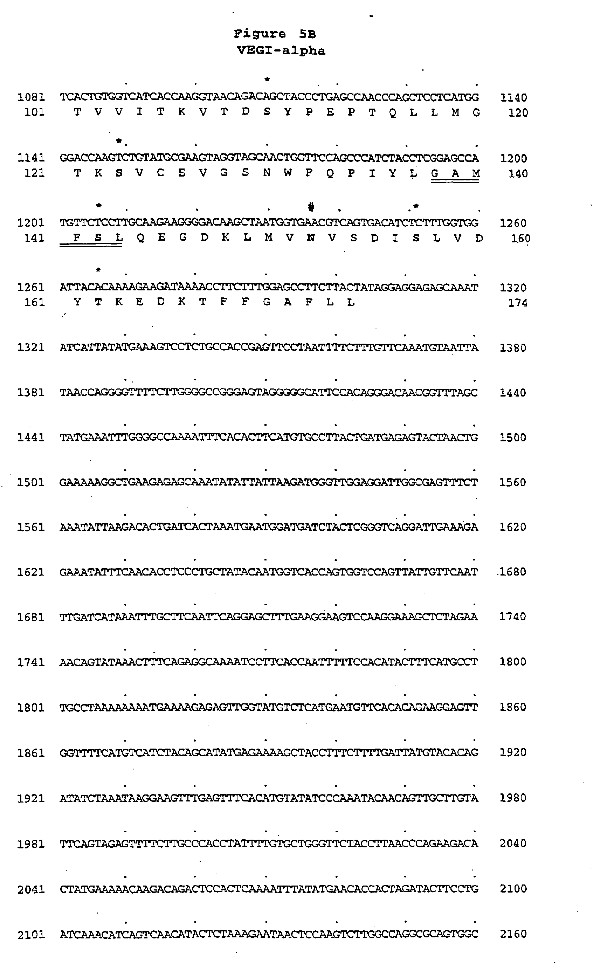

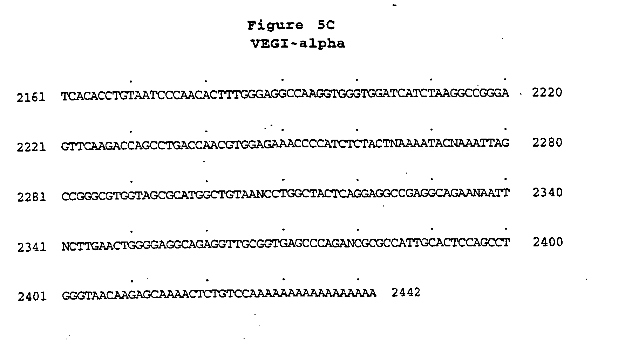

**Figures 5A****,** **5B****, and** **5C** illustrate the cDNA (SEQ ID NO:8) and corresponding deduced amino acid sequence (SEQ ID NO:9) of the polypeptide of VEGI-alpha of the present invention. The initial 27 amino acids (underlined) are the putative leader sequence. The standard one-letter abbreviations for amino acids are used. Potential asparagine-linked , glycosylation sites are marked in Figures 5A, 5B, and 5C with a bolded asparagine symbol (N) in the VEGI-alpha amino acid sequence and a bolded pound sign (#) above the first nucleotide encoding that asparagine residue in the VEGI-alpha nucleotide sequence. Potential N-linked glycosylation sequences are found at the following locations in the VEGI-alpha amino acid sequence: N-29 through N-32 (N-29, Y-30, T-31, N-32) and N-125 through D-128 (N-125, V-126, S-127, D-128). Potential Protein Kinase C (PKC) phosphorylation sites are also marked in Figures 5A, 5B, and 5C with a bolded threonine symbol (T) in the VEGI-alpha amino acid sequence and an asterisk (*) above the first nucleotide encoding that threonine residue in the VEGI-alpha nucleotide sequence. Potential PKC phosphorylation sequences are found at the following locations in the VEGI-alpha amino acid sequence: T-32 through K-34 (T-32, N-33, K-34) and T-50 through R-52 (T-50, F-51, R-52). Potential Casein Kinase II (CK2) phosphorylation sites are also marked in Figures 5A, 5B, and 5C with a bolded serine or threonine symbol (S or T) in the VEGI-alpha amino acid sequence and an asterisk (*) above the first nucleotide encoding the appropriate serine or threonine residue in the VEGI-alpha nucleotide sequence. Potential CK2 phosphorylation sequences are found at the following locations in the VEGI-alpha amino acid sequence: S-83 through E-86 (S-83, Y-84, P-85, E-86); S-96 through E-99 (S-96, V-97, C-98, E-99); S-115 through E-118 (S-115, L-116, Q-117, E-118); S-130 through D-133 (S-130, L-131, V-132, D-133); and T-135 through D-138 (T-135, K-136, E-137, D-138). Potential myristylation sites are also marked in Figures 5A, 5B, and 5C with a double underline in the VEGI-alpha amino acid sequence. Potential myristylation sequences are found at the following locations in the VEGI-alpha amino acid sequence: G-20 through K-25 (G-20, L-21, A-22, F-23, T-24, K-25) and G-111 through L-116 (G-111, A-112, M-113,F-114,S-115,L-116).

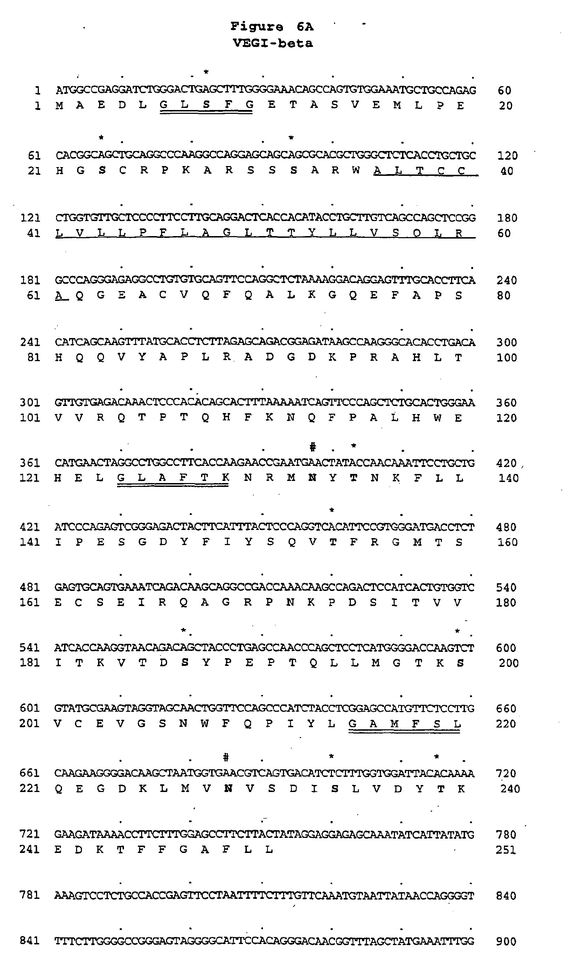

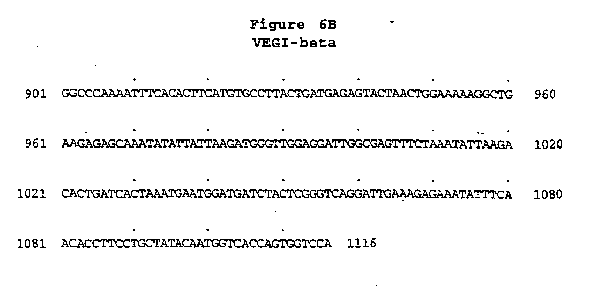

**Figures 6A** **and** **6B** illustrate the cDNA (SEQ ID NO:26) and corresponding deduced amino acid sequence (SEQ ID NO:27) of the polypeptide of the VEGI-beta of the present invention. The standard one-letter abbreviations for amino acids are used. Amino acids methionine-1 to tryptophan-35 are the predicted intracellular domain. Amino acid residues alanine-36 through alanine-61 (underlined) are the putative transmembrane sequence. Amino acid residues glutamine-62 through leucine-251 (underlined) are the putative transmembrane sequence. Potential asparagine-linked glycosylation sites are marked in Figures 6A and 6B with a bolded asparagine symbol (N) in the VEGI-beta amino acid sequence and a bolded pound sign (#) above the first nucleotide encoding that asparagine residue in the VEGI-beta nucleotide sequence. Potential N-linked glycosylation sequences are found at the following locations in the VEGI-beta amino acid sequence: N-133 through N-136 (N-133, Y-134, T-135, N-136) and N-229 through D-232 (N-229, V-230, S-231, D-232). Potential Protein Kinase C (PKC) phosphorylation sites are also marked in Figures 6A and 6B with a bolded serine or threonine symbol (S or T) in the VEGI-beta amino acid sequence and an asterisk (*) above the first nucleotide encoding that threonine residue in the VEGI-beta nucleotide sequence. Potential PKC phosphorylation sequences are found at the following locations in the VEGI-beta amino acid sequence: S-23 through R-25 (S-23, C-24, R-25); S-32 through R-34 (S-32, A-33, R-34); T-135 through K-137 (T-135, N-136, K-137); and T-154 through R-156 (T-154, F-155, R-156). Potential Casein Kinase II (CK2) phosphorylation sites are also marked in Figures 6A and 6B with a bolded serine or threonine symbol (S or T) in the VEGI-beta amino acid sequence and an asterisk (*) above the first nucleotide encoding the appropriate serine or threonine residue in the VEGI-beta nucleotide sequence. Potential CK2 phosphorylation sequences are found at the following locations in the VEGI-beta amino acid sequence: S-8 through E-11 (S-8, F-9, G-10, E-11); S-187 through E-190 (S-187, Y-188, P-189, E-190); S-200 through E-203 (S-200, V-201, C-202, E-203); S-219 through E-222 (S-219, L-220, Q-221, E-222); S-234 through D-237 (S-234, L-235, V-236, D-237); and T-239 through D-242 (T-239, K-240, E-241, D-242). Potential myristylation sites are also marked in Figures 6A and 6B with a double underline in the VEGI-beta amino acid sequence. Potential myristylation sequences are found at the following locations in the VEGI-beta amino acid sequence: G-6 through E-11 (G-6, L-7, S-8, F-9, G-10, E-11); G-124 through G-129 (G-124, L-125, A-126, F-127, T-128, K-129); and G-215 through L-220 (G-215, A-216, M-217, F-218, S-219, L-220).

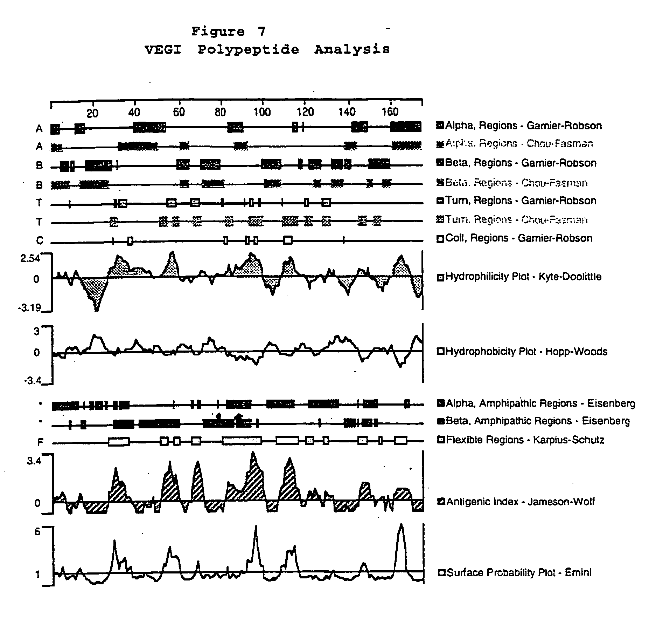

**Figure 7** shows an analysis of the VEGI-alpha amino acid sequence (SEQ ID NO:9). Alpha, beta, turn and coil regions; hydrophilicity and hydrophobicity; amphipathic regions; flexible regions; antigenic index and surface probability are shown, as predicted using the default parameters of the recited computer programs. In the "Antigenic Index or Jameson-Wolf' graph, the positive peaks indicate locations of the highly antigenic regions of the VEGI polypeptide, i.e., regions from which epitope-bearing peptides of the invention can be obtained.

### Detailed Description

Even though the DNA sequence and amino acid sequence of the protein of the present invention was described in EP95903521.3 as TNF-gamma the novel anti-angiogenic activity and the novel endothelial cell inhibitory activity of this molecule were not disclosed.

Therefore, in one embodiment, the present invention relates to VEGI polynucleotides and polypeptides, which inhibit vascular endothelial cell growth and methods for the treatment of diseases and processes that are mediated by or associated with angiogenesis via administering these polynucleotides and polypeptides. The VEGI polynucleotides or polypeptides of the invention, can be isolated from body fluids including, but not limited to, serum, urine and ascites, or synthesized by chemical or biological methods (e.g. cell culture, recombinant gene expression). Recombinant techniques include gene amplification from DNA sources using the polymerase chain reaction (PCR), and gene amplification from RNA sources using reverse transcriptase/PCR. VEGI inhibits the growth of blood vessels into tissues such as unvascularized or vascularized tumors. The present invention includes a protein that has a molecular weight of approximately 22 kD and any modified form thereof of the protein, including, but not limited to, a truncation or a post-translational modification such as a glycosylated form of the protein that is capable of overcoming the angiogenic activity of endogenous growth factors. The nucleotide sequence for VEGI and shown in SEQ ID NO:1, SEQ ID NO:8, and SEQ ID NO:26 and amino acid sequence for VEGI are shown in SEQ ID NO:2, SEQ ID NO:9, and SEQ ID NO:27, respectively. An active form of VEGI spans amino acids 39-174 of the protein shown in SEQ ID NO:2. The natural form of VEGI is undetermined. It is possible that the biologically active form may be dimeric, trimeric, or polymeric. The present invention also relates to any allelic variation of the sequence and the specific epitopes or domains contained within the VEGI polynucleotides or polypeptides of the invention which result in the inhibition of angiogenesis. These sequences can be used to design anti-VEGI agents such as antisense oligonucleotides or to produce antibodies which inhibit VEGI function, such sequences and agents are useful for amelioration or prevention of angiogenic-associated diseases.

Even though the sequence of human VEGI is shown and described, it is understood that since human VEGI is capable of inhibiting-bovine and mouse endothelial cell growth, VEGI is well conserved among species. The sequence of VEGI from other species can be cloned using sequence and function information disclosed in this application. Therefore, VEGI polynucleotides and polypeptides sequences from species other than human are also encompassed in this application.

The present invention additionally provides isolated nucleic acid molecules comprising polynucleotides encoding VEGI polypeptide having the amino acid sequence shown in Figures 5A, 5B, and 5C (SEQ ID NO:9), which was determined by sequencing a cloned cDNA (HUVE091). The VEGI polypeptides of the present invention share sequence homology with human VEGI (SEQ ID NO:10), VEGI-beta (SEQ ID NO:11), human lymphotoxinbeta (SEQ ID NO:12) and FAS ligand (SEQ ID NO:13). The VEGI polynucleotides or polypeptides of the invention functions include, but are not limited to, the inhibition of angiogenesis, as an anti-tumor cytokine-like molecule, as a treatment for arthritis by the inhibition of angiogenesis and/or endothelial cell proliferation associated with invading pannus in bone and cartilage, as an inducer of NF-κB and c-Jun kinase (JNK), an inducer of cell adhesion, and as an inducer of apoptosis. The nucleotide sequence hown in SEQ ID NO:8 was obtained by sequencing a cDNA clone (HUVE091), which was deposited on October 26, 1994 at the American Type Culture Collection, 10801 University Boulevard, Manassas, VA 20110-2209, and given accession number 75927. The deposited plasmid is contained in pBluescript SK(-) plasmid (Stratagene, La Jolla, CA).

The present invention also provides isolated nucleic acid molecules comprising a polynucleotide (SEQ ID NO:26) encoding VEGI-beta polypeptide having the amino acid sequence shown in Figures 6A and 6B (SEQ ID NO:27), which was determined by sequencing a cloned cDNA (HEMCZ56). The VEGI-beta polypeptide is a splice variant of the VEGI polypeptide disclosed herein. The nucleotide sequence shown in SEQ ID NO:26 was obtained by sequencing a cDNA clone (HEMCZ56), which was deposited on July 9, 1998 at the American Type Culture Collection, 10801 University Boulevard, Manassas, Virginia 20110-2209, and given accession number 203055. The deposited plasmid is contained in pBluescript SK(-) plasmid (Stratagene, La Jolla, CA).

### Nucleic Acid Molecules

By "nucleotide sequence" of a nucleic acid molecule or polynucleotide is intended, for a DNA molecule or polynucleotide, a sequence of deoxyribonucleotides, and for an RNA molecule or polynucleotide, the corresponding sequence of ribonucleotides (A, G, C and U), where each thymidine deoxyribonucleotide (T) in the specified deoxyribonucleotide sequence is replaced by the ribonucleotide uridine (U).

Using the information provided herein, such as the nucleotide sequence in Figures 5A, 5B, and 5C (SEQ ID NO:8), or the nucleotide sequence in Figures 6A and 6B (SEQ ID NO:26), a nucleic acid molecule (i.e., polynucleotide) of the present invention encoding a VEGI or VEGI-beta polypeptide may be obtained using standard cloning and screening procedures, such as, for example, those for cloning cDNAs using mRNA as the starting material. For example, polynucleotides encoding VEGI polypeptides may routinely be obtained from any cell or tissue source that expresses VEGI, such as, for example, human kidney and umbilical vein endothelial cells. Illustrative of the invention, the nucleic acid molecules described in Figures 5A, 5B, and 5C (SEQ ID NO:8) was discovered in a cDNA library derived from human umbilical vein endothelial cells. The cDNA clone corresponding to VEGI (clone HUVE091) contains an open reading frame encoding a protein of 174 amino acid residues of which approximately the first 27 amino acids residues are the putative leader sequence such that the mature protein comprises 147 amino acids. The protein exhibits the highest degree of homology at the C-terminus to Rabbit TNF-α (Ito, H., et al., DNA 5:157-165 (1986); GenBank Accession No. M12846; SEQ ID NO:14) with 38% identity and 58% similarity over a 111 amino acid stretch. Sequences conserved throughout the members of the TNF family are also conserved in VEGI.

Further, polynucleotides encoding a VEGI-beta polypeptide may routinely be obtained from induced and resting endothelial cells, umbilical vein, tonsils, and several other cell and tissue types. Illustrative of the invention, the nucleic acid molecules described in Figures 6A and 6B (SEQ ID NO:26) was discovered in a cDNA library derived from induced endothelial cells. The cDNA clone corresponding to VEGI-beta (HEMCZ56) contains an open reading frame encoding a protein of 251 amino acid residues of which approximately the first 35 amino acids residues are the putative intracellular domain and amino acids 36-61 are a putative transmembrane domain and amino acid residues 62-251 are a putative extracellular domain.

The amino acid residues constituting the extracellular, transmembrane, and intracellular domains have been predicted by computer analysis. Thus, as one of ordinary skill would appreciated, the amino acid residues constituting these domains may vary slightly (e.g., by about 1 to about 15 amino acid residues) depending on the criteria used to define each domain.

In accordance with an aspect of the present invention, there is provided an isolated nucleic acid (polynucleotide) which encodes for the mature polypeptide having the deduced amino acid sequence of Figures 5A, 5B, and 5C (SEQ ID NO:9), or for the mature polypeptide encoded by the cDNA of the clone designated HUVEO91 deposited as ATCC Deposit No. 75927 on October 26, 1994.

In addition, in accordance with another aspect of the present invention, there is provided an isolated nucleic acid (polynucleotide) which encodes for the mature polypeptide having the deduced amino acid sequence of Figures 6A and 6B (SEQ ID NO:27), or for the mature polypeptide encoded by the cDNA of the clone designated HEMCZ56 deposited as ATCC Deposit No. 203055 on July 9, 1998.

By "isolated" nucleic acid molecule(s) or polynucleotide is intended a molecule, DNA or RNA, which has been removed form its native environment. For example, recombinant DNA molecules (polynucleotides) contained in a vector are considered isolated for the purposes of the present invention. Further examples of isolated DNA molecules (polynucleotides) include recombinant DNA molecules maintained in heterologous host cells or purified (partially or substantially) DNA molecules in solution. Isolated RNA molecules (polynculeotides) *include in vivo or in vitro* RNA transcripts of the DNA molecules (polynucleotides) of the present invention. Isolated nucleic acid molecules or polynucleotides according to the present invention further include such molecules produced synthetically.

The polynucleotide of the present invention may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand.

Isolated nucleic acid molecules of the present invention include the polynucleotide sequence disclosed in SEQ ID NO:8 and encoding the mature VEGI polypeptide, the polynucleotide sequence depicted in SEQ ID NO:26 encoding the mature VEGI-beta polypeptide, the polynucleotide sequences contained in deposited clone (HUVE091) deposited as ATCC Deposit No. 75927 encoding the mature VEGI-beta polypeptide, the polynucleotide sequences contained in deposited clone (HEMCZ56) deposited as ATCC Deposit No. 203055 encoding the mature VEGI-beta polypeptide, and polynucleotide sequences which comprise a sequence different from those described above, but which due to the degeneracy of the genetic code, encodes the same mature polypeptide as the DNA of SEQ ID NO:1, SEQ ID NO:8, and SEQ ID NO:26, or the deposited cDNA. Of course, the genetic code is well known in the art. Thus, it would be routine for one skilled in the art to generate such degenerate variants.

The present invention provides nucleic acid molecules encoding a mature form of the VEGI polypeptide protein. The amino acid sequence of the complete VEGI polypeptide includes a leader sequence and a mature protein, as shown in Figures 5A, 5B, and 5C (SEQ ID NO:9) and SEQ ID NO:2. According to the signal hypothesis, once export of the growing protein chain across the rough endoplasmic reticulum has been initiated, proteins secreted by mammalian cells have a signal or secretory leader sequence which is cleaved from the complete polypeptide to produce a secreted "mature" form of the protein. Most mammalian cells and even insect cells cleave secreted proteins with the same specificity. However, in some cases, cleavage of a secreted protein is not entirely uniform, which results in two or more mature species of the protein. Further, it has long been known that the cleavage specificity of a secreted protein is ultimately determined by the primary structure of the complete protein, that is, it is inherent in the amino acid sequence of the polypeptide. Therefore, the present invention provides a nucleotide sequence encoding the mature VEGI polypeptide having the amino acid sequence encoded by the cDNA clone contained in ATCC Deposit No. 75927. By the "mature VEGI polypeptide having the amino acid sequence encoded by the cDNA clone in ATCC Deposit No. 75927" is meant the mature form(s) of the VEGI polypeptide produced by expression in a mammalian cell (e.g., COS cells, as described below) of the complete open reading frame encoded by the human DNA sequence of the deposited clone.

The polynucleotide which encodes for the mature polypeptide of Figures 6A and 6B (SEQ ID NO:26) or for the mature polypeptide encoded by the deposited cDNA (HEMCZ56) may include, but is not limited to: only the coding sequence for the mature polypeptide; the coding sequence for the mature polypeptide and additional coding sequence such as a leader or secretory sequence or a transmembrane sequence or a proprotein sequence; the coding sequence for the mature polypeptide (and optionally additional coding sequence) and non-coding sequence, such as introns or non-coding sequence 5' and/or 3' of the coding sequence for the mature polypeptide.

The present invention also includes polynucleotides, wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains.

Thus, for example, the polynucleotide of the present invention may encode for a mature protein, or for a protein having a prosequence or for a protein having both a prosequence and a presequence (leader sequence).

The polynucleotides of the present invention may also have the coding sequence fused in frame to a marker sequence which allows for purification of the polypeptide of the present invention. The marker sequence may be a hexa-histidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments (i.e., portions), analogs and derivatives of the polypeptide having the deduced amino acid sequence of Figures 5A, 5B, and 5C and 6A and 6B, (SEQ ID NO: 2, SEQ ID NO:9, and SEQ ID NO:27), and the polypeptide encoded by the cDNA of the deposited clones. The variant of the polynucleotide may be a naturally occurring allelic variant of the polynucleotide or a non-naturally occurring variant of the polynucleotide.

Thus, the present invention includes polynucleotides encoding the same mature polypeptide as shown in (SEQ ID NO: 2 and SEQ ID NO:9), or the mature polypeptide encoded by the cDNA of the deposited clone HUVEO91 as well as variants of such polynucleotides which variants encode for a fragment, derivative or analog of the polypeptide of Figures 1A and B, or the polypeptide encoded by the cDNA of the deposited clone HUVE091. Such nucleotide variants include deletion variants, substitution variants and addition or insertion variants.

Additionally, the present invention includes polynucleotides encoding the mature polypeptide as shown in SEQ ID NO: 27, or the mature polypeptide encoded by the cDNA of the deposited clone HEMCZ56 as well as variants of such polynucleotides which variants encode for a fragment, derivative or analog of the polypeptide of SEQ ID NO: 203055, or the polypeptide encoded by the cDNA of the deposited clone HEMCZ56. Such nucleotide variants include deletion variants, substitution variants and addition or insertion variants.

As hereinabove indicated, the polynucleotide may have a coding sequence which is a naturally occurring allelic variant of the coding sequence shown in SEQ ID NO:1 and SEQ ID NO:8 or of the coding sequence of the deposited clone HUVEO91. Alternatively, the polynucleotide may have a coding sequence which is a naturally occurring allelic variant of the coding sequence shown in SEQ ID NO:26 or of the coding sequence of the deposited clone HEMCZ56. As known in the art, an allelic variant is an alternate form of a polynucleotide sequence which may have a substitution, deletion or addition of one or more nucleotides, which does not substantially alter the function of the encoded polypeptide.

The present invention is further directed to fragments of the isolated nucleic acid molecules described herein. By a fragment of an isolated nucleic acid molecule having the nucleotide sequence of the deposited cDNAs (HUVEO91 and HEMCZ56), or the nucleotide sequence shown in Figures 5A, 5B, and 5C (SEQ ID NO: 1 and SEQ ID NO:8) and Figures 6A and 6B (SEQ ID NO:26), or the complementary strand thereto, is intended fragments at least 15 nt, and more preferably at least 20 nt, still more preferably at least 30 nt, and even more preferably, at least 40, 50, 100, 150, 200, 250, 300, 400, or 500 nt in length. These fragments have numerous uses which include, but are not limited to, diagnostic probes and primers as discussed herein. Of course, larger fragments 50-1500 nt in length are also useful according to the present invention as are fragments corresponding to most, if not all, of the nucleotide sequence of the deposited cDNA clone HUVEO91, the deposited cDNA clone HEMCZ56, the nucleotide sequence disclosed in SEQ ID NO:8, or the nucleotide sequence disclosed in SEQ ID NO:27. By a fragment at least 20 nt in length, for example, is intended fragments which include 20 or more contiguous bases from the nucleotide sequence of the deposited cDNA clones (HUVEO91 and HEMCZ56), the nucleotide sequence as shown in SEQ ID NO:1, SEQ ID NO:8, and SEQ ID NO:26.

In specific embodiments, the polynucleotide fragments of the invention encode a polypeptide which demonstrates a functional activity. By a polypeptide demonstrating "functional activity" is meant, a polypeptide capable of displaying one or more known functional activities associated with a complete or mature VEGI polypeptide. Such functional activities include, but are not limited to, biological activity ((e.g., inhibition of angiogenesis, inhibition of endothelial cell proliferation, induction of cell adhesion, antigenicity [ability to bind (or compete with a VEGI and/or VEGI-beta polypeptide for binding) to an anti-VEGI and/or anti-VEGI-beta antibody], immunogenicity (ability to generate antibody which binds to a VEGI and/or VEGI-beta polypeptide), the ability to form polymers with other VEGI polpyeptides, and ability to bind to a receptor or ligand for a VEGI polypeptide (e.g. DR3).

The invention also provides nucleic acid molecules having nucleotide sequences related to extensive fragments of SEQ ID NO:8 which have been determined from the following related cDNA clones: HUVEO91 (SEQ ID NO:15), HMPAP05 (SEQ ID NO:16), HSXCA44 (SEQ ID NO:17), HEMFG66 (SEQ ID NO:18), and HELAM93 (SEQ ID NO:19).

The invention also provides nucleic acid molecules having nucleotide sequences related to extensive fragments of SEQ ID NO:26 which have been determined from the following related cDNA clones: HUVEO91 P01 (SEQ ID NO:28), HMPTI24R (SEQ ID NO:29), HELAM93R (SEQ ID NO:30), and HEMFG66R (SEQ ID NO:31).

In specific embodiments, the polynucleotide fragments of the invention comprise, or alternatively, consist of, a polynucleotide comprising any portion of at least 30 nucleotides, preferably at least 50 nucleotides, of SEQ ID NO:8 from nucleotide residue 1 to 2442, preferably excluding the nucleotide sequences determined from the abovelisted cDNA clones. Representative examples of the VEGI polynucleotide fragments of the invention, include fragments that comprise, or alternatively, consist of, nucleotides: 783-1304, 800-1300, 850-1300, 900-1300, 950-1300, 1000-1300, 1050-1300, 1100-1300, 1150-1300, 1200-1300, 1250-1300, 783-1250, 800-1250, 850-1250, 900-1250, 950-1250, 1000-1250, 1050-1250, 1100-1250, 1150-1250, 1200-1250, 783-1200, 800-1200, 850-1200, 900-1200, 950-1200, 1000-1200, 1050-1200, 1100-1200, 1150-1200, 783-1150, 800-1150, 850-1150, 900-1150, 950-1150, 1000-1150, 1050-1150, 1100-1150, 783-1100, 800-1100, 850-1100, 900-1100, 950-1100, 1000-1100, 1050-1100, 783-1050, 800-1050, 850-1050, 900-1050, 950-1050, 1000-1050, 783-1000, 800-1000, 850-1000, 900-1000, 950-1000, 783-950, 800-950, 850-950, 900-950, 783-900, 800-900, and 850-900 of SEQ ID NO:1, or the complementary polynucleotide strand thereto, or the cDNA contained in the deposited clone HUVEO91.

In additional specific embodiments, the polynucleotide fragments of the invention comprise, or alternatively, consist of, a polynucleotide comprising any portion of at least 30 nucleotides, preferably at least 50 nucleotides, of SEQ ID NO:26 from nucleotide residue 1 to 1116, preferably excluding the nucleotide sequences determined from the abovelisted cDNA clones.

Preferred embodiments of the invention encompass polynucleotides encoding polypeptides comprising, or alternatively consisting of, the amino acid sequence of residues -1-147 (i.e., -1 to 147), 1-147 (i.e., +1 to 147), 2-147, 3-147, 4-147, 5-147, 6-147, 7-147, 8-147, 9-147, 10-147, 11-147, 12-147, and 13-147 of SEQ ID NO:2. Polynucleotides encoding these polypeptides are also provided.

Representative examples of the VEGI-beta polynucleotide fragments of the invention, include fragments that comprise, or alternativelty, consist of, nucleotides 1-1116, 50-1116, 100-1116, 150-1116, 200-1116, 250-1116, 300-1116, 350-1116, 400-1116, 450-1116, 500-1116, 550-1116, 600-1116, 650-1116, 700-1116, 750-1116, 800-1116, 850-1116, 900-1116, 950-1116, 1000-1116, 1050-1116, 1-1100, 50-1100, 100-1100, 150-1100, 200-1100, 250-1100, 300-1100, 350-1100, 400-1100, 450-1100, 500-1100, 550-1100, 600-1100, 650-1100, 700-1100, 750-1100, 800-1100, 850-1100, 900-1100, 950-1100, 1000-1100, 1050-1100, 1-1050, 50-1050, 100-1050, 150-1050, 200-1050, 250-1050, 300-1050, 350-1050, 400-1050, 450-1050, 500-1050, 550-1050, 600-1050, 650-1050, 700-1050, 750-1050, 800-1050, 850-1050, 900-1050, 950-1050, 1000-1050, 1-1000, 50-1000, 100-1000, 150-1000, 200-1000,250-1000,300-1000,350-1000,400-1000,450-1000, 500-1000, 550-1000, 600-1000, 650-1000, 700-1000, 750-1000, 800-1000; 850-1000, 900-1000, 950-1000, 1-950, 50-950, 100-950, 150-950, 200-950, 250-950, 300-950, 350-950, 400-950, 450-950, 500-950, 550-950, 600-950, 650-950, 700-950, 750-950, 800-950, 850-950, 900-950, 1-900, 50-900, 100-900, 150-900, 200-900. 250-900, 300-900, 350-900, 400-900, 450-900, 500-900, 550-900, 600-900, 650-900, 700-900, 750-900, 800-900, 850-900, 1-850, 50-850, 100-850, 150-850, 200-850, 250-850, 300-850, 350-850, 400-850, 450-850, 500-850, 550-850, 600-850, 650-850, 700-850, 750-850, 800-850, 1-800, 50-800,100-800,150-800,200-800,250-800,300-800,350-800, 400-800, 450-800, 500-800, 550-800, 600-800, 650-800, 700-800, 750-800, 1-750, 50-750, 100-750, 150-750, 200-750, 250-750, 300-750, 350-750, 400-750, 450-750, 500-750, 550-750, 600-750, 650-750, 700-750, 1-700, 50-700, 100-700, 150-700, 200-700, 250-700 300-700, 350-700, 400-700, 450-700, 500-700, 550-700, 600-700, 650-700, 1-650, 50-650, 100-650, 150-650, 200-650, 250-650, 300-650, 350-650, 400-650, 450-650, 500-650, 550-650, 600-650, 1-600, 50-600, 100-600, 150-600, 200-600, 250-600, 300-600, 350-600, 400-600, 450-600, 500-600, 550-600, 1-550, 50-550, 100-550, 150-550, 200-550, 250-550, 300-550, 350-550, 400-550, 450-550, 500-550, 1-500, 50-500, 100-500, 150-500, 200-500, 250-500, 300-500, 350-500, 400-500, 450-500, 1-450, 50-450, 100-450, 150-450, 200-450, 250-450, 300-450, 350-450, 400-450, 1-400, 50-400, 100-400, 150-400, 200-400, 250-400, 300-400, 350-400, 1-350, 50-350, 100-350, 150-350, 200-350, 250-350, 300-350, 1-300, 50-300, 100-300, 150-300, 200-300, 250-300, 1-250, 50-250, 100-250, 150-250, 200-250, 1-200, 50-200, 100-200, 150-200, 1-150, 50-150, 100-150, 1-100, 50-100, and 1-50 of SEQ ID NO: 19 or the complementary polynucleotide strand thereto, or the cDNA contained in the deposited clone HEMCZ56.

Preferred nucleic acid fragments of the present invention also include nucleic acid molecules encoding one or more of the following domains of VEGI potential asparagine-linked glycosylation sites N-29 through N-32 (N-29, Y-30, T-31, N-32) and N-125 through D-128 (N-125, V-126, S-127, D-128); potential Protein Kinase C (PKC) phosphorylation sites T-32 through K-34 (T-32, N-33, K-34) and T-50 through R-52 (T-50, F-51, R-52); potential Casein Kinase II (CK2) phosphorylation sites S-83 through E-86 (S-83, Y-84, P-85, E-86); S-96 through E-99 (S-96, V-97, C-98, E-99); S-115 through E-118 (S-115, L-116, Q-117, E-118); S-130 through D-133 (S-130, L-131, V-132, D-133); and T-135 through D-138 (T-135, K-136, E-137, D-138); and potential myristylation sites G-20 through K-25 (G-20, L-21, A-22, F-23, T-24, K-25) and G-111 through L-116 (G-111, A-112, M-113, F-114, S-115, L-116) of SEQ ID NO:2.

Among the especially preferred polynucleotides of the invention are those characterized by encoding structural or functional attributes of VEGI. Such polynucleotides encode amino acid residues that comprise alpha-helix and alpha-helix forming regions ("alpha-regions"), beta-sheet and beta-sheet forming regions ("beta regions"), turn and turn-forming regions ("turn-regions"), coil and coil-forming regions ("coil regions"), hydrophilic regions, hydrophobic regions, alpha amphipathic regions, beta amphipathic regions, surface forming regions, and high antigenic index regions (i.e., having an antigenic regions of three or more contiguous amino acid residues each of which having an antigenic index of greater than or equal to 1.5) of VEGI. Certain preferred regions are those set out in Figure 7, and include, but are not limited to, regions of the aforementioned types identified by analysis of the amino acid sequence depicted in SEQ ID NO:9 using the default parameters of the identified computer programs, such preferred regions include; Garnier-Robson alpha-regions, beta-regions, turn-regions, and coil-regions, Chou-Fasman alpha-regions, beta-regions, and coil-regions, Kyte-Doolittle hydrophilic regions and hydrophobic regions, Eisenberg alpha- and beta-amphipathic regions, Karplus-Schulz flexible regions, Emini surface-forming regions and Jameson-Wolf regions of high antigenic index.

Data which represent VEGI-beta in a fashion as described above for VEGI may easily be prepared using the amino acid sequence disclosed in SEQ ID NO:27. As such, each of the abovelisted structural or functional attributes of VEGI listed above (i.e. Garnier-Robson alpha-regions, beta-regions, turn-regions, and coil-regions, Chou-Fasman alpha-regions, beta-regions, and coil-regions, Kyte-Doolittle hydrophilic regions and hydrophobic regions, Eisenberg alpha- and beta-amphipathic regions, Karplus-Schulz flexible regions, Emini surface-forming regions and Jameson-Wolf regions of high antigenic index, etc.) apply equally well to VEGI and VEGI-beta.

Certain preferred regions in these regards are set out in Figure 7, but may also be represented or identified by using a tabular representation of the data presented in Figure 7. The DNA*STAR computer algorithm used to generate Figure 7 (set on the original default parameters) will easily present the data in Figure 7 in such a tabular format. A tabular format of the data in Figure 7 may be used to easily determine specific boundaries of a preferred region.

The above-mentioned preferred regions set out in Figure 7 include, but are not limited to, regions of the aforementioned types identified by analysis of the amino acid sequence set out in SEQ ID NO: 2, SEQ ID NO:9, and SEQ ID NO:27. As set out in Figure 7, such preferred regions include Garnier-Robson alpha-regions, beta-regions, turn-regions, and coil-regions, Chou-Fasman alpha-regions, beta-regions, and coil-regions, Kyte-Doolittle hydrophilic regions and hydrophobic regions, Eisenberg alpha- and beta-amphipathic regions, Karplus-Schulz flexible regions, Emini surface-forming regions and Jameson-Wolf regions of high antigenic index.

Among highly preferred fragments in this regard are those that comprise reigons of VEGI and/or VEGI-beta that combine several structural features, such as several (e.g., 1, 2, 3 or 4) of the features set out above.

Additional preferred nucleic acid fragments of the present invention include nucleic acid molecules encoding one or more epitope-bearing portions of the VEGI polypeptide. In particular, such nucleic acid fragments of the present invention included nucleic acid molecules encoding: a polypeptide comprising amino acid residues from about Thr-24 to about Asn-32 in SEQ ID NO:9; a polypeptide comprising amino acid residues from about Ile-37 to about Ile-45 in SEQ ID NO:9; a polypeptide comprising amino acid residues from about Met-54 to about Arg-62 in SEQ ID NO:9; a polypeptide comprising amino acid residues from about Gln-63 to about Asp-71 in SEQ ID NO:9; a polypeptide comprising amino acid residues from about Glu-57 to about Gly-65 in SEQ ID NO:9; a polypeptide comprising amino acid residues from about Val-80 to about Thr-88 in SEQ ID NO:9; a polypeptide comprising amino acid residues from about Leu-116 to about Val-124 in SEQ ID NO:9; and a polypeptide comprising amino acid residues from about Asp-133 to about Phe-141 in SEQ ID NO:9. These polypeptide fragments have been determined to bear antigenic epitopes of the VEGI polypeptide by the analysis of the Jameson-Wolf antigenic index, as shown in Figure 7, above. Methods for determining other such epitope-bearing portions of VEGI are described in detail below. Polypeptide fragments which bear antigenic epitopes of the VEGI-beta protein may be easily determined by one of skill in the art using the above-described analysis of the Jameson-Wolf antigenic index, as shown in Figure 7. Methods for determining other such epitope-bearing portions of VEGI-beta are described in detail below.

Another embodiment of the invention is directed to polynucleotides that hybridize, preferably under stringent hybridization conditions, to a portion of the polynucleotide sequence of a polynucleotide of the invention such as, for instance, the cDNA clone contained in ATCC Deposit No. 75927, the cDNA clone contained in ATCC Deposit 203055 or a VEGI polynucleotide fragment as described herein. By "stringent hybridization conditions" is intended overnight incubation at 42°C in a solution comprising: 50% formamide, 5x SSC (750 mM NaCl, 75mM trisodium citrate), 50 mM sodium phosphate (pH 7.6); 5x Denhardt's solution, 10% dextran sulfate, and 20 µg/ml denatured, sheared salmon sperm DNA, followed by washing the filters in 0.1x SSC at about 65°C. By a polynucleotide which hybridizes to a "portion" of a polynucleotide is intended a polynucleotide (either DNA or RNA) hybridizing to at least 15 nucleotides (nt), and more preferably at least 20 nt, still more preferably at least 30 nt, and even more preferably 30-70, or 80-150 nt, or the entire length of the reference polynucleotide. These are useful as diagnostic probes and primers as discussed above and in more detail below. Of course, a polynucleotide which hybridizes only to a poly A sequence (such as the 3' terminal poly tract of the VEGI cDNA shown in SEQ ID NO:1, SEQ ID NO:8 or SEQ ID NO:26), or to a complementary stretch of T (or U) residues, would not be included in a polynucleotide of the invention used to hybridize to a portion of a nucleic acid of the invention, since such a polynucleotide would hybridize to any nucleic acid molecule containing a poly (A) stretch or the complement thereof (e.g., practically any double-stranded cDNA clone generated using oligo dT as a primer).

In preferred embodiments, polynucleotides which hybridize to the reference polynucleotides disclosed herein encode polypeptides which either retain substantially the same biological function or activity as the mature polypeptide encoded by the polynucleotide sequences disclosed in SEQ ID NO: 1, SEQ ID NO:8 and/or SEQ ID NO:26, or the cDNAs contained in the deposit.

The present invention further relates to variants of the nucleic acid molecules of the present invention, which encode portions, analogs or derivatives of the VEGI polypeptide. Variants may occur naturally, such as a natural allelic variant. By an "allelic variant" is intended one of several alternate forms of a gene occupying a given locus on a chromosome of an organism *(*Genes II, Lewin, B., ed., John Wiley & Sons, New York (1985)). Non-naturally occurring variants may be produced using art-known mutagenesis techniques.

Such variants include those produced by nucleotide substitutions, deletions or additions of the polynucleotide sequences described herein (including fragments). The substitutions, deletions or additions may involve one or more nucleotides. The variants may be altered in coding regions, non-coding regions, or both. Alterations in the coding regions may produce conservative or non-conservative amino acid substitutions, deletions or additions. Especially preferred among these are silent substitutions, additions and deletions, which do not alter the properties and activities of the VEGI polypeptide or portions thereof. Also especially preferred in this regard are conservative substitutions.

Further embodiments of the invention are directed to isolated nucleic acid molecules comprising a polynucleotide sequence at least 70% or at least 80% identical, more preferably at least 90% identical, and still more preferably at least 95%, 96%, 97%, 98% or 99% identical to a polynucleotide having a nucleotide sequence selected from the group consisting of: (a) a nucleotide sequence encoding the VEGI polypeptide having the complete amino acid sequence in SEQ ID NO:2 or SEQ ID NO:9 (i.e., positions -27 to 147 of SEQ ID NO:9); (b) a nucleotide sequence encoding VEGI polypeptide having the complete amino acid sequence in SEQ ID NO:2 or SEQ ID NO:9 excepting the N-terminal methionine (i.e., positions -26 to 147 of SEQ ID NO:9); (c) a nucleotide sequence encoding the mature VEGI polypeptide having the amino acid sequence in SEQ ID NO:9 shown as positions 1 to 147 of SEQ ID NO:9; (d) a nucleotide sequence encoding the extracellular domain of the VEGI polypeptide having the amino acid sequence in SEQ ID NO:2 or SEQ ID NO:9 shown as positions 1 to 147 of SEQ ID NO:9; (e) a nucleotide sequence encoding the VEGI polypeptide having the complete amino acid sequence encoded by the cDNA clone HUVEO91 contained in ATCC Deposit No. 75927; (f) a nucleotide sequence encoding the VEGI polypeptide having the complete amino acid sequence excepting the N-terminal methionine encoded by the cDNA clone HUVEO91 contained in ATCC Deposit No. 75927; (g) a nucleotide sequence encoding the mature VEGI polypeptide having the amino acid sequence encoded by the cDNA clone HUVEO91 contained in ATCC Deposit No. 75927; (h) a nucleotide sequence encoding the extracellular domain of the VEGI polypeptide having the amino acid sequence encoded by the cDNA clone HUVEO91 contained in ATCC Deposit No. 75927; (i) a nucleotide sequence encoding a polyeptide fragment described herein; and (j) a nucleotide sequence complementary to any of the nucleotide sequences in (a), (b), (c), (d), (e), (f), (g), (h) or (i), above. The polypeptides of the present invention also include polypeptides having an amino acid sequence at least 80% identical, more preferably at least 90% identical, and still more preferably 95%, 96%, 97%, 98% or 99% identical to those described in (a), (b), (c), (d), (e), (f), (g), (h), (i) or (j), above, as well as polypeptides having an amino acid sequence with at least 90% similarity, and more preferably at least 95% similarity, to those above.

Further embodiments of the invention are directed to isolated nucleic acid molecules comprising a polynucleotide sequence at least 70% or at least 80% identical, more preferably at least 90% identical, and still more preferably at least 95%, 96%, 97%, 98% or 99% identical to a polynucleotide having a nucleotide sequence selected from the group consisting of: (a) a nucleotide sequence encoding the VEGI-beta polypeptide having the complete amino acid sequence in SEQ ID NO:27 (i.e., positions 1 to 251 of SEQ ID NO:27); (b) a nucleotide sequence encoding the VEGI-beta polypeptide having the complete amino acid sequence in SEQ ID NO:27 excepting the N-terminal methionine (i.e., positions 2 to 251 of SEQ ID NO:27); (c) a nucleotide sequence encoding the mature VEGI-beta polypeptide having the amino acid sequence in SEQ ID NO:27 shown as positions 62 to 251 of SEQ ID NO:27; (d) a nucleotide sequence encoding the intracellular domain of the VEGI-beta polypeptide having the amino acid sequence in SEQ ID NO:27 shown as positions 1 to 35 of SEQ ID NO:27; (e) a nucleotide sequence encoding the extracellular domain of the VEGI-beta polypeptide having the amino acid sequence in SEQ ID NO:27 shown as positions 62 to 251 of SEQ ID NO:27; (f) a nucleotide sequence encoding the VEGI-beta polypeptide having the complete amino acid sequence encoded by the cDNA clone HEMCZ56 contained in ATCC Deposit No. 203055; (g) a nucleotide sequence encoding the VEGI-beta polypeptide having the complete amino acid sequence excepting the N-terminal methionine encoded by the cDNA clone HEMCZ56 contained in ATCC Deposit No. 203055; (h) a nucleotide sequence encoding the mature VEGI-beta polypeptide having the amino acid sequence encoded by the cDNA clone HEMCZ56 contained in ATCC Deposit No. 203055; (i) a nucleotide sequence encoding the intracellular domain of the VEGI-beta polypeptide having the amino acid sequence encoded by the cDNA clone HEMCZ56 contained in ATCC Deposit No. 203055; (j) a nucleotide sequence encoding the extracellular domain of the VEGI-beta polypeptide having the amino acid sequence encoded by the cDNA clone HEMCZ56 contained in ATCC Deposit No. 203055; and (k) a nucleotide sequence complementary to any of the nucleotide sequences in (a), (b), (c), (d), (e), (f), (g), (h), (i) or (j), above. The polypeptides of the present invention also include polypeptides having an amino acid sequence at least 80% identical, more preferably at least 90% identical, and still more preferably 95%, 96%, 97%, 98% or 99% identical to those described in (a), (b), (c), (d), (e), (f), (g), (h), (i), (j) or (k), above, as well as polypeptides having an amino acid sequence with at least 90% similarity, and more preferably at least 95% similarity, to those above.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the VEGI polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The reference (query) sequence may be the entire nucleotide sequence disclosed in SEQ ID NO:1, SEQ ID NO:8, and SEQ ID NO:26, or any fragment as described herein.

As a practical matter, whether any particular nucleic acid molecule is at least 70%, 80%, 90%, 95%, 96%, 97%, 98% or 99% identical to, for instance, the nucleotide sequence shown in SEQ ID NO:8, SEQ ID NO:26, or to the nucleotide sequence of the deposited cDNA clones can be determined conventionally using known computer programs such as the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2:482-489 (1981), to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

In a specific embodiment, the identity between a reference (query) sequence (a sequence of the present invention) and a subject sequence, also referred to as a global sequence alignment, is determined using the FASTDB computer program based on the algorithm of Brutlag et al. (Comp. App. Biosci. 6:237-245 (1990)). Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter. According to this embodiment, if the subject sequence is shorter than the query sequence because of 5' or 3' deletions, not because of internal deletions, a manual correction is made to the results to take into consideration the fact that the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. A determination of whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of this embodiment. Only bases outside the 5' and 3' bases of the subject sequence, as displayed by the FASTDB alignment, which are not matched/aligned with the query sequence, are calculated for the purposes of manually adjusting the percent identity score. For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for. No other manual corrections are made for the purposes of this embodiment.

In further embodiments, the present invention is directed to polynucleotides having at least a 70% identity, preferably at least 90% and more preferably at least a 95% identity to a polynucleotide which encodes the polypeptide of SEQ ID NO:2 as well as fragments thereof, which fragments have at least 30 bases and preferably at least 50 bases and to polypeptides encoded by such polynucleotides.

In further embodiments, the present invention is directed to polynucleotides having at least a 70% identity, preferably at least 90% and more preferably at least a 95% identity to a polynucleotide which encodes the polypeptide of SEQ ID NO:27 as well as fragments thereof, which fragments have at least 30 bases and preferably at least 50 bases and to polypeptides encoded by such polynucleotides.

The present application is directed to nucleic acid molecules at least 70%, 80%, 90%, 95%, 96%, 97%, 98% or 99% identical to the polynucleotide sequence shown in SEQ ID NO:1, SEQ ID NO:8, SEQ ID NO:26, or to the nucleic acid sequence of the deposited cDNA clones, or fragments thereof, irrespective of whether they encode a polypeptide having VEGI functional activity. It is preferred however, that the nucleic acid molecules of the invention encode a polypeptide having VEGI functional activity. By "a polypeptide having VEGI functional activity" is intended polypeptides exhibiting activity similar, but not necessarily identical, to an activity of the VEGI and/or VEGI-beta polypeptide of the invention (either the full-length protein or, preferably, the mature protein), as measured in a particular immunoassay and/or biological assay. For example, VEGI activity can be measured by determining the relative ability of VEGI to inhibit the FGF-2-induced formation of capillary-like tubular structure formation in cultures of ABAE cells as described in detail in Example 6 or in a chorioallantoic membrane (CAM) angiogenesis assay as described in Example 10, and in several additional ways described in the remaining Examples and in the art.

Of course, due to the degeneracy of the genetic code, one of ordinary skill in the art will immediately recognize that a large number of the nucleic acid molecules having a sequence at least 70%, 80%, 90%, 95%, 96%, 97%, 98%, or 99% identical to the nucleic acid sequence of the deposited cDNA or the nucleic acid sequence disclosed in SEQ ID NO:1, SEQ ID NO:8, SEQ ID NO:26, or fragments thereof, will encode a polypeptide "having VEGI activity." In fact, since degenerate variants of these nucleotide sequences all encode the same polypeptide, in many instances, this will be clear to the skilled artisan even without performing the above described assay. It will be further recognized in the art that, for such nucleic acid molecules that are not degenerate variants, a reasonable number will also encode a polypeptide having VEGI activity. This is because the skilled artisan is fully aware of amino acid substitutions that are either less likely or not likely to significantly effect protein function (e.g., replacing one aliphatic amino acid with a second aliphatic amino acid). For example, guidance concerning how to make phenotypically silent amino acid substitutions is provided in J.U. Bowie et al., "Deciphering the Message in Protein Sequences: Tolerance to Amino Acid Substitutions," Science 247:1306-1310 (1990), wherein the authors indicate that proteins are surprisingly tolerant of amino acid substitutions.

Additional embodiments of the invention are directed to isolated nucleic acid molecules comprising a polynucleotide which encodes the amino acid sequence of a VEGI polypeptide (e.g., a VEGI polypeptide fragment described herein) having an amino acid sequence which contains at least one conservative amino acid substitution, but not more than 50 conservative amino acid substitutions, even more preferably, not more than 40 conservative amino acid substitutions, still more preferably, not more than 30 conservative amino acid substitutions, and still even more preferably, not more than 20 conservative amino acid substitutions, 10-20 conservative amino acid substitutions, 5-10 conservative amino acid substitutions, 1-5 conservative amino acid substitutions, 3-5 conservative amino acid substitutions, or 1-3 conservative amino acid substitutions. Of course, in order of ever-increasing preference, it is highly preferable for a polynucleotide which encodes the amino acid sequence of a VEGI polypeptide to have an amino acid sequence which contains not more than 10, 9, 8, 7, 6, 5, 4, 3, 2 or 1 conservative amino acid substitutions.

### Vectors and Host Cells

The present invention also relates to vectors which include the isolated polynucleotides of the present invention, host cells which are genetically engineered with the recombinant vectors, or which are otherwise engineered to produce the polypeptides of the invention, and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the VEGI genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operably associated with an appropriate expression control sequence(s) (promoter) to direct mRNA synthesis. As representative examples of such promoters, there may be mentioned: LTR or SV40 promoter, the *E. coli lac* or *trp,* the phage lambda P promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. *coli.*

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as *E. coli, Streptomyces, Salmonella typhimurium;* fungal cells, such as yeast; insect cells such as *Drosophila* S2 and Sf9; animal cells such as CHO, COS or Bowes melanoma, adenoviruses, plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably associated with the sequence. Large numbers of suitable vectors and promoters are known to those of skill in the art, and are commercially available. The following vectors are provided by way of example. Bacterial: pHE4-5 (ATCC Accession No. 209311; and variations thereof), pQE70, pQE60, pQE-9 (Qiagen), pBS, pD10, phagescript, psiX174, pBluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include *lacl, lacZ,* T3, T7, gpt, lambda P, P, and *trp.* Eukaryotic promoters include CMV immediate early, HSV thymidine kinase, early and late SV40, LTRs from retroviruses, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., VEGI coding sequence), and/or to include genetic material (e.g., heterologous polynucleotide sequences) that is operably associated with VEGI polynucleotides of the invention, and which activates, alters, and/or amplifies endogenous VEGI polynucleotides. For example, techniques known in the art may be used to operably associate heterologous control regions (e.g., promoter and/or enhancer) and endogenous VEGI polynucleotide sequences via homologous recombination (see, e.g., U.S. Patent No. 5,641,670, issued June 24, 1997; International Publication No. WO 96/29411, published September 26, 1996; International Publication No. WO 94/12650, published August 4, 1994; Koller et al., Proc. Natl. Acad. Sci. USA 86:8932-8935 (1989); and Zijlstra et al., Nature 342:435-438 (1989), the disclosures of each of which are incorporated by reference in their entireties).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are *cis*-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of *E*. *coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), a-factor, acid phosphatase, or heat shock proteins, among others.
The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, for example, stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include *E. coli, Bacillus subtilis, Salmonella typhimurium,* and various species within the genera *Pseudomonas, Streptomyces,* and *Staphylococcus,* although others may also be employed as a matter of choice.

As a representative, but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period. Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman (Cell 23:175 (1981)), and other cell lines capable of expressing a compatible vector, for example, the C127,3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The VEGI polypeptides can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

At least fifteen VEGI expression constructs have been generated by the inventors herein to facilitate the production of VEGI polypeptides of several sizes and in several systems. Of these, four have been constructed which encode a full-length VEGI polypeptide. The full-length constructs are: (i) pQE9TNFg-27/147, (ii) pQE70TNFg, (iii) pC1TNFg, and pcDNA3TNFg. In the case of the first expression construct listed (pQE9TNFg-27/147), the construct was used to produce a full-length VEGI polypeptide with an N-terminal six histidine amino acid tag according to the method of Example 1. A full-length VEGI polypeptide lacking the histidine tag was produced in bacteria by using the pQE70TNFg construct essentially as was done in Example 1. In addition, a full-length VEGI polypeptide lacking a histidine tag was produced in mammalian cells by using either the pC1TNFg or pcDNA3TNFg constructs according to the method of Example 3. Further, the mature VEGI polypeptide was produced and secreted from mammalian cells under direction of the interleukin (IL)-6 signal peptide from a construct designated pcDNA3/IL6TNFg-1/149.

The remaining VEGI expression constructs were used to express various VEGI muteins from bacterial, baculoviral, and mammalian systems. Four N-terminal deletion mutations have been generated using the pQE60 bacterial expression vector. These N-terminal deletion mutation constructs are: (i) pQE60TNFg-3/147 (representing a possible mature VEGI polypeptide; the polypeptide expressed by this construct is identical to amino acid residues 107-251 of the VEGI-beta of SEQ ID NO:27), (ii) pQE60TNFg12/147 (representing amino acid residues 12-147 of SEQ ID NO:9 and residues 116-251 of SEQ ID NO:27), (iii) pQE60TNFg22/147 (representing amino acid residues 22-147 and residues 126-251 of SEQ ID NO:27), and (iv) pQE60TNFg28/147 (representing amino acid residues 28-147 and residues 132-251 of SEQ ID NO:27). Each of these expression constructs can be used to produce a VEGI polypeptide in a bacterial system which exhibits an N-terminal deletion of 25, 39, 49, and 55 amino acids, respectively, with regard to the full-length VEGI polypeptide or an N-terminal deletion of 106, 115, 125, and 131 amino acids, respectively, with regard to the full-length VEGI-beta polypeptide.

Further N-terminal deletion mutation bacterial expression constructs have been generated. A construct designated pHE4 VEGI T30-L174 has been generated using the bacterial expression vector pHE4 to express amino acids threonine-30 to leucine-174 of the VEGI sequence disclosed as residues threonine-3 to leucine-147 of SEQ ID NO:9 which correspond exactly to amino acid residues threonine-107 to leucine-251 of the VEGI-beta sequence residues threonine-107 to leucine-251 of SEQ ID NO:27). Additional bacterial expression constructs generated include pQE9.VEGI.his.T28-L174, pHE4.VEGLT28-L174, pHE4.VEGLT51-L174, and pHE4.VEGLT58-L174. These constructs are based on either the pQE9 or pHE4 bacterial expression vectors. The construct designations indicate the expression vector, the gene name, and the amino acid residues expressed by the construct (e.g. pQE9.VEGI.T28-L174 indicates that the pQE9 bacterial expression vector is used to express amino acids threonine (T)-28 through leucine (L)-174 of the VEGI.

A VEGI expression construct has been generated which can be used to produce a secreted mature VEGI polypeptide from a mammalian system. The construct has been designated pCl/IL6TNFg-3/147. It encodes the signal peptide from the human IL-6 gene fused to the mature VEGI sequence. A similar construct has been generated which contains the CK-b8 signal peptide (amino acids -21 to -1 of the CK-b8 sequence disclosed in published PCT application PCT/US95/09058; filed 6/23/95) fused to the amino terminus of amino acids 12-149 of VEGI (SEQ ID NO:9; that is, amino acids 116-251 of VEGI-beta (SEQ ID NO:27)) in the context of the pC4 mammalian expression vector. This construct has been designated pC4/CK-b8TNFg12/147. A variant of this construct has been generated which can be used to express amino acids 12-147 of VEGI fused to the human IgG Fc region at the VEGI carboxy terminus. This fusion protein is also secreted under the direction of the CK-β8 signal peptide and has been designated pC4/CK-β8TNFg12/147/Fc. The sequence of the human Fc portion of the fusion molecule is shown in SEQ ID NO:25. Other sequences could be used which are known to those of skill in the art.

Amino acids -3 to 147 of VEGI (SEQ ID NO:9; which correspond to amino acid residues 102 to 251 of VEGI-beta (SEQ ID NO:27)) can be expressed and secreted from a baculovirus system by using a construct designated pA2GPTNFg-3/147. This expression construct encodes the mature VEGI coding sequence fused at its amino terminus to the baculoviral GP signal peptide.

Two retroviral VEGI expression constructs have also been generated. The first of these has been designated pG1SamEN/TNFg-3/149. This expression construct can be used to produce full-length VEGI polypeptide from a mammalian system. A related construct, pG1SamEN/CK-β8TNFg12/149, has been generated which can be used to produce and secrete mature VEGI polypeptide from a mammalian system under the direction of the CK-β8 signal peptide.

Further polypeptides of the present invention include polypeptides which have at least 90% similarity, more preferably at least 95% similarity, and still more preferably at least 96%, 97%, 98% or 99% similarity to those described above. The polypeptides of the invention also comprise those which are at least 80% identical, more preferably at least 90% or 95% identical, still more preferably at least 96%, 97%, 98% or 99% identical to the polypeptide encoded by the deposited cDNA or to the polypeptide of SEQ ID NO:9, and also include portions of such polypeptides with at least 30 amino acids and more preferably at least 50 amino acids.

Methods for producing the recombinant or fusion protein described above are known in the art (See e.g., Maniatis, Fitsch and Sambrook, Molecular Cloning: A Laboratory Manual (1982) or DNA Cloning, Volumes I and II (D. N. Glover ed. (1985) for general cloning methods) and include culturing host-cells stably transformed with an expression vector containing a DNA fragment which encodes a polypeptide of the present invention under conditions such that the DNA fragment is expressed and the recombinant or fusion protein is produced. In addition the protein or polynucleotide can be fused to other or polypeptides which increase its function, or specify its localization in the cell, such as a secretion sequence as presented in Examples 1-3. The recombinant or fusion protein can be isolated by methods known in the art. The transformed host cells can be used to analyze the effectiveness of drugs and agents which inhibit or activate VEGI function, such as host proteins or chemically derived agents or other proteins which may interact with VEGI polynucleotides to down-regulate or alter the expression of VEGI polypeptides or affect its ability to inhibit angiogenesis. A method for testing the effectiveness of an anti-VEGI or anti-angiogenesis drug or agent can for example be the blockage of the endothelial cell growth inhibitory activity of VEGI. That is, the presence of such an anti-VEGI agent would lead for lowered ability-of VEGI to inhibit endothelial cell growth. As a result, more VEGI will be required to achieve a comparable inhibition observed in the absence of the anti-VEGI agent. On the other hand, the presence of an anti-angiogenic agent may augment or synergism the ability of VEGI to inhibit endothelial cell growth. Consequently, lower concentrations of VEGI will be required to achieve a similar inhibition observed in the absence of the agent.

### Polypeptides and Fragments

The present invention further relates to an isolated VEGI polypeptide which has the deduced amino acid sequence of SEQ ID NO:2 or SEQ ID NO:9 or which has the amino acid sequence encoded by the deposited cDNA HUVE091, as well as fragments, analogs and derivatives of such polypeptide.

The present invention also relates to a VEGI-beta polypeptide which has the deduced amino acid sequence of SEQ ID NO:27 or which has the amino acid sequence encoded by the deposited cDNA HEMCZ56, as well as fragments, analogs and derivatives of such polypeptide.

The polypeptides and polynucleotides of the present invention are preferably provided in an isolated form, and preferably are purified to a point within the range of near complete (e.g., >90% pure) to complete (e.g., >99% pure) homogeneity. The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Also intended as an "isolated polypeptide" are polypeptides that have been purified partially or substantially from a recombinant host cell. For example, a recombinantly produced version of a VEGI polypeptide can be substantially purified by the one-step method described by Smith and Johnson (Gene 67:31-40 (1988)). Such polynucleotides could be part of a vector and/or such polynucleotides or polypeptides could be part of a composition, and still be isolated in that such vector or composition is not part of its natural environment. Isolated polypeptides and polynucleotides according to the present invention also include such molecules produced naturally or synthetically. Polypeptides and polynucleotides of the invention also can be purified from natural or recombinant sources using anti-VEGI antibodies of the invention in methods which are well known in the art of protein purification.

The terms "fragment," "derivative" and "analog" when referring to the polypeptides of SEQ ID NO:2, SEQ ID NO:9, or SEQ ID NO:27, and those polypeptodes encoded by the deposited cDNAs, means a polypeptide which retains a VEGI functional activity, i.e,. displays one or more functional activities associated with a full-length and/or mature VEGI polypeptide disclosed in SEQ ID NO:2, SEQ ID NO:9, or SEQ ID NO:27, and/or encloded by one or both of the deposited clones (HUVEO91 and HEMCZ56). As one example, such fragments, derivatives, or analogs, which have the desired immunogenicity or antigenicity can be used, for example, in immunoassays, for immunization, for inhibition of VEGI activity, etc. Thus, a specific embodiment of the invention relates to a VEGI fragment that can be bound by an antibody that specifically binds the VEGI polypeptide sequence disclosed in SEQ ID NO:2, SEQ ID NO:9, SEQ ID NO:27, and/or which is encloded by one or both of the deposited clones (HUVE091 and HEMCZ56).

As another example, VEGI fragments, derivatives or analogs which have VEGI biological activity (e.g., a mature VEGI polypeptide or the extracellular domain of a VEGI-beta polypeptide) are provided. VEGI fragments, derivatives, and analogs that retain, or alternatively lack a desired VEGI property of interest (e.g., inhibition of cell prolifieration, tumor inhibition, inhibition of angiogenesis, anti-arthritis by the inhibition of angiogenesis and/or endothelial cell proliferation associated with invading pannus in bone and cartilage, an inducer of NF-κB and c-Jun kinase (JNK), an inducer of cell adhsion, and as an inducer apoptosis can be used as inducers or inhibitors, respectively, of such properties and its physiological correlates.

The polypeptides of the invention may exist as a membrane bound receptor having a transmembrane region and an intra- and extracellular region or they may exist in soluble form wherein the transmembrane domain is lacking. One example of such a form of VEGI is the VEGI-beta polypeptide sequence shown in SEQ ID NO:27 which contains a transmembrane, intracellular and extracellular domain.

It will be recognized in the art that some amino acid sequences of the VEGI polypeptide can be varied without significant effect of the structure or function of the protein. If such differences in sequence are contemplated, it should be remembered that there will be critical areas on the protein which determine activity. Thus, the invention further includes variations of the VEGI polypeptide which show substantial VEGI polypeptide activity or which include regions of VEGI such as the polypeptide fragments disclosed herein. Such variants include deletions, insertions, inversions, repeats, and type substitutions selected according to general rules known in the art so as have little effect on activity. For example, guidance concerning how to make phenotypically silent amino acid substitutions is provided wherein the authors indicate that there are two main approaches for studying the tolerance of an amino acid sequence to change (Bowie et al., Science 247:1306-1310 (1990)). The first method relies on the process of evolution, in which mutations are either accepted or rejected by natural selection. The second approach uses genetic engineering to introduce amino acid changes at specific positions of a cloned gene and selections or screens to identify sequences that maintain functionality. As the authors state, these studies have revealed that proteins are surprisingly tolerant of amino acid substitutions. The authors further indicate which amino acid changes are likely to be permissive at a certain position of the protein. For example, most buried amino acid residues require nonpolar side chains, whereas few features of surface side chains are generally conserved. Other such phenotypically silent substitutions are described by Bowie and coworkers *(supra)* and the references cited therein. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Thus, the fragment, derivative or analog of the polypeptide of SEQ ID NO:9, or of SEQ ID NO:27, or those encoded by the deposited cDNAs, may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature form of the VEGI polypeptide is fused with another compound, such as a compound to increase the half-life of the polypeptide (for example, polyethylene glycol), or (iv) one in which the additional amino acids are fused to the above form of the polypeptide, such as an IgG Fc fusion region peptide or leader or secretory sequence or a sequence which is employed for purification of the above form of the polypeptide or a proprotein sequence. Such fragments, derivatives and analogs are deemed to be within the scope of those skilled in the art from the teachings herein.

Thus, the VEGI of the present invention may include one or more amino acid substitutions, deletions or additions, either from natural mutations or human manipulation. As indicated, changes are preferably of a minor nature, such as conservative amino acid substitutions that do not significantly affect the folding or activity of the protein (see Table 1).

**TABLE 1. Conservative Amino Acid Substitutions.**

| | |
|---|---|
| Aromatic | Phenylalanine |
| | Tryptophan |
| | Tyrosine |
| | |
| Hydrophobic | Leucine |
| | Isoleucine |
| | Valine |
| | |
| Polar | Glutamine |
| | Asparagine |
| | |
| Basic | Arginine |
| | Lysine |
| | Histidine |
| | |
| Acidic | Aspartic Acid |
| | Glutamic Acid |
| | |
| Small | Alanine |
| | Serine |
| | Threonine |
| | Methionine |
| | Glycine |

Embodiments of the invention are directed to polypeptides which comprise the amino acid sequence of a VEGI polypeptide described herein, but having an amino acid sequence which contains at least one conservative amino acid substitution, but not more than 50 conservative amino acid substitutions, even more preferably, not more than 40 conservative amino acid substitutions, still more preferably, not more than 30 conservative amino acid substitutions, and still even more preferably, not more than 20 conservative amino acid substitutions, when compared with the VEGI polynucleotide sequence described herein. Of course, in order of ever-increasing preference, it is highly preferable for a peptide or polypeptide to have an amino acid sequence which comprises the amino acid sequence of a VEGI polypeptide, which contains at least one, but not more than 10, 9, 8, 7, 6, 5, 4, 3, 2 or 1 conservative amino acid substitutions.

In further specific embodiments, the number of substitutions, additions or deletions in the amino acid sequence of SEQ ID NO:2, SEQ ID NO:9, and SEQ ID NO:27, a polypeptide sequence encoded by the deposited clones, and/or any of the polypeptide fragments described herein (e.g., the extracellular domain or intracellular domain) is 75, 70, 60, 50, 40, 35, 30, 25, 20, 15, 10, 9, 8, 7, 6, 5, 4, 3, 2, 1 or 150-50, 100-50, 50-20, 30-20, 20-15, 20-10, 15-10, 10-1, 5-10, 1-5, 1-3 or 1-2.

To improve or alter the characteristics of VEGI polypeptides, protein engineering may be employed. Recombinant DNA technology known to those skilled in the art can be used to create novel mutant proteins or muteins including single or multiple amino acid substitutions, deletions, additions or fusion proteins. Such modified polypeptides can show, e.g., enhanced activity or increased stability. In addition, they may be purified in higher yields and show better solubility than the corresponding natural polypeptide, at least under certain purification and storage conditions.

Thus, the invention also encompasses VEGI derivatives and analogs that have one or more amino acid residues deleted, added, or substituted to generate VEGI polypeptides that are better suited for expression, scale up, etc., in the host cells chosen. For example, cysteine residues can be deleted or substituted with another amino acid residue in order to eliminate disulfide bridges; N-linked glycosylation sites can be altered or eliminated to acheive, for example, expression of a homogeneous product that is more easily recovered and purified from yeast hosts which are known to hyperglycosylate N-linked sites. To this end, a variety of amino acid substitutions at one or both of the first or third amino acid positions on any one or more of the glycosylation recognitions sequences in the VEGI polypeptides of the invention, and/or an amino acid deletion at the second position of any one or more such recognition sequences will prevent glycosylation of the VEGI polypeptide at the modified tripeptide sequence (see, e.g., Miyajimo et al., EMBO J 5(6):1193-1197).

Amino acids in the VEGI polypeptides of the present invention that are essential for function can be identified by methods known in the art, such as site-directed mutagenesis or alanine-scanning mutagenesis (Cunningham and Wells, Science 244:1081-1085 (1989)). The latter procedure introduces single alanine mutations at every residue in the molecule. The resulting mutant molecules are then tested for biological activity such as receptor binding *or in vitro* proliferative activity.

Of special interest are substitutions of charged amino acids with other charged or neutral amino acids which may produce proteins with highly desirable improved characteristics, such as less aggregation. Aggregation may not only reduce activity but also be problematic when preparing pharmaceutical formulations, because aggregates can be immunogenic (Pinckard, et al., Clin. Exp. Immunol. 2:331-340 (1967); Robbins, et al., Diabetes 36:838-845 (1987); Cleland, et al., Crit. Rev. Therapeutic Drug Carrier Systems 10:307-377 (1993)).

Since VEGI is a member of the TNF-related protein family, to modulate rather than completely eliminate biological activities of VEGI preferably additions, substitutions, or deletions are made in sequences encoding amino acids in the conserved TNF-like domain, i.e., in positions 17-147 of SEQ ID NO:9 or positions 121-251 of SEQ ID NO:27, more preferably in residues within this region which are not conserved in all members of the VEGI-related polypeptide family. Also forming part of the present invention are isolated polynucleotides comprising nucleic acid sequences which encode the above VEGI variants.

Several amino acids of the VEGI polypeptide are highly conserved across the known members of the TNF-related protein family. By making a specific mutation in VEGI in such residues as tyrosine-15 (as numbered in SEQ ID NO:9), leucine-35, glycine-41, tyrosine-43, tyrosine-46, glutamine-48, leucine-90, leucine-116, glycine-119, aspartic acid-120, phenylalanine-141, phenylalanine-142, and leucine-147, it is likely that an noticeable effect on biological activity will be observed. These identical amino acid residues are, of course, present in the corresponding positions of VEGI-beta shown in SEQ ID NO:27.

The present invention also encompasses fragments of the above-described VEGI polypeptides. Polypeptide fragments of the present invention include polypeptides comprising an amino acid sequence contained in SEQ ID NO:2 and SEQ ID NO:9, encoded by the cDNA contained in the deposited clone (HUVEO91), or encoded by nucleic acids which hybridize (e.g. under stringent hybridization conditions) to the nucleotide sequence contained in the deposited clones, that disclosed in SEQ ID NO:2, SEQ ID NO:8 and/or SEQ ID NO:26, or the complementary strand thereto.

Polypeptide fragments may be "free-standing" or comprised within a larger polypeptide of which the fragment forms a part or region, most preferably as a single continuous region. Representative examples of polypeptide fragments of the invention, included, for example, fragmens that comprise or alternatively, consist of, from about amino acid residues, 1 to 20, 21 to 40, 41 to 60, 61 to 83, 84 to 100, 101 to 120, 121 to 140, 141 to 160, 160 to 167, 161 to 174,161 to 180, 181 to 200, 201 to 220, 221 to 240, 241 to 251 of SEQ ID NO:9 and/or SEQ ID NO:27. Moreover, polypeptide fragments can be at least about 20, 30, 40, 50, 60, 70, 80, 90, 100, 110, 120, 130, 140 or 150 amino acids in length. In this context "about" includes the particularly recited ranges, larger or smaller by several (i.e. 5, 4, 3, 2 or 1) amino acids, at either extreme or at both extremes.

In other embodiments, the fragments or polypeptides of the invention (i.e., those described herein) are not larger than 250, 225, 200, 185, 175, 170, 165, 160, 155, 150, 145, 140, 135, 130, 125, 120, 115, 110, 105, 100, 90, 80, 75, 60, 50, 40, 30 or 25 amino acids residues in length.

Further preferred embodiments encompass polypeptide fragments comprising, or alternatively consisting of, the mature domain of VEGI (amino acid residues 1-147 of SEQ ID NO:9), the intracellular domain of VEGI-beta (amino acid residues 1-35 of SEQ ID NO:27), the transmembrane domain of VEGI-beta (amino acid residues 36-61 of SEQ ID NO:27), and/or the extracellular domain of VEGI-beta (amino acid residues 62-251 of SEQ ID NO:27).

In specific embodiments, polypeptide fragments of the invention comprise, or alternatively, consist of, amino acid residues leucine-35 to valine-49, tryptophan-104 to leucine-116, glycine-119 to serine-127, lysine-139 to leucine-147 of SEQ ID NO:9). These domains are regions of high identity identified by comparison of the VEGI family member polypeptides.

Among the especially preferred fragments of the invention are fragments characterized by structural or functional attributes of VEGI. Such fragments include amino acid residues that comprise alpha-helix and alphahelix forming regions ("alpha-regions"), beta-sheet and beta-sheet-forming regions ("beta-regions"), turn and turn-forming regions ("turn-regions"), coil and coil-forming regions ("coil-regions"), hydrophillic regions, hydrophobic regions, alpha amphipathic regions, beta amphipathic regions, surface forming regions, and high antigenic index regions (i.e., regions of polypeptides consisting of amino acid residues having an antigenic index of or equal to greater than 1.5, as identified using the default parameters of the Jameson-Wolf program) of VEGI. Certain preferred regions are those disclosed in Figure 7 and include, but are not limited to, regions of the aforementioned types identified by analysis of the amino acid sequence depicted in in Figures 5A, 5B, and 5C and SEQ ID NO:2 and SEQ ID NO:9, such preferred regions include; Garnier-Robson predicted alpha-regions, beta-regions, turn-regions, and coil-regions; Chou-Fasman predicted alpha-regions, beta-regions, turn-regions, and coil-regions; Kyte-Doolittle predicted hydrophilic and hydrophobic regions; Eisenberg alpha and beta amphipathic regions; Emini surface-forming regions; and Jameson-Wolf high antigenic index regions, as predicted using the default parameters of these computer programs. Polynucleotides encoding these polypeptides are also encompassed by the invention.

Additionally, analogs of the invention include a proprotein which can be activated by cleavage of the proprotein portion to produce an active mature polypeptide.

In another embodiment, the invention provides a VEGI polypeptide (e.g., fragment) comprising, or alternatively, consisting of, an epitope-bearing portion of a polypeptide of the invention. The epitope of this polypeptide portion is an immunogenic or antigenic epitope of a polypeptide of the invention. An "immunogenic epitope" is defined as a part of a protein that elicits an antibody response when the whole protein is the immunogen. On the other hand, a region of a protein molecule to which an antibody can bind is defined as an "antigenic epitope". The number of immunogenic epitopes of a protein generally is less than the number of antigenic epitopes (see, for instance, Geysen, et al., Proc. Natl. Acad. Sci. USA 81:3998-4002 (1983)).

As to the selection of peptides or polypeptides bearing an antigenic epitope (i.e., that contain a region of a protein molecule to which an antibody can bind), it is well known in that art that relatively short synthetic peptides that mimic part of a protein sequence are routinely capable of eliciting an antiserum that reacts with the partially mimicked protein (see, for instance, Sutcliffe, J. G., et aL, Science 219:660-666 (1983)). Peptides capable of eliciting protein-reactive sera are frequently represented in the primary sequence of a protein, can be characterized by a set of simple chemical rules, and are confined neither to immunodominant regions of intact proteins (i.e., immunogenic epitopes) nor to the amino or carboxyl termini. Antigenic epitope-bearing peptides and polypeptides of the invention are therefore useful to raise antibodies, including monoclonal antibodies, that bind specifically to a polypeptide of the invention (see, for instance, Wilson, et aL, Cell 37:767-778 (1984)).

Antigenic epitope-bearing peptides and polypeptides of the invention preferably contain a sequence of at least seven, more preferably at least nine and most preferably between about 15 to about 30 amino acids contained within the amino acid sequence of a polypeptide of the invention. Nonlimiting examples of antigenic polypeptides or peptides that can be used to generate VEGI-specific antibodies include: a polypeptide comprising amino acid residues from about Thr-24 to about Asn-32 in SEQ ID NO:9; a polypeptide comprising amino acid residues from about Ile-37 to about Ile-45 in SEQ ID NO:9; a polypeptide comprising amino acid residues from about Met-54 to about Arg-62 in SEQ ID NO:9; a polypeptide comprising amino acid residues from about Gln-63 to about Asp-71 in SEQ ID NO:9; a polypeptide comprising amino acid residues from about Glu-57 to about Gly-65 in SEQ ID NO:9; a polypeptide comprising amino acid residues from about Val-80 to about Thr-88 in SEQ ID NO:9; a polypeptide comprising amino acid residues from about Leu-116 to about Val-124 in SEQ ID NO:9; and a polypeptide comprising amino acid residues from about Asp-133 to about Phe-141 in SEQ ID NO:9. These polypeptide fragments have been determined to bear antigemc epitopes of the VEGI polypeptide by the analysis of the Jameson-Wolf antigenic index, as shown in Figure 7, above.

One of ordinary skill in the art may easily determine antigenic regions for VEGI-beta by using data prepared through a DNA*STAR analysis of the VEGI-beta polypeptide sequence (SEQ ID NO:27) using the default parameters and selecting regions with a high antigenic index as described above.

The epitope-bearing peptides and polypeptides of the produced by any conventional means (see, for example, Houghten, R. A., et aL, Proc. Natl. Acad Sci. USA 82:5131-5135 (1985); and U.S. Patent No. 4,631,211 to Houghten, *et al.* (1986)).

Epitope-bearing peptides and polypeptides of the invention invention. have uses which include, but are not limited to, inducing antibodies according to methods well known in the art (see, for instance, Sutcliffe, *et aL, supra; Wilson, et aL, supra;* Chow, M., et al., Proc. NatL Acad Sci. USA 82:910-914; and Bittle, F. J., et aL, J Gen. ViroL 66:2347-2354 (1985)). Immunogenic epitope-bearing peptides of the invention, i.e., those parts of a protein that elicit an antibody response when the whole protein is the immunogen, are identified according to methods known in the art (see, for instance, *Geysen, et aL, supra).* Further still, U.S. Patent No. 5,194,392, issued to Geysen, describes a general method of detecting or determining the sequence of monomers (amino acids or other compounds) which is a topological equivalent of the epitope (i.e., a "mimotope") which is complementary to a particular paratope (antigen binding site) of an antibody of interest. More generally, U.S. Patent No. 4,433,092, issued to Geysen, describes a method of detecting or determining a sequence of monomers which is a topographical equivalent of a ligand which is complementary to the ligand binding site of a particular receptor of interest (e.g. DR3). Similarly, U.S. Patent No. 5,480,971, issued to Houghten and colleagues, on Peralkylated Oligopeptide Mixtures discloses linear C1-C7-alkyl peralkylated oligopeptides and sets and libraries of such peptides, as well as methods for using such oligopeptide sets and libraries for determining the sequence of a peralkylated oligopeptide that preferentially binds to an acceptor molecule of interest. Thus, non-peptide analogs of the epitope-bearing peptides of the invention also can be made routinely by these methods.

As one of skill in the art will appreciate, VEGI and/or VEGI-beta polypeptides of the present invention and the epitope-bearing fragments thereof described above can be combined with parts of the constant domain of immunoglobulins (IgG), resulting in chimeric polypeptides. These fusion proteins facilitate purification and show an increased half-life *in vivo.* This has been shown, e.g., for chimeric proteins consisting of the first two domains of the human CD4-polypeptide and various domains of the constant regions of the heavy or light chains of mammalian immunoglobulins (EP A 394,827; Traunecker, et al., Nature 331:84-86 (1988)). Fusion proteins that have a disulfide-linked dimeric structure due to the IgG part can also be more efficient in binding and neutralizing other molecules than the monomeric VEGI polypeptide or protein fragment alone (Fountoulakis, et al., J. Biochem. 270:3958-3964 (1995)). As an example, one such VEGI-Fc fusion has been produced herein as described above.

Fragments (i.e., portions) of the VEGI polypeptides of the present invention have uses which include, but are not limited to, intermediates for producing full-length polypeptides.

For many proteins, including the extracellular domain of a membrane associated protein or the mature form(s) of a secreted protein, it is known in the art that one or more amino acids may be deleted from the N-terminus or C-terminus without substantial loss of biological function. For instance, Ron and colleagues (J. Biol. Chem., 268:2984-2988 (1993)) reported modified KGF proteins that had heparin binding activity even if 3, 8, or 27 N-terminal amino acid residues were missing. Further, several investigators have reported TNF-α muteins in which two, four or seven N-terminal amino acids had been removed which showed a 2- to 3-fold increase in functional acitivity when compared to the naturally-occurring TNF-α polypeptide (Creasey, A. A., et al., Cancer Res. 47:145-149 (1987); Sidhu, R. S. and Bollon, A. P. Anticancer Res. 9:1569-1576 (1989); Kamijo, R., et al., Biochem. Biophys. Res. Comm. 160:820-827 (1989)). Further, even if deletion of one or more amino acids from the N-terminus or C-terminus of a protein results in modification or loss of one or more biological functions of the protein, other VEGI functional activities may still be retained

In the present case, since the proteins of the invention are members of the TNF polypeptide family, deletions of N-terminal amino acids up to the leucine residue at position 35 of SEQ ID NO:9 (which corresponds exactly to the leucine residue at position 134 of SEQ ID NO:27) may retain some biological activity such as regulation of growth and differentiation of many types of hematopoietic and endothelial cells. Polypeptides having further N-terminal deletions including the leucine-36 residue in SEQ ID NO:9 (corresponding to leucine-135 in SEQ ID NO:27) would not be expected to retain such biological activities because it is known that this residue in TNF-related polypeptides is in the beginning of the conserved domain required for biological activities.

However, even if deletion of one or more amino acids from the N-terminus of a full-length VEGI polypeptide results in modification or loss of one or more biological functions of the polypeptide, other biological activities may still be retained. Thus, the ability of the shortened polypeptide to induce and/or bind to antibodies which recognize the full-length or mature form of the polypeptide generally will be retained when less than the majority of the residues of the full-length or mature polypeptide are removed from the N-terminus. Whether a particular polypeptide lacking N-terminal residues of a complete polypeptide retains such immunologic activities can readily be determined by routine methods described herein and otherwise known in the art.

Accordingly, the present invention further provides polypeptides having one or more residues deleted from the amino terminus of the amino acid sequence of the VEGI disclosed in SEQ ID NO:9, up to the leucine residue at position number 35, and polynucleotides encoding such polypeptides. In particular, the present invention provides polypeptides comprising the amino acid sequence of residues n¹-149 of SEQ ID NO:9, where n¹ is an integer in the range of-27 to 35, and 35 is the position of the first residue from the N-terminus of the complete VEGI polypeptide (shown in SEQ ID NO:9) believed to be required for regulation of growth and differentiation of many types of hematopoietic and endothelial cells.

In specific embodiments, the invention provides polynucleotides encoding polypeptides comprising, or alternatively, consisting of, the amino acid sequence of residues: -27 to 147, -26 to 147, -25 to 147, -24 to 147, -23 to 147, -22 to 147, -21 to 147, -20 to 147, -19 to 147, -18 to 147, -17 to 147, -16 to 147,-15 to 147, -14 to 147, -13 to 147, -12 to 147, -11 to 147, -10 to 147, -9 to 147, -8 to 147, -7 to 147, -6 to 147, -5 to 147, -4 to 147, -3 to 147, -2 to 147, -1 to 147, 1 to 147, 2 to 147, 3 to 147, 4 to 147, 5 to 147, 6 to 147, 7 to 147, 8 to 147, 9 to 147, 10 to 147, 11 to 147, 12 to 147, 13 to 147, 14 to 147, 15 to 147, 16 to 147, 17 to 147, 18 to 147, 19 to 147, 20 to 147, 21 to 147, 22 to 147, 23 to 147, 24 to 147, 27 to 147, 26 to 147, 27 to 147, 28 to 147, 29 to 147, 30 to 147, 31 to 147, 32 to 147, 33 to 147, 34 to 147, and 35 to 147 of SEQ ID NO:9. Polynucleotides encoding these polypeptides are also encompassed by the invention.

Accordingly, the present invention further provides polypeptides having one or more residues deleted from the amino terminus of the amino acid sequence of the VEGI-beta shown in SEQ ID NO:27, up to the leucine residue at position number 134, and polynucleotides encoding such polypeptides. In particular, the present invention provides polypeptides comprising the amino acid sequence of residues n²-251 of SEQ ID NO:27, where n² is an integer in the range of 1 to 134, and 135 is the position of the first residue from the N-terminus of the complete VEGI-beta polypeptide (shown in SEQ ID NO:27) believed to be required for regulation of growth and differentiation of many types of hematopoietic and endothelial cells activity of the VEGI-beta polypeptide.

In specific embodiments, the invention provides polynucleotides encoding polypeptides comprising, or alternatively, consisting of, the amino acid sequence of residues: 1 to 251, 2 to 251, 3 to 251, 4 to 251, 5 to 251, 6 to 251, 7 to 251, 8 to 251, 9 to 251, 10 to 251,11 to 251, 12 to 251, 13 to 251, 14 to 251, 15 to 251, 16 to 251, 17 to 251, 18 to 251, 19 to 251, 20 to 251, 21 to251,22to251,23to251,24to251,25to251,26to251,27to 251,28to251,29to251,30to251,31to251,32to251,33to251,34to 251,35to251,36to251,37to251,38to251,39to251,40to251,41 to 251, 41 to 251, 42 to 251, 43 to 251, 44 to 251, 45 to 251, 46 to 251, 47 to 251, 48 to 251, 49 to 251, 50 to 251, 51 to 251, 52 to 251, 53 to 251, 54 to 251, 55 to 251, 56 to 251, 57 to 251, 58 to 251, 59 to 251, 60 to 251, 61 to 251, 62 to 251, 63 to 251, 64 to 251, 65 to 251, 66 to 251, 67 to 251, 68 to 251, 69 to 251, 70 to 251, 71 to 251, 72 to 251, 73 to 251, 74 to 251, 75 to 251,76to251,77to251,78to251,79to251,80to251,81to251,82to 251, 83 to 251, 84 to 251, 85 to 251, 86 to 251, 87 to 251, 88 to 251, 89 to 251, 90 to 251, 91 to 251, 92 to 251, 93 to 251, 94 to 251, 95 to 251, 96 to 251, 97 to 251, 98 to 251, 99 to 251, 100 to 251, 101 to 251, 102 to 251, 103 to 251, 104 to 251, 105 to 251, 106 to 251, 107 to 251, 108 to 251, 109 to 251, 110 to 251, 111 to 251, 112 to 251, 113 to 251, 114 to 251, 115 to 251, 116 to 251, 117 to 251, 118 to 251, 119 to 251, 120 to 251, 121 to 251, 122 to 251, 123 to 251, 124 to 251, 125 to 251, 126 to 251, 127 to 251, 128 to 251, 129 to 251, 130 to 251, 131 to 251, 133 to 251, 134 to 251, and 134 to 251 of SEQ ID NO:27. Polynucleotides encoding these polypeptides are also encompassed by the invention.

As mentioned above, even if deletion of one or more amino acids from the N-terminus of a polypeptide results in modification of loss of one or more biological functions of the polypeptide, other biological activities may still be retained. Thus, the ability of the shortened VEGI mutein to induce and/or bind to antibodies which recognize the full-length or mature form of the polypeptide generally will be retained when less than the majority of the residues of the full-length or mature polypeptide are removed from the N-terminus. Whether a particular polypeptide lacking N-terminal residues of a complete protein retains such immunologic activities can readily be determined by routine methods described herein and otherwise known in the art. It is not unlikely that a VEGI mutein with a large number of deleted N-terminal amino acid residues may retain some biological or immungenic activities. In fact, peptides composed of as few as six VEGI amino acid residues may often evoke an immune response.

Accordingly, the present invention further provides polypeptides having one or more residues deleted from the amino terminus of the predicted mature amino acid sequence of the VEGI disclosed in SEQ ID NO:9, up to the phenylalanine residue at position number 142 of SEQ ID NO:9 and polynucleotides encoding such polypeptides. In particular, the present invention provides polypeptides comprising the amino acid sequence of residues n³-147 of SEQ ID NO:9, where n³ is an integer in the range of 1 to 169, and 170 is the position of the first residue from the N-terminus of the complete VEGI polypeptide believed to be required for at least immunogenic activity of the VEGI polypeptide.

More in particular, the invention provides polynucleotides encoding polypeptides comprising, or alternatively consisting of, the amino acid sequence of residues of R-2 to L-174; R-3 to L-174; F-4 to L-174; L-5 to L-174; S-6 to L-174; K-7 to L-174; V-8 to L-174; Y-9 to L-174; S-10 to L-174: F-11 to L-174; P-12 to L-174; M-13 to L-174; R-14 to L-174; K-15 to L-174; L-16 to L-174; I-17 to L-174; L-18 to L-174; F-19 to L-174; L-20 to L-174; V-21 to L-174; F-22 to L-174; P-23 to L-174; V-24 to L-174; V-25 to L-174; R-26 to L-174; Q-27 to L-174; T-28 to L-174; P-29 to L-174; T-30 to L-174; Q-31 to L-174; H-32 to L-174; F-33 to L-174; K-34 to L-174; N-35 to L-174; Q-36 to L-174; F-37 to L-174; P-38 to L-174; A-39 to L-174; L-40 to L-174; H-41 to L-174; W-42 to L-174; E-43 to L-174; H-44 to L-174; E-45 to L-174; L-46 to L-174; G-47 to L-174; L-48 to L-174; A-49 to L-174; F-50 to L-174; T-51 to L-174; K-52 to L-174; N-53 to L-174; R-54 to L-174; M-55 to L-174; N-56 to L-174; Y-57 to L-174; T-58 to L-174; N-59 to L-174; K-60 to L-174; F-61 to L-174; L-62 to L-174; L-63 to L-174; I-64 to L-174; P-65 to L-174; E-66 to L-174; S-67 to L-174; G-68 to L-174; D-69 to L-174; Y-70 to L-174; F-71 to L-174; I-72 to L-174; Y-73 to L-174; S-74 to L-174; Q-75 to L-174; V-76 to L-174; T-77 to L-174; F-78 to L-174; R-79 to L-174; G-80 to L-174; M-81 to L-174; T-82 to L-174; S-83 to L-174; E-84 to L-174; C-85 to L-174; S-86 to L-174; E-87 to L-174; I-88 to L-174; R-89 to L-174; Q-90 to L-174; A-91 to L-174; G-92 to L-174; R-93 to L-174; P-94 to L-174; N-95 to L-174; K-96 to L-174; P-97 to L-174; D-98 to L-174; S-99 to L-174; I-100 to L-174; T-101 to L-174; V-102 to L-174; V-103 to L-174; I-104 to L-174; T-105 to L-174; K-106 to L-174; V-107 to L-174; T-108 to L-174; D-109 to L-174; S-110 to L-174; Y-111 to L-174; P-112 to L-174; E-113 to L-174; P-114 to L-174; T-115 to L-174; Q-116 to L-174; L-117 to L-174; L-118 to L-174; M-119 to L-174; G-120 to L-174; T-121 to L-174; K-122 to L-174; S-123 to L-174; V-124 to L-174; C-125 to L-174; E-126 to L-174; V-127 to L-174; G-128 to L-174; S-129 to L-174; N-130 to L-174; W-131 to L-174; F-132 to L-174; Q-133 to L-174; P-134 to L-174; I-135 to L-174; Y-136 to L-174; L-137 to L-174; G-138 to L-174; A-139 to L-174; M-140 to L-174; F-141 to L-174; S-142 to L-174; L-143 to L-174; Q-144 to L-174; E-145 to L-174; G-146 to L-174; D-147 to L-174; K-148 to L-174; L-149 to L-174; M-150 to L-174; V-151 to L-174; N-152 to L-174; V-153 to L-174; S-154 to L-174; D-155 to L-174; I-156 to L-174; S-157 to L-174; L-158 to L-174; V-159 to L-174: D-160 to L-174; Y-161 to L-174; T-162 to L-174; K-163 to L-174; E-164 to L-174; D-165 to L-174; K-166 to L-174; T-167 to L-174; F-168 to L-174; and F-169 to L-174 of the VEGI sequence shown in Figures.5A, 5B, and 5C (the VEGI amino acid sequence shown in Figures 5A, 5B, and 5C is identical to that in SEQ ID NO:9, however, the numbering scheme differs between the two; the numbering of the above amino acid residues in this case reflects that of Figures 5A, 5B, and 5C). Polynucleotides encoding these polypeptides are also encompassed by the invention.

Accordingly, the present invention further provides polypeptides having one or more residues deleted from the amino terminus of the predicted mature amino acid sequence of the VEGI-beta shown in SEQ ID NO:27, up to the phenylalanine residue at position number 246 and polynucleotides encoding such polypeptides. In particular, the present invention provides polypeptides comprising the amino acid sequence of residues n⁴-251 of SEQ ID NO:27, where n⁴ is an integer in the range of 2 to 246, and 247 is the position of the first residue from the N-terminus of the complete VEGI-beta polypeptide believed to be required for at least immunogenic activity of the VEGI-beta protein.

More in particular, the invention provides polynucleotides encoding polypeptides comprising, or alternatively consisting of, the amino acid sequence of residues of A-2 to L-251; E-3 to L-251; D-4 to L-251; L-5. to L-251; G-6 to L-251; L-7 to L-251; S-8 to L-251; F-9 to L-251; G-10 to L-251; E-11 to L-251; T-12 to L-251; A-13 to L-251; S-14 to L-251; V-15 to L-251; E-16 to L-251; M-17 to L-251; L-18 to L-251; P-19 to L-251; E-20 to L-251; H-21 to L-251; G-22 to L-251; S-23 to L-251; C-24 to L-251; R-25 to L-251; P-26 to L-251; K-27 to L-251; A-28 to L-251; R-29 to L-251; S-30 to L-251; S-31 to L-251; S-32 to L-251; A-33 to L-251; R-34 to L-251; W-35 to L-251; A-36 to L-251; L-37 to L-251; T-38 to L-251; C-39 to L-251; C-40 to L-251; L-41 to L-251; V-42 to L-251; L-43 to L-251; L-44 to L-251; P-45 to L-251; F-46 to L-251; L-47 to L-251; A-48 to L-251; G-49 to L-251; L-50 to L-251; T-51 to L-251; T-52 to L-251; Y-53 to L-251; L-54 to L-251; L-55 to L-251; V-56 to L-251; S-57 to L-251; Q-58 to L-251; L-59 to L-251; R-60 to L-251; A-61 to L-251; Q-62 to L-251; G-63 to L-251; E-64 to L-251; A-65 to L-251; C-66 to L-251; V-67 to L-251; Q-68 to L-251; F-69 to L-251; Q-70 to L-251; A-71 to L-251; L-72 to L-251; K-73 to L-251; G-74 to L-251; Q-75 to L-251; E-76 to L-251; F-77 to L-251; A-78 to L-251; P-79 to L-251; S-80 to L-251; H-81 to L-251; Q-82 to L-251; Q-83 to L-251; V-84 to L-251; Y-85 to L-251; A-86 to L-251; P-87 to L-251; L-88 to L-251; R-89 to L-251; A-90 to L-251; D-91 to L-251; G-92 to L-251; D-93 to L-251; K-94 to L-251; P-95 to L-251; R-96 to L-251; A-97 to L-251; H-98 to L-251; L-99 to L-251; T-100 to L-251; V-101 to L-251; V-102 to L-251; R-103 to L-251; Q-104 to L-251; T-105 to L-251; P-106 to L-251; T-107 to L-251; Q-108 to L-251; H-109 to L-251; F-110 to L-251; K-111 to L-251; N-112 to L-251; Q-113 to L-251; F-114 to L-251; P-115 to L-251; A-116 to L-251; L-117 to L-251; H-118 to L-251; W-119 to L-251; E-120 to L-251; H-121 to L-251; E-122 to L-251; L-123 to L-251; G-124 to L-251; L-125 to L-251; A-126 to L-251; F-127 to L-251; T-128 to L-251; K-129 to L-251; N-130 to L-251; R-131 to L-251; M-132 to L-251; N-133 to L-251; Y-134 to L-251; T-135 to L-251; N-136 to L-251; K-137 to L-251; F-138 to L-251; L-139 to L-251; L-140 to L-251; I-141 to L-251; P-142 to L-251; E-143 to L-251; S-144 to L-251; G-145 to L-251; D-146 to L-251; Y-147 to L-251; F-148 to L-251; 1-149 to L-251; Y-150 to L-251; S-151 to L-251; Q-152 to L-251; V-153 to L-251; T-154 to L-251; F-155 to L-251; R-156 to L-251; G-157 to L-251; M-158 to L-251; T-159 to L-251; S-160 to L-251; E-161 to L-251; C-162 to L-251; S-163 to L-251; E-164 to L-251; I-165 to L-251; R-166 to L-251; Q-167 to L-251; A-168 to L-251; G-169 to L-251; R-170 to L-251; P-171 to L-251; N-172 to L-251; K-173 to L-251; P-174 to L-251; D-175 to L-251; S-176 to L-251; I-177 to L-251; T-178 to L-251; V-179 to L-251; V-180 to L-251; I-181 to L-251; T-182 to L-251; K-183 to L-251; V-184 to L-251; T-185 to L-251; D-186 to L-251; S-187 to L-251; Y-188 to L-251; P-189 to L-251; E-190 to L-251; P-191 to L-251; T-192 to L-251; Q-193 to L-251; L-194 to L-251; L-195 to L-251; M-196 to L-251; G-197 to L-251; T-198 to L-251; K-199 to L-251; S-200 to L-251; V-201 to L-251; C-202 to L-251; . E-203 to L-251; V-204 to L-251; G-205 to L-251; S-206 to L-251; N-207 to L-251; W-208 to L-251; F-209 to L-251; Q-210 to L-251; P-211 to L-251; I-212 to L-251; Y-213 to L-251; L-214 to L-251; G-215 to L-251; A-216 to L-251; M-217 to L-251; F-218 to L-251; S-219 to L-251; L-220 to L-251; Q-221 to L-251; E-222 to L-251; G-223 to L-251; D-224 to L-251; K-225 to L-251; L-226 to L-251; M-227 to L-251; V-228 to L-251; N-229 to L-251; V-230 to L-251; S-231 to L-251; D-232 to L-251; I-233 to L-251; S-234 to L-251; L-235 to L-251; V-236 to L-251; D-237 to L-251; Y-238 to L-251; T-239 to L-251; K-240 to L-251; E-241 to L-251; D-242 to L-251; K-243 to L-251; T-244 to L-251; F-245 to L-251; and F-246 to L-251 of the VEGI-beta sequence shown in SEQ ID NO:27. Polynucleotides encoding these polypeptides are also encompassed by the invention.

Similarly, many examples of biologically functional C-terminal deletion muteins are known. For instance, Interferon gamma shows up to ten times higher activities by deleting 8 to 10 amino acid residues from the carboxy terminus of the protein (Dobeli, et al., J. Biotechnology 7:199-216 (1988)). Further, several investigators have reported biologically inactive TNF-α muteins in which as few as two amino acids had been removed from the C-terminus (Carlino, J. A., et al., J Biol. Chem. 262:958-961 (1987); Creasey, A. A., et al., Cancer Res. 47:145-149 (1987); Sidhu, R. S. and Bollon, A. P. Anticancer Res. 9:1569-1576 (1989); Gase, K., et al., Immunology 71:368-371 (1990)).

In the present case, since the proteins of the invention are members of the TNF polypeptide family, deletions of C-terminal amino acids up to the leucine at position 146 of SEQ ID NO:9 (which corresponds to the leucine at position 250 of SEQ ID NO:27) may retain some biological activity such as regulation of growth and differentiation of many types of hematopoietic and endothelial cells. Polypeptides having further C-terminal deletions including the leucine residue at position 146 of SEQ ID NO:9 (or the leucine residue at position 250 of SEQ ID NO:27) would not be expected to retain such biological activities because it is known that this residue in TNF-related polypeptides is in the beginning of the conserved domain required for biological activities.

However, even if deletion of one or more amino acids from the C-terminus of a protein results in modification of loss of one or more biological functions of the protein, other biological activities may still be retained. Thus, the ability of the shortened protein to induce and/or bind to antibodies which recognize the complete or mature form of the protein generally will be retained when less than the majority of the residues of the complete or mature protein are removed from the C-terminus. Whether a particular polypeptide lacking C-terminal residues of a complete protein retains such immunologic activities can readily be determined by routine methods described herein and otherwise known in the art.

In additional embodiments, the present invention further provides polypeptides having one or more residues removed from the carboxy terminus of the amino acid sequence of the VEGI polypeptide shown in SEQ ID NO:9, up to the leucine residue at position 146 of SEQ ID NO:9, and polynucleotides encoding such polypeptides. In particular, the present invention provides polypeptides having the amino acid sequence of residues -27-m¹ of the amino acid sequence in SEQ ID NO:9, where m¹ is any integer in the range of 146 to 147, and residue 146 is the position of the first residue from the C- terminus of the complete VEGI polypeptide (shown in SEQ ID NO:9) believed to be required for regulation of growth and differentiation of many types of hematopoietic and endothelial cells by the VEGI polypeptide.

More in particular, the invention provides polynucleotides encoding polypeptides having the amino acid sequence of residues -27-146 and -27-147 of SEQ ID NO:9. Polynucleotides encoding these polypeptides also are provided.

The present invention also provides polypeptides having one or more residues removed from the carboxy terminus of the amino acid sequence of the VEGI-beta shown in SEQ ID NO:27, up to the leucine residue at position 250 of SEQ ID NO:27, and polynucleotides encoding such polypeptides. In particular, the present invention provides polypeptides having the amino acid sequence of residues 1-m² of the amino acid sequence in SEQ ID NO:27, where m² is any integer in the range of 250 to 251, and residue 249 is the position of the first residue from the C- terminus of the complete VEGI-beta polypeptide (shown in SEQ ID NO:27) believed to be required for regulation of growth and differentiation of many types of hematopoietic and endothelial cells.

More in particular, the invention provides polynucleotides encoding polypeptides having the amino acid sequence of residues 1-250 and 1-251 of SEQ ID NO:27. Polynucleotides encoding these polypeptides also are provided.

The invention also provides polypeptide fragments comprising, or alternatively consisting of, one or more amino acids deleted from both the amino and the carboxyl termini of VEGI-alpha, which may be described generally as having residues n¹-m¹ of SEQ ID NO:9, where n and m are integers as described above. The invention further provides polypeptides having one or more amino acids deleted from both the amino and the carboxyl termini of VEGI-beta, which may be described generally as having residues n²-m² of SEQ ID NO:27, where n² and m² are integers as described above.

As mentioned above, even if deletion of one or more amino acids from the C-terminus of a polypeptide results in modification of loss of one or more biological functions of the polypeptide, other biological activities may still be retained. Thus, the ability of the shortened VEGI mutein to induce and/or bind to antibodies which recognize the full-length or mature of the polypeptide generally will be retained when less than the majority of the residues of the complete or mature polypeptide are removed from the C-terminus. Whether a particular polypeptide lacking C-terminal residues of a full-length polypeptide retains such immunologic activities can readily be determined by routine methods described herein and otherwise known in the art. It is not unlikely that a VEGI mutein with a large number of deleted C-terminal amino acid residues may retain some biological or immungenic activities. In fact, peptides composed of as few as six VEGI amino acid residues may often evoke an immune response.

Accordingly, the present invention further provides polypeptides having one or more residues deleted from the carboxy terminus of the amino acid sequence of the VEGI shown in Figures 5A, 5B, and 5C (or in SEQ ID NO:9), up to the serine residue at position number 6 in Figures 5A, 5B, and 5C (or -22 in SEQ ID NO:9), and polynucleotides encoding such polypeptides. In particular, the present invention provides polypeptides comprising the amino acid sequence of residues 1-m³ of SEQ ID NO:9, where m³ is an integer in the range of 6 to 174, and 6 is the position of the first residue from the C-terminus of the complete VEGI polypeptide believed to be required for at least immunogenic activity of VEGI-alpha.

More in particular, the invention provides polynucleotides encoding polypeptides comprising, or alternatively consisting of, the amino acid sequence of residues M-1 to L-173; M-1 to F-172; M-1 to A-171; M-1 to G-170; M-1 to F-169; M-1 to F-168; M-1 to T-167; M-1 to K-166; M-1 to D-165; M-1 to E-164; M-1 to K-163; M-1 to T-162; M-1 to Y-161; M-1 to D-160; M-1 to V-159; M-1 to L-158; M-1 to S-157; M-1 to I-156; M-1 to D-155; M-1 to S-154; M-1 to V-153; M-1 to N-152; M-1 to V-151; M-1 to M-150; M-1 to L-149; M-1 to K-148; M-1 to D-147; M-1 to G-146; M-1 to E-145; M-1 to Q-144; M-1 to L-143; M-1 to S-142; M-1 to F-141; M-1 to M-140; M-1 to A-139; M-1 to G-138; M-1 to L-137; M-1 to Y-136; M-1 to I-135; M-1 to P-134; M-1 to Q-133; M-1 to F-132; M-1 to W-131; M-1 to N-130; M-1 to S-129; M-1 to G-128; M-1 to V-127; M-1 to E-126; M-1 to C-125; M-1 to V-124; M-1 to S-123; M-1 to K-122; M-1 to T-121; M-1 to G-120; M-1 to M-119; M-1 to L-118; M-1 to L-117; M-1 to Q-116; M-1 to T-115; M-1 to P-114; M-1 to E-113; M-1 to P-112; M-1 to Y-111; M-1 to S-110; M-1 to D-109; M-1 to T-108; M-1 to V-107; M-1 to K-106; M-1 to T-105; M-1 to I-104; M-1 to V-103; M-1 to V-102; M-1 to T-101; M-1 to I-100; M-1 to S-99; M-1 to D-98; M-1 to P-97; M-1 to K-96; M-1 to N-95; M-1 to P-94; M-1 to R-93; M-1 to G-92; M-1 to A-91; M-1 to Q-90; M-1 to R-89; M-1 to 1-88; M-1 to E-87; M-1 to S-86; M-1 to C-85; M-1 to E-84; M-1 to S-83; M-1 to T-82; M-1 to M-81; M-1 to G-80; M-1 to R-79; M-1 to F-78; M-1 to T-77; M-1 to V-76; M-1 to Q-75; M-1 to S-74; M-1 to Y-73; M-1 to 1-72; M-1 to F-71; M-1 to Y-70; M-1 to D-69; M-1 to G-68; M-1 to S-67; M-1 to E-66; M-1 to P-65; M-1 to 1-64; M-1 to L-63; M-1 to L-62; M-1 to F-61; M-1 to K-60; M-1 to N-59; M-1 to T-58; M-1 to Y-57; M-1 to N-56; M-1 to M-55; M-1 to R-54; M-1 to N-53; M-1 to K-52; M-1 to T-51; M-1 to F-50; M-1 to A-49; M-1 to L-48; M-1 to G-47; M-1 to L-46; M-1 to E-45; M-1 to H-44; M-1 to E-43; M-1 to W-42; M-1 to H-41; M-1 to L-40; M-1 to A-39; M-1 to P-38; M-1 to F-37; M-1 to Q-36; M-1 to N-35; M-1 to K-34; M-1 to F-33; M-1 to H-32; M-1 to Q-31; M-1 to T-30; M-1 to P-29; M-1 to T-28; M-1 to Q-27; M-1 to R-26; M-1 to V-25; M-1 to V-24; M-1 to P-23; M-1 to F-22; M-1 to V-21; M-1 to L-20; M-1 to F-19; M-1 to L-18; M-1 to I-17; M-1 to L-16; M-1 to K-15; M-1 to R-14; M-1 to M-13; M-1 to P-12; M-1 to F-11; M-1 to S-10; M-1 to Y-9; M-1 to V-8; M-1 to K-7; and M-1 to S-6 of the sequence of the VEGI sequence shown in Figures 5A, 5B, and 5C (the VEGI amino acid sequence shown in Figures 5A, 5B, and 5C is identical to that in SEQ ID NO:9, however, the numbering scheme differs between the two; the numbering of the above amino acid residues in this case reflects that of Figures 5A, 5B, and 5C). Polynucleotides encoding these polypeptides also are provided.

The invention also provides polypeptides having one or more amino acids deleted from both the amino and the carboxyl termini of a VEGI polypeptide, which may be described generally as having residues n³-m³ of SEQ ID NO:9, where n³ and m³ are integers as described above. Polynucleotides encoding the polypeptides are also encompassed by the invention.

The present invention further provides polypeptides having one or more residues deleted from the carboxy terminus of the amino acid sequence of the VEGI-beta shown in SEQ ID NO:27, up to the glycine residue at position number 6, and polynucleotides encoding such polypeptides. In particular, the present invention provides polypeptides comprising the amino acid sequence of residues 1-m⁴ of SEQ ID NO:27, where m⁴ is an integer in the range of 6 to 250, and 6 is the position of the first residue from the C-terminus of the complete VEGI-beta polypeptide believed to be required for at least immunogenic activity of the VEGI-beta protein.

More in particular, the invention provides polynucleotides encoding polypeptides comprising, or alternatively consisting of, the amino acid sequence of residues M-1 to L-250; M-1 to F-249; M-1 to A-248; M-1 to G-247; M-1 to F-246; M-1 to F-245; M-1 to T-244; M-1 to K-243; M-1 to D-242; M-1 to E-241; M-1 to K-240; M-1 to T-239; M-1 to Y-238; M-1 to D-237; M-1 to V-236; M-1 to L-235; M-1 to S-234; M-1 to I-233; M-1 to D-232; M-1 to S-231; M-1 to V-230; M-1 to N-229; M-1 to V-228; M-1 to M-227; M-1 to L-226; M-1 to K-225; M-1 to D-224; M-1 to G-223; M-1 to E-222; M-1 to Q-221; M-1 to L-220; M-1 to S-219; M-1 to F-218; M-1 to M-217; M-1 to A-216; M-1 to G-215; M-1 to L-214; M-1 to Y-213; M-1 to I-212; M-1 to P-211; M-1 to Q-210; M-1 to F-209; M-1 to W-208; M-1 to N-207; M-1 to S-206; M-1 to G-205; M-1 to V-204; M-1 to E-203; M-1 to C-202; M-1 to V-201; M-1 to S-200; M-1 to K-199; M-1 to T-198; M-1 to G-197; M-1 to M-196; M-1 to L-195; M-1 to L-194; M-1 to Q-193; M-1 to T-192; M-1 to P-191; M-1 to E-190; M-1 to P-189; M-1 to Y-188; M-1 to S-187; M-1 to D-186; M-1 to T-185; M-1 to V-184; M-1 to K-183; M-1 to T-182; M-1 to I-181; M-1 to V-180; M-1 to V-179; M-1 to T-178; M-1 to I-177; M-1 to S-176; M-1 to D-175; M-1 to P-174; M-1 to K-173; M-1 to N-172; M-1 to P-171; M-1 to R-170; M-1 to G-169; M-1 to A-168; M-1 to Q-167; M-1 to R-166; M-1 to I-165; M-1 to E-164; M-1 to S-163; M-1 to C-162; M-1 to E-161; M-1 to S-160; M-1 to T-159; M-1 to M-158; M-1 to G-157; M-1 to R-156; M-1 to F-155; M-1 to T-154; M-1 to V-153; M-1 to Q-152; M-1 to S-151; M-1 to Y-150; M-1 to I-149; M-1 to F-148; M-1 to Y-147; M-1 to D-146; M-1 to G-145; M-1 to S-144; M-1 to E-143; M-1 to P-142; M-1 to I-141; M-1 to L-140; M-1 to L-139; M-1 to F-138; M-1 to K-137; M-1 to N-136; M-1 to T-135; M-1 to Y-134; M-1 to N-133; M-1 to M-132; M-1 to R-131; M-1 to N-130; M-1 to K-129; M-1 to T-128; M-1 to F-127; M-1 to A-126; M-1 to L-125; M-1 to G-124; M-1 to L-123; M-1 to E-122; M-1 to H-121; M-1 to E-120; M-1 to W-119; M-1 to H-118; M-1 to L-117; M-1 to A-116; M-1 to P-115; M-1 to F-114; M-1 to Q-113; M-1 to N-112; M-1 to K-111; M-1 to F-110; M-1 to H-109; M-1 to Q-108; M-1 to T-107; M-1 to P-106; M-1 to T-105; M-1 to Q-104; M-1 to R-103; M-1 to V-102; M-1 to V-101; M-1 to T-100; M-1 to L-99; M-1 to H-98; M-1 to A-97; M-1 to R-96; M-1 to P-95; M-1 to K-94; M-1 to D-93; M-1 to G-92; M-1 to D-91; M-1 to A-90; M-1 to R-89; M-1 to L-88; M-1 to P-87; M-1 to A-86; M-1 to Y-85; M-1 to V-84; M-1 to Q-83; M-1 to Q-82; M-1 to H-81; M-1 to S-80; M-1 to P-79; M-1 to A-78; M-1 to F-77; M-1 to E-76; M-1 to Q-75; M-1 to G-74; M-1 to K-73; M-1 to L-72; M-1 to A-71; M-1 to Q-70; M-1 to F-69; M-1 to Q-68; M-1 to V-67; M-1, to C-66; M-1 to A-65; M-1 to E-64; M-1 to G-63; M-1 to Q-62; M-1 to A-61; M-1 to R-60; M-1 to L-59; M-1 to Q-58; M-1 to S-57; M-1 to V-56; M-1 to L-55; M-1 to L-54; M-1 to Y-53; M-1 to T-52; M-1 to T-51; M-1 to L-50; M-1 to G-49; M-1 to A-48; M-1 to L-47; M-1 to F-46; M-1 to P-45; M-1 to L-44; M-1 to L-43; M-1 to V-42; M-1 to L-41; M-1 to C-40; M-1 to C-39; M-1 to T-38; M-1 to L-37; M-1 to A-36; M-1 to W-35; M-1 to R-34; M-1 to A-33; M-1 to S-32; M-1 to S-31; M-1 to S-30; M-1 to R-29; M-1 to A-28; M-1 to K-27; M-1 to P-26; M-1 to R-25; M-1 to C-24; M-1 to S-23; M-1 to G-22; M-1 to H-21; M-1 to E-20; M-1 to P-19; M-1 to L-18; M-1 to M-17; M-1 to E-16; M-1 to V-15; M-1 to S-14; M-1 to A-13; M-1 to T-12; M-1 to E-11; M-1 to G-10; M-1 to F-9; M-1 to S-8; M-1 to L-7; and M-1 to G-6 of the sequence of the VEGI-beta sequence shown in SEQ ID NO:27. Polynucleotides encoding these polypeptides also are provided.

The invention also provides polypeptides having one or more amino acids deleted from both the amino and the carboxyl termini of a VEGI-beta polypeptide, which may be described generally as having residues n⁴-m⁴ of SEQ ID NO:27, where n⁴ and m⁴ are integers as described above. Polynucleotides encoding these polypeptides are also encompassed by the invention.

Further embodiments of the invention are directed to polypeptide fragments comprising, or alternatively, consisting of, amino acids described by the general formula m^{x} to n^{x}, where m and n correspond to any one of the amino acid residues specified above for these symbols, respectively, and x represents any integer. Polynucleotides encoding these polypeptides are also encompassed by the invention.

Specific embodiments of the invention are directed to nucleotide sequences encoding a polypeptide consisting of a portion of the complete VEGI amino acid sequence encoded by the cDNA clone contained in ATCC Deposit No. 75927, where this portion excludes from 1 to about 62 amino acids from the amino terminus of the complete amino acid sequence encoded by the cDNA clone contained in ATCC Deposit No. 75927, or about 1 amino acid from the carboxy terminus, or any combination of the above amino terminal and carboxy terminal deletions, of the complete amino acid sequence encoded by the cDNA clone contained in ATCC Deposit No. 75927. Polynucleotides encoding all of the above deletion mutant polypeptide forms also are provided.

In another embodiment, the invention is directed to a nucleotide sequence encoding a polypeptide consisting of a portion of the complete VEGI-beta amino acid sequence encoded by the cDNA clone contained in ATCC Deposit No. 203055, where this portion excludes from 1 to about 134 amino acids from the amino terminus of the complete amino acid sequence encoded by the cDNA clone contained in ATCC Deposit No. 203055, or excludes a number of amino acids from the amino terminus of the complete amino acid sequence encoded by the cDNA clone contained in ATCC Deposit No. 203055 (where the number is selected from any integer from 1 to 134), or about 1 amino acid from the carboxy terminus, or any combination of the above amino terminal and carboxy terminal deletions, of the complete amino acid sequence encoded by the cDNA clone contained in ATCC Deposit No. 203055. Polynucleotides encoding all of the above polypeptides are also encompassed by the invention.

The invention further provides an isolated VEGI polypeptide comprising an amino acid sequence selected from the group consisting of: (a) the amino acid sequence of the full-length VEGI polypeptide having the complete amino acid sequence shown in SEQ ID NO:9 (i.e., positions -27 to 147 of SEQ ID NO:9); (b) the amino acid sequence of the full-length VEGI polypeptide having the complete amino acid sequence shown in SEQ ID NO:9 excepting the N-terminal methionine (i.e., positions -26 to 147 of SEQ ID NO:9); (c) the amino acid sequence of the predicted mature VEGI polypeptide having the amino acid sequence at positions 1-147 in SEQ ID NO:9 (d) the complete amino acid sequence encoded by the cDNA clone HUVEO91 contained in the ATCC Deposit No. 75927; (e) the complete amino acid sequence excepting the N-terminal methionine encoded by the cDNA clone contained in the ATCC Deposit No. 75927; and (f) the complete amino acid sequence of the predicted mature VEGI polypeptide encoded by the cDNA clone HUVEO91 contained in the ATCC Deposit No. 75927. The polypeptides of the present invention also include polypeptides having an amino acid sequence at least 70% identical, at least 80% identical, more preferably at least 90% identical, and still more preferably 95%, 96%, 97%, 98% or 99% identical to those described in (a), (b), (c), (d), (e) or (f), above, or fragments thereof, as described herein.

The invention further provides an isolated VEGI polypeptide comprising an amino acid sequence selected from the group consisting of: (a) the amino acid sequence of the full-length VEGI-beta polypeptide having the complete amino acid sequence shown in SEQ ID NO:27 (i.e., positions 1 to 251 of SEQ ID NO:27); (b) the amino acid sequence of the full-length VEGI-beta polypeptide having the complete amino acid sequence shown in SEQ ID NO:27 excepting the N-terminal methionine (i.e., positions 2 to 251 of SEQ ID NO:27); (c) the amino acid sequence of the predicted mature VEGI-beta polypeptide having the amino acid sequence at positions 62-251 in SEQ ID NO:27; (d) the complete amino acid sequence encoded by the cDNA clone HEMCZ56 contained in the ATCC Deposit No. 203055; (e) the complete amino acid sequence excepting the N-terminal methionine encoded by the cDNA clone HEMCZ56 contained in the ATCC Deposit No. 203055; and (f) the complete amino acid sequence of the predicted mature VEGI polypeptide encoded by the cDNA clone HEMCZ56 contained in the ATCC Deposit No. 203055. The polypeptides of the present invention also include polypeptides having an amino acid sequence at least 70% identical, at least 80% identical, more preferably at least 90% identical, and still more preferably 95%, 96%, 97%, 98% or 99% identical to those described in (a), (b), (c), (d), (e) or (f), above, or fragments thereof, as described herein. In specific embodiments, these polypeptides are at least 10 amino acids, at least 15 amino acids, at leat 20 amino acids, at least 25 amino acids, at least 30 amino acids and more preferably at least 50 amino acids.

By a polypeptide having an amino acid sequence at least, for example, 95% "identical" to a reference amino acid sequence of a VEGI polypeptide is intended that the amino acid sequence of the polypeptide is identical to the reference sequence except that the polypeptide sequence may include up to five amino acid alterations per each 100 amino acids of the reference amino acid of the VEGI polypeptide. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequence shown in Figures 5A, 5B, and 5C (SEQ ID NO:9), the amino acid sequence encoded by deposited cDNA clone HUVEO91, or fragments thereof, can be determined conventionally using known computer programs such the Bestfit program (Wisconsin Sequence Analysis Package, Version 8 for Unix, Genetics Computer Group, University Research Park, 575 Science Drive, Madison, WI 53711). When using Bestfit or any other sequence alignment program to determine whether a particular sequence is, for instance, 95% identical to a reference sequence according to the present invention, the parameters are set, of course, such that the percentage of identity is calculated over the full length of the reference amino acid sequence and that gaps in homology of up to 5% of the total number of amino acid residues in the reference sequence are allowed.

In a specific embodiment, the identity between a reference (query) sequence (a sequence of the present invention) and a subject sequence, also referred to as a global sequence alignment, is determined using the FASTDB computer program based on the algorithm of Brutlag et al. (Comp. App. Biosci. 6:237-245 (1990)). Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter. According to this embodiment, if the subject sequence is shorter than the query sequence due to N- or C-terminal deletions, not because of internal deletions, a manual correction is made to the results to take into consideration the fact that the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. A determination of whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of this embodiment. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence. For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for. No other manual corrections are made for the purposes of this embodiment.

The polypeptides of the present invention include the polypeptide of SEQ ID NO:9 (in particular the mature polypeptide) as well as polypeptides which have at least 70% similarity (preferably at least 70% identity) to the polypeptide of SEQ ID NO:9 and more preferably at least 90% similarity (more preferably at least 90% identity) to the polypeptide of SEQ ID NO:9 and still more preferably at least 95% similarity (still more preferably at least 95% identity) to the polypeptide of SEQ ID NO:9 and also include portions of such polypeptides with such portion of the polypeptide generally containing at least 30 amino acids and more preferably at least 50 amino acids.

The polypeptides of the present invention also include the polypeptide of SEQ ID NO:27 (in particular the extracellular domain of the polypeptide) as well as polypeptides which have at least 70% similarity (preferably at least 70% identity) to the polypeptide of SEQ ID NO:27 and more preferably at least 90% similarity (more preferably at least 90% identity) to the polypeptide of SEQ ID NO:27 and still more preferably at least 95% similarity (still more preferably at least 95% identity) to the polypeptide of SEQ ID NO:27 and also include portions of such polypeptides with such portion of the polypeptide generally containing at least 30 amino acids and more preferably at least 50 amino acids.

Further polypeptides of the present invention include polypeptides have at least 70% similarity, at least 90% similarity, more preferably at least 95% similarity, and still more preferably at least 96%, 97%, 98% or 99% similarity to those polypeptides described herein. The polypeptides of the invention also comprise those which are at least 70% identical, at least 80% identical, more preferably at least 90% or 95% identical, still more preferably at least 96%, 97%, 98% or 99% identical to the polypeptides disclosed herein. In specific embodiments, such polypeptides comprise at least 30 amino acids and more preferably at least 50 amino acids.

As known in the art "similarity" between two polypeptides is determined by comparing the amino acid sequence and its conserved amino acid substitutes of one polypeptide to the sequence of a second polypeptide. By "% similarity" for two polypeptides is intended a similarity score produced by comparing the amino acid sequences of the two polypeptides using the Bestfit program (Wisconsin Sequence Analysis Package, Version 8 for Unix, Genetics Computer Group, University Research Park, 575 Science Drive, Madison, WI 53711) and the default settings for determining similarity. Bestfit uses the local homology algorithm of Smith and Waterman (Advances in Applied Mathematics 2:482-489, 1981) to find the best segment of similarity between two sequences.

As used herein, a polypeptide or amino acid sequence "derived from" the amino acid sequence in SEQ ID NO:2, SEQ ID NO:9, and SEQ ID NO:27. refers to a polypeptide having an amino acid sequence identical to that of a polypeptide encoded in the sequence, or a portion thereof wherein the portion consists of at least 2-5 amino acids, and more preferably at least 8-10 amino acids, and even more preferably at least 11-15 amino acids, or which is immunologically identifiable with a polypeptide encoded in the sequence.

The invention also encompasses fusion proteins in which the full length VEGI polypeptide or fragment, variant, derivative, or analog thereof is fused to an unrelated protein. These fusion proteins can be routinely designed on the basis of the VEGI nucleotide and polypeptide sequences disclosed herein. For example, as one of skill in the art will appreciate, VEGI and/or VEGI-beta polypeptides and fragments (including epitope-bearing fragments) thereof described herein can be combined with parts of the constant domain of immunoglobulins (IgG), resulting in chimeric (fusion) polypeptides. These fusion proteins facilitate purification and show an increased half-life *in vivo.* This has been shown, e.g., for chimeric proteins consisting of the first two domains of the human CD4-polypeptide and various domains of the constant regions of the heavy or light chains of mammalian immunoglobulins (EP A 394,827; Traunecker, et aL, Nature 331:84-86 (1988)). Fusion proteins that have a disulfide-linked dimeric structure due to the IgG part can also be more efficient in binding and neutralizing other molecules than the monomeric VEGI protein or protein fragment alone (Fountoulakis, et al., J. Biochem. 270:3958-3964 (1995)). As an example, one such VEGI-Fc fusion has been produced herein as described above. Additional examples of VEGI fusion proteins that are encompassed by the invention include, but are not limited to, fusion of the VEGI polypeptide sequence to any amino acid sequence that allows the fusion protein to be displayed on the cell surface; or fusions to an enzyme, fluorescent protein, or luminescent protein which provides a marker function.

### Functional Activities

The functional activity of VEGI polypeptides, and fragments, variants derivatives, and analogs thereof, can be assayed by various methods.

For example, in one embodiment where one is assaying for the ability to bind or compete with full-length VEGI polypeptide for binding to anti-VEGI antibody, various immunoassays known in the art can be used, including but not limited to, competitive and non-competitive assay systems using techniques such as radioimmunoassays, ELISA (enzyme linked immunosorbent assay), "sandwich" immunoassays, immunoradiometric assays, gel diffusion precipitation reactions, immunodiffusion *assays, in situ* immunoassays (using colloidal gold, enzyme or radioisotope labels, for example), western blots, precipitation reactions, agglutination assays (e.g., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labelled. Many means are known in the art for detecting binding in an immunoassay and are within the scope of the present invention.

In another embodiment, where a TNF-ligand is identified, binding can be assayed, e.g., by means well-known in the art. In another embodiment, physiological correlates of VEGI binding to its substrates (signal transduction) can be assayed.

In addition, assays described herein (see Examples 5-8 and 10, and otherwise known in the art may routinely be applied to measure the ability of VEGI polypeptides and fragments, variants derivatives and analogs thereof to elicit VEGI related biological activity (e.g., to inhibit, or alternatively promote, cell proliferation, angiogenesis, and cell *adhesion in vitro or in vivo).*

Other methods will be known to the skilled artisan and are within the scope of the invention.

### Antibodies

Antibodies that specifically recognize one or more epitopes of VEGI or epitopes of conserved variants of VEGI, or polypeptide fragments of VEGI, are also encompassed by the invention. Thus, the polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, F(ab') fragments, the products of an Fab expression library, anti-idiotypic antibodies, and epitope binding fragments of any of the above. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies have uses which include, but are not limited to, isolation of the polypeptide from tissue expressing that polypeptide and for detection of VEGI in a biological sample, and may be used as part of a diagnostic or prognostic technique whereby patients may be tested for abnormal amounts of VEGI. Such antibodies may also be used in a method of inhibition of VEGI biological activity.

For the production of antibodies, various host animals may be immunized by injection with VEGI polypeptide (e.g., one corresponding to a functional domain of the polypeptide, such as, the extracellular domain). Such host animals may include but are not limited to rabbits, mice, and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.* Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein (Nature 256:495-497 (1975)); and U.S. Patent No. 4,376,110), the human B-cell hybridoma technique (Kosbor, et al., Immunology Today 4:72 (1983); Cole, et al., Proc. Natl. Acad. Sci. USA 80:2026-2030 (1983), and the EBV-hybridoma technique *(*Cole, et al., Monoclonal Antibodies And Cancer Therapy, Alan R. Liss, Inc., pp. 77-96 (1985)). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison, et al., Proc. Natl. Acad. Sci., 81:6851-6855 (1984); Neuberger, et al., Nature 312:604-608 (1984); Takeda, et al., Nature 314:452-454 (1985)) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

For *in vivo* use of anti-VEGI in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art (Morrison, Science 229:1202 (1985); Oi, et al, BioTechniques 4:214 (1986); Cabilly, *et aL,* U.S. Patent No. 4,816,567; Taniguchi, *et al,* EP 171496; Morrison, *et al.,* EP 173494; Neuberger, *et al.,* WO 8601533; Robinson, *et al.,* WO 8702671; Boulianne, et al, Nature 312:643 (1984); Neuberger, et al, Nature 314:268 (1985).

Alternatively, techniques described for the production of single chain antibodies (U.S. Patent 4,946,778; Bird, Science 242:423-426 (1988); Huston, et al., Proc. Natl. Acad. Sci. USA 85:5879-5883 (1988); and Ward, et al., Nature 334:544546 (1989)) can be adapted to produce single chain antibodies against VEGI polypeptides. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include but are not limited to: the F(ab')2 fragments which can be produced by pepsin digestion of the antibody molecule and the Fab fragments which can be generated by reducing the disulfide bridges of the F(ab')2 fragments. Alternatively, Fab expression libraries may be constructed *(*Huse, et al., Science 246:1275-1281 (1989)) to allow rapid and easy identification of monoclonal Fab fragments with the desired specificity.

Antibodies to the VEGI polypeptide can, in turn, be utilized to generate anti-idiotype antibodies that "mimic" the ObR, using techniques well known to those. skilled in the art. (See, e.g., Greenspan & Bona, FASEB J. 7(5):437-444; (1989) and Nissinoff, J. Immunol. 147(8):2429-2438 (1991)). For example, antibodies which bind to the VEGI extracellular domain and competitively inhibit the binding of ligand to the VEGI can be used to generate anti-idiotypes that "mimic" the VEGI extracellular domain and, therefore, bind and neutralize VEGI ligand. Such neutralizing anti-idiotypes or Fab fragments of such anti-idiotypes can be used in therapeutic regimens to neutralize VEGI ligand.

Thus, as disclosed above, persons with ordinary skill in the art using standard methodology can raise monoclonal and polyclonal antibodies to VEGI protein (or polypeptide) of the present invention. Material and methods for producing antibodies are well known in the art (see for example Goding, in, Monoclonal Antibodies: Principles and Practice, Chapter 4, 1986). In addition, the polypeptide can be fused to other proteins or polypeptides which increase its antigenicity, thereby producing higher titers of antibodies. Examples of such proteins or polypeptides include any adjuvants or carriers, such as aluminum hydroxide. These antibodies can be used in a passive antibody therapy wherein antibodies that specifically bind VEGI can be employed to modulate angiogenic-dependent processes such as reproduction, development, wound healing, and tissue repair, to name a few.

### Immune and Circulatory Systems-Related and Other Medical Disorders

In another embodiment. the present invention relates to a method of reducing symptoms of an angiogenic-associated disease in an animal or human patient by administering to said patient an effective amount of VEGI. Since angiogenesis is required for the survival of a tumour, inhibiting or reversing angiogenesis may reduce or eliminate the tumour. When providing a patient with VEGI, the dosage of administered agent will vary depending upon such factors as the patient's age, weight, height. sex, general medical condition, previous medical history, etc. In general, it is desirable to provide the recipient with a dosage of the above compounds which is in the range of from about lpg/kg to 10 mg/kg (body weight of patient), although a lower or higher dosage may be administered. The methods of the present invention contemplate single as well as multiple administrations, given either simultaneously or over an extended period of time.

The VEGI polynucleotides can be provided by using plasmids or viral vectors such as adenovirus or retrovirus which contain VEGI polynucleotides of the present invention described in SEQ ID NO:1 or a portion thereof, or an allelic form thereof. The vectors used are preferably capable of providing VEGI polynucleotides to a cell such that the polynucleotides are transcribed and translated, and VEGI polypeptide is produced. The polynucleotides of the invention can also be administered as DNA, as DNA encapsulated in liposomes, or as DNA entrapped in proteoliposomes containing viral envelope receptor proteins (Kanoda, Y. et al. (1989) Science 243:375*).* Alternatively, the polynucleotides of the invention can be injected along with a carrier. A carrier can be a protein such as a cytokine, for example interleukin 2, or polylysine glycoprotein carrier. Such carrier proteins and vectors and methods of using same are known in the art (See for example Ascadi G. et al. Nature 1991. 352:815). In addition, the polynucleotides of the invention may be coated onto tiny gold beads and said beads introduced into the skin with, for example, a gene gun (Ulmer, J.B. et al. Science 1993, 259: 1745*)*

Alternatively VEGI polypeptides of the invention can be provided in pharmaceutically acceptable formulations using formulation methods known to those of ordinary skill in the art. These formulations can be administered by standard routes. In general, the combinations may be administered by the topical. transdermal, introperitoneal, oral, rectal, or parenteral (e.g. intravenous, subcutaneous, or intramuscular) route. In addition, the VEGI polypeptides of the invention may be incorporated into biodegradable polymers being implanted in the vicinity of where drug delivery is desired or implanted so that the VEGI polypeptide is slowly released systemically. The biodegradable polymers and their use are described. for example, in detail in Brem et al., J. Neurosurg. 74:441-446 (1991)*.*

The VEGI polypeptide formulations include those suitable for oral, rectal, ophthalmic (including intravitreal or intracameral), nasal, topical (including buccal and sublingual), vaginal or parenteral (including subcutaneous, intraperitoneal, and epidural) administration. The VEGI polypeptide formulations may conveniently be presented in unit dosage form and may be prepared by conventional pharmaceutical techniques. Such techniques include the step of bringing into associated the active ingredient and the pharmaceutical carrier(s) or excipient(s). In general, the formulations are prepared by uniformly and intimately bringing into association the active ingredient with liquid carriers or finely divided solid carriers or both, and then, if necessary, shaping the product.

Formulations suitable for parenteral administration include aqueous and non-aqueous sterile injection solutions which may contain anti-oxidants, buffers, and solutes which render the formulation isotonic with regard to the blood of the intended recipient; and aqueous and non-aqueous sterile suspensions which may include suspending agents and thickening agents. The formulations may be presented in unit-dose or multidose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of the administered ingredient. It should be understood that in addition to the ingredients, particularly mentioned above, the formulations of the present invention may include other agents conventional in the art having regard to the type of formulation in question.

In another embodiment, the present invention relates to a method of reducing symptoms of uncontrolled pathological angiogenesis in a patient by administering to said patient an effective amount of VEGI polynucleotides, VEGI polypeptides or analogs thereof, or agents capable of inducing expression of endogenous or exogenous VEGI polynucleotides or of endogenous or exogenous VEGI polypeptides such that symptoms of pathological angiogenesis are reduced. The diseases contemplated include, but are not limited to, hemangioma, telangiectasia psoriasis scleroderma, pyogenic granuloma, myocardial angiogenesis, plague neovascularization, coronary collaterals, ischemic limb angiogenesis, corneal diseases, rubeosis, neovascular glaucoma, diabetic retinopathy, retrolental fibroplasia, Arthritis, diabetic neovascularization. The diseases and disease states described herein do not include cancer or tumors of any kind.

In a specific embodiment, a VEGI-alpha and/or VEGI-beta polypeptide of the present invention is combined with a therapeutically effective amount of another molecule which negatively regulates angiogenesis which may be, but is not limited to, platelet factor-4, thrombospondin-1, tissue inhibitors of metallopreteases (TIMP and TIMP2), prolactin (16 kDa fragment), angiostatin (38 kDa fragment of plasminogen), bFGF soluble receptor, transforming growth factor β, interferon alpha, and placental proliferin-related protein.

Ocular disorders associated with neovascularization which can be treated with VEGI-alpha and/or VEGI-beta polypeptides of the invention include, but are not limited to, neovascular glaucoma, diabetic retinopathy, retinoblastoma, retrolental fibroplasia, uveitis, retinopathy of prematurity, macular degeneration, corneal graft neovascularization, as well as other eye inflammatory diseases and diseases associated with choroidal or iris neovascularization. (See, e.g., reviews by Waltman, et al., 1978 Am. J. Ophthal. 85:704-710 and Gartner, et al., 1978, Surv. Ophthal. 22:291-312.)

In another embodiment, the present invention relates to a method of promoting angiogenesis in a patient by administering to said patient an effective amount of an agent capable of reducing VEGI polypeptide function by decreasing transcription of VEGI polynucleotides, decreasing the amount of RNA encoding a VEGI polypeptide, for example by using a VEGI-specific ribozyme or an antisense oligonucleotide specific for VEGI RNA, or by inhibiting the production of VEGI polypeptides by inhibition of proteases which may process and/or activate VEGI polypeptides as discussed above (Kayagaki, et al. (1995) J. Exp. Med. 182: 1777-1783*:* Black. et al. (1997) Nature 385:729-733), or by blocking or removing agents and/or metal ions which permit, or facilitate the activation of VEGI polypeptides, or by directly inhibiting VEGI polypeptide function by blocking a VEGI receptor, or by using an antibody which recognizes and binds to a VEGI polypeptide, or by using an antagonist to inhibit the function of a VEGI polypeptide. The engineering and delivery of ribozymes and antisense oligonucleotides are known in the art *(See,* Usman N., et al. (1996) Curr. Opin. Struct. Biol. 6:527-533 and Ho and Parkinson, (1997) Semin. Oncol. 24:187-202*),* and can be delivered into mammalian cells for example by mixing with lipids or by using a viral or plasmid vector. Diseases and conditions contemplated include, but are not limited to macular degeneration, wound healing, peptic ulcer, fractures, keloids, vasculogenesis, hematopoiesis, ovulation, menstruation, and placentation.

### Additional Embodiments

In a further embodiment, the present invention relates to a method of detecting the presence of VEGI polypeptides in a sample for the purpose of diagnosis or prognosis of an angiogenic-associated disease. Using standard methodology well known in the art, a diagnostic assay can be constructed by coating on a surface (i.e. a solid support). for example. a microtitration plate or a membrane (e.g. nitrocellulose membrane), antibodies specific for VEGI polypeptides, and contacting the coated surface with serum, tissue or other biological or chemical sample obtained from a person suspected of having an angiogenic-associated disease. The presence of a resulting complex formed between VEGI polypeptide in the sample and antibodies specific therefor can be detected by any of the known methods common in the art, such as fluorescent antibody spectroscopy or colorimetry. This method of detection can be used, for example, for the diagnosis or prognosis of cancer.

In another embodiment, the present invention relates to a diagnostic kit which contains antibodies specific for VEGI polypeptides and ancillary reagents that are well known in the art and that are suitable for use in detecting the presence of VEGI polypeptides in a serum, tissue or other sample. Tissue samples contemplated can be obtained from monkey or human. or other mammals.

In another embodiment, the present invention relates to RNA, DNA or other nucleotide sequences for use in detecting the presence or absence of VEGI polynucleotides using the polymerase chain reaction (PCR) or reverse transcription PCR (RT-PCR). The DNA sequence of the present invention shown in SEQ ID NO:1, SEQ ID NO:8, or in SEQ ID NO:26 can be used to design primers which specifically bind to the VEGI polynucleotide sequence in the case of PCR, or to a VEGI cDNA produced from reverse transcription of an RNA encoding a VEGI polypeptide, for the purpose of detecting the presence, absence, or quantitating the amount of VEGI polynucleotide by comparison to a standard. The primers can be any length ranging from 7-40 nucleotides, preferably 10-15 nucleotides, most preferably 18-25 nucleotides. Reagents and controls necessary for PCR or RT-PCR reactions are well known in the art. The amplified products can then be analyzed for the presence or absence of VEGI polynucleotide sequences, for example by gel fractionation, with or without hybridization, by radiochemistry, and immunochemical techniques. This method is advantageous since only a small sample size is required to generate a sufficient amount of template DNA with which to perform PCR or RT-PCR.

In another embodiment, the present invention relates to a diagnostic kit which contains PCR or RT-PCR primers specific for VEGI polynucleotides, and ancillary reagents that are well known in the art and that are suitable for use in detecting the presence or absence of VEGI polynucleotides, or quantitating the amount of an RNA which encodes a VEGI polypeptide in a sample using PCR or RT-PCR. Samples contemplated can be obtained from humans or other mammals.

VEGI is expressed in human umbilical vein endothelial cells, induced endothelial cells, macrophages, and substantia nigra tissue. For a number of immune and circulatory systems-related disorders, substantially altered (increased or decreased) levels of VEGI-alpha and/or VEGI-beta gene expression can be detected in immune and circulatory systems tissue or other cells or bodily fluids (e.g., sera, plasma, urine, synovial fluid or spinal fluid) taken from an individual having such a disorder, relative to a "standard" VEGI-alpha and/or VEGI-beta gene expression level, that is, the VEGI-alpha and/or VEGI-beta expression level in immune and circulatory systems tissues or bodily fluids from an individual not having the immune and circulatory systems disorder. Thus, the invention provides a diagnostic method useful during diagnosis of a immune and circulatory systems disorder, which involves measuring the expression level of the gene encoding the VEGI-alpha and/or VEGI-beta protein in immune and circulatory systems tissue or other cells or body fluid from an individual and comparing the measured gene expression level with a standard VEGI-alpha and/or VEGI-beta gene expression level, whereby an increase or decrease in the gene expression level compared to the standard is indicative of an immune and circulatory systems disorder.

Thus, the invention provides a diagnostic method useful during diagnosis of a immune and circulatory systems disorder which involves measuring the expression level of the gene encoding the VEGI-alpha and/or VEGI-beta protein in immune and circulatory systems tissue or other cells or body fluid from an individual and comparing the measured gene expression level with a standard VEGI-alpha and/or VEGI-beta gene expression level, whereby an increase or decrease in the gene expression level compared to the standard is indicative of an immune and circulatory systems disorder.

Where a diagnosis of a disorder in the immune and circulatory systems has already been made according to conventional methods, the present invention is useful as a prognostic indicator, whereby patients exhibiting depressed VEGI-alpha and/or VEGI-beta gene expression will experience a worse clinical outcome relative to patients expressing the gene at a level nearer the standard level.

By "assaying the expression level of the gene encoding the VEGI-alpha and/or VEGI-beta protein" is intended qualitatively or quantitatively measuring or estimating the level of the VEGI-alpha and/or VEGI-beta polypeptide or the level of the mRNA encoding the VEGI-alpha and/or VEGI-beta polypeptide in a first biological sample either directly (e.g., by determining or estimating absolute polypeptide level or mRNA level) or relatively (e.g., by comparing to the VEGI-alpha and/or VEGI-beta polypeptide level or mRNA level in a second biological sample). Preferably, the VEGI-alpha and/or VEGI-beta polypeptide level or mRNA level in the first biological sample is measured or estimated and compared to a standard VEGI-alpha and/or VEGI-beta polypeptide level or mRNA level, the standard being taken from a second biological sample obtained from an individual not having the disorder or being determined by averaging levels from a population of individuals not having a disorder of the immune and circulatory systems. As will be appreciated in the art, once a standard VEGI-alpha and/or VEGI-beta polypeptide level or mRNA level is known, it can be used repeatedly as a standard for comparison.

By "biological sample" is intended any biological sample obtained from an individual, body fluid, cell line, tissue culture, or other source which contains VEGI-alpha and/or VEGI-beta polypeptide or mRNA. As indicated, biological samples include body fluids (such as sera, plasma, urine, synovial fluid and spinal fluid) which contain free VEGI-alpha and/or VEGI-beta polypeptide, immune and circulatory systems tissue, and other tissue sources found to express complete or mature VEGI-alpha and/or VEGI-beta polypeptides or a VEGI-alpha and/or VEGI-beta receptor. Methods for obtaining tissue biopsies and body fluids from mammals are well known in the art. Where the biological sample is to include mRNA, a tissue biopsy is the preferred source.

Total cellular RNA can be isolated from a biological sample using any suitable technique such as the single-step guanidinium-thiocyanate-phenolchloroform method described by Chomczynski and Sacchi (Anal. Biochem. 162:156-159 (1987)). Levels of mRNA encoding the VEGI-alpha and/or VEGI-beta polypeptide are then assayed using any appropriate method. These include Northern blot analysis; S 1 nuclease mapping, the polymerase chain reaction (PCR), reverse transcription in combination with the polymerase chain reaction (RT-PCR), and reverse transcription in combination with the ligase chain reaction (RT-LCR).

Assaying VEGI-alpha and/or VEGI-beta polypeptide levels in a biological sample can occur using antibody-based techniques. For example, VEGI-alpha and/or VEGI-beta polypeptide expression in tissues can be studied with classical immunohistological methods (Jalkanen, M., et al., J. Cell. Biol. 101:976-985 (1985); Jalkanen, M., et al., J. Cell. Biol. 105:3087-3096 (1987)). Other antibody-based methods useful for detecting VEGI-alpha and/or VEGI-beta polypeptide gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), and fluorescent labels, such as fluorescein and rhodamine, and biotin.

In addition to assaying VEGI-alpha and/or VEGI-beta polypeptide levels in a biological sample obtained from an individual, VEGI-alpha and/or VEGI-beta polypeptide can also be *detected in vivo* by imaging. Antibody labels or markers for *in vivo* imaging of VEGI-alpha and/or VEGI-beta polypeptide include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A VEGI-alpha and/or VEGI-beta polypeptide-specific antibody or antibody fragment which has been labeled with an appropriate detectable imaging moiety, such as a radioisotope (for example, ¹³¹I, ¹¹²In, ^{99m}Tc), a radio-opaque substance, or a material detectable by nuclear magnetic resonance, is introduced (for example, parenterally, subcutaneously or intraperitoneally) into the mammal to be examined for immune system disorder. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of^{99m}Tc. The labeled antibody or antibody fragment will then preferentially accumulate at the location of cells which contain VEGI-alpha and/or VEGI-beta polypeptide. *In vivo* tumor imaging is described by Burchiel and coworkers (Chapter 13 in Tumor Imaging: The Radiochemical Detection of Cancer, Burchiel, S. W. and Rhodes, B. A., eds., Masson Publishing Inc. (1982)).

As noted above, VEGI-alpha and/or VEGI-beta polynucleotides and polypeptides are useful for diagnosis of conditions involving abnormally high or low expression of VEGI-alpha and/or VEGI-beta activities. Given the cells and tissues where VEGI-alpha and/or VEGI-beta is expressed as well as the activities modulated by VEGI-alpha and/or VEGI-beta, it is readily apparent that a substantially altered (increased or decreased) level of expression of VEGI-alpha and/or VEGI-beta in an individual compared to the standard or "normal" level produces pathological conditions related to the bodily system(s) in which VEGI-alpha and/or VEGI-beta is expressed and/or is active.

The present invention is also useful for diagnosis or treatment of various immune and circulatory system-related disorders in mammals, preferably humans. Such disorders include infections by bacteria, viruses, and other parasites, immunodeficiencies, inflammatory diseases, lymphadenopathy, autoimmune diseases, graft versus host disease, and any disregulation of immune and circulatory systems cell function including, but not limited to, autoimmunity, leukemias, lymphomas, immunosuppression, immunity, humoral immunity, inflammatory bowel disease, myelo suppression, and the like.

Since VEGI polypeptides may induce activation of cellular NF-κB and c-jun N-terminal kinase (JNK), it is also useful in therapeutically regulating such cellular and immune systemic disorders as infections by bacteria, viruses, and other parasites, immunodeficiencies, inflammatory diseases, lymphadenopathy, autoimmune diseases, graft versus host disease, autoimmunity, immunosuppression, inflammatory bowel disease, myelosuppression, and related sequelae.

Since VEGI-alpha and VEGI-beta belong to the TNF superfamily, they also also modulate angiogenesis. In addition, since VEGI-alpha and/or VEGI-beta polypeptides inhibit immune cell functions, it will have a wide range of anti-inflammatory activities. VEGI-alpha and/or VEGI-beta polypeptides may be employed as an anti-neovascularizing agent to stimulate the invasion and activation of host defense cells, e.g., cytotoxic T-cells and macrophages. Those of skill in the art will recognize many non-cancer indications where blood vessel proliferation is not desired. VEGI-alpha and VEGI-beta polypeptides may also be employed to enhance host defenses against resistant chronic and acute infections, for example, myobacterial infections via the attraction and activation of microbicidal leukocytes. VEGI-alpha and/or VEGI-beta polypeptides may also be employed to inhibit T-cell proliferation by the inhibition of IL-2 biosynthesis for the treatment of T-cell mediated auto-immune diseases. VEGI-alpha and/or VEGI-beta polypeptides may also be employed to stimulate wound healing, both via the recruitment of debris clearing and connective tissue promoting inflammatory cells. In this same manner, VEGI-alpha and/or VEGI-beta polypeptides may also be employed to treat other fibrotic disorders, including liver cirrhosis, osteoarthritis and pulmonary fibrosis. VEGI-alpha and/or VEGI-beta polypeptides also increases the presence of eosinophils which have the distinctive function of killing the larvae of parasites that invade tissues, as in schistosomiasis, trichinosis and ascariasis. VEGI-alpha and/or VEGI-beta polypeptides may also be employed to regulate hematopoiesis, by regulating the activation and differentiation of various hematopoietic progenitor cells, for example, to release mature leukocytes from the bone marrow following chemotherapy, i.e., in stem cell mobilization. VEGI-alpha and/or VEGI-beta polypeptides may also be employed to treat sepsis.

It will also be appreciated by one of ordinary skill that, since the VEGI-alpha and/or VEGI-beta polypeptides of the invention are members of the TNF family the mature secreted form of the protein may be released in soluble form from the cells which express VEGI by proteolytic cleavage. Therefore, when the mature form or soluble extracellular domain of VEGI-alpha and/or VEGI-beta is added from an exogenous source to cells, tissues or the body of an individual, the polypeptide will exert its physiological activities on its target cells of that individual. Also, cells expressing this type II transmembrane polypeptide may be added to cells, tissues or the body of an individual and these added cells will bind to cells expressing receptor for VEGI-alpha and/or VEGI-beta, whereby the cells expressing VEGI-alpha and/or VEGI-beta polypeptides can cause actions (e.g. regulation of endothelial cell growth and regulation) on the receptor-bearing target cells.

Therefore, it will be appreciated that conditions caused by a decrease in the standard or normal level of VEGI-alpha and/or VEGI-beta activities in an individual, particularly disorders of the immune and circulatory systems, can be treated by administration of VEGI-alpha and/or VEGI-beta polypeptide (in the form of the mature or extracellular domain polypeptide). Thus, the invention also provides a method of treatment of an individual in need of an increased level of VEGI-alpha and/or VEGI-beta activity comprising administering to such an individual a pharmaceutical composition comprising an amount of an isolated VEGI-alpha and/or VEGI-beta polypeptide of the invention, particularly a mature form of the VEGI-alpha and/or VEGI-beta polypeptide of the invention, effective to increase the VEGI-alpha and/or VEGI-beta activity level in such an individual.

The cell adhesion activity of VEGI may be employed for wound healing. VEGI has a strong endothelial cell proliferation effect which is an indication that VEGI plays a role in wound healing. The cell adhesive effects of VEGI may also play a role in wound healing.

VEGI polypeptides may also be employed to treat diseases which require growth promotion activity, for example, restenosis. As stated above, VEGI is shown to have strong proliferation effects on endothelial cell growth. Accordingly, VEGI may also be employed to regulate hematopoiesis and endothelial cell development.

The VEGI polypeptide, through its ability to stimulate the activation of T-cells, is an important mediator of the immune response. Accordingly, this polypeptide may be used to stimulate an immune response against a variety of parasitic, bacterial and viral infections. VEGI polypeptides may lyse virus-infected cells and, therefore, be employed to arrest HIV infected cells.

The VEGI polypeptide may also be employed to treat autoimmune diseases such as Type I diabetes by enhancing the T-cell proliferative response.

**Table 2**

| Summary of VEGI activity | | | | | |
|---|---|---|---|---|---|
| Cell lines Differentiation | Source and type Adhesion | Cytotoxicity | | Proliferation | |
| | mouse | | | | |
| L929 | fibroblast | + | - | - | - |
| | mouse | | | | |
| WEHI 164 | fibrosarcoma | +++ | - | - | - |
| | rat kidney | | | | |
| NRK-54E | epithelial-like | + | - | - | - |
| | human | | | | |
| THP-1 | monocytic | + | - | ++ | ++ |
| | leukemia | | | | |
| | human | | | | |
| HL-60 | promyelocytic | - | - | - | ++ |
| | leukemia | | | | |
| | human | | | | |
| Raji | Burkitt | - | - | - | - |
| | lymphoma | | | | |
| | human | | | | |
| Jurkat | T-cell | ++ | - | - | - |
| | lymphoma | | | | |
| Primary | HUVEC | - | ++ | - | ? |
| | human aterial | | | | |
| Primary | endothelial | +* | ++ | - | ? |
| | human | | | | |
| A-431 | epidermoid | ++ | - | - | - |
| | carcinoma | | | | |
| | human | | | | |
| 293 | embryonal | - | ++ | - | - |
| | kidney | | | | |

| | | | | | |
|---|---|---|---|---|---|
| Legend: * At high dose only. The numbers of "+" indicate the relative level of activities. "-" indicates no detected activity at the concentration range tested. | | | | | |

VEGI may be used to inhibit the proliferation of enodothelial cells, for example, aortic endothelial cells. At concentrations of up to 10 µg/ml, VEGI can nearly completely inhibit the growth of endothelial cells while having no apparent effect on the growth of human breast cancer cells. As a result, VEGI can be used to treat diseases and disorders in which inhibition of endothelial cell growth is advantageous.

VEGI polypeptides of the present invention have been used to reduce the formation of capillary-like tubular structures formed by endothelial cells *in vitro.* VEGI polypeptides of the present invention can be used to inhibit the formation of endothelial cells organized into capillary-like tubular structures in response to angiogenic factors such as FGF-2. Furthermore, isolated VEGI of the present invention can also be used to inhibit the growth and organization of endothelial cells into capillary vessels in a modified chicken embryo chorioallantoic membrane (CAM). As a result, VEGI of the present invention can be used to inhibit the formation of capillaries or capillary-like structures from endothelial cells *in vitro.*

The polynucleotides and polypeptides of the present invention may be employed as research reagents and materials for discovery of treatments and diagnostics to human disease.

This invention provides a method for identification of the receptor for VEGI. The gene encoding the receptor can be identified by numerous methods known to those of skill in the art, for example, ligand panning and FACS sorting (Coligan, et al., Current Protocols in Immun., 1(2), Chapter 5, (1991)). Preferably, expression cloning is employed wherein polyadenylated RNA is prepared from a cell responsive to VEGI, and a cDNA library created from this RNA is divided into pools and used to transfect COS cells or other cells that are not responsive to VEGI. Transfected cells which are grown on glass slides are exposed to labeled VEGI. VEGI can be labeled by a variety of means including iodination or inclusion of a recognition site for a site-specific protein kinase. Following fixation and incubation, the slides are subjected to autoradiographic analysis. Positive pools are identified and sub-pools are prepared and retransfected using an iterative sub-pooling and rescreening process, eventually yielding a single clone that encodes the putative receptor.

As an alternative approach for receptor identification, labeled VEGI can be photoaffinity-linked with cell membrane or extract preparations that express the receptor molecule. Cross-linked material is resolved by PAGE and exposed to X-ray film. The labeled complex containing the VEGI-receptor can be excised, resolved into peptide fragments, and subjected to protein microsequencing. The amino acid sequence obtained from microsequencing would be used to design a set of degenerate oligonucleotide probes to screen a cDNA library to identify the gene encoding the putative receptor.

The VEGI polypeptide composition will be formulated and dosed in a fashion consistent with good medical practice, taking into account the clinical condition of the individual patient (especially the side effects of treatment with VEGI polypeptide alone), the site of delivery of the VEGI polypeptide composition, the method of administration, the scheduling of administration, and other factors known to practitioners. The "effective amount" of VEGI polypeptide for purposes herein is thus determined by such considerations.

The antagonists may be employed in a composition with a pharmaceutically acceptable carrier, e.g., as hereinafter described.

The VEGI polypeptides and agonists and antagonists of the present invention may be employed in combination with a suitable pharmaceutical carrier. Such compositions comprise a therapeutically effective amount of the compound, and a pharmaceutically acceptable carrier or excipient Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the pharmaceutical compositions of the present invention may be employed in conjunction with other. therapeutic compounds.

The pharmaceutical compositions may be administered in a convenient manner such as by the topical, intravenous, intraperitoneal, intramuscular, subcutaneous, intranasal or intradermal routes. The pharmaceutical compositions are administered in an amount which is effective for treating and/or prophylaxis of the specific indication. In general, they are administered in an amount of at least about 10 g/kg body weight and in most cases they will be administered in an amount not in excess of about 8 mg/Kg body weight per day. In most cases, the dosage is from about 10 g/kg to about 1 mg/kg body weight daily, taking into account the routes of administration, symptoms, etc.

Various delivery systems are known and can be used to administer VEGI-alpha and/or VEGI-beta polypeptides of the present invention, e.g., encapsulation in liposomes, microparticles, microcapsules, receptor-mediated endocytosis (see, e.g., Wu and Wu 1987, J. Biol. Chem. 262:4429-4432). Methods of introduction include, but are not limited to, topical, intradermal, intramuscular, intraperitoneal, intravenous, subcutaneous, intranasal, epidural, ophthalmic, and oral routes. The compounds may be administered by any convenient route, for example, by infusion or bolus injection, by absorption through epithelial or mucocutaneous linings (e.g., oral mucosa, rectal and intestinal mucosa, etc.) and may be administered together with other biologically active agents.

In a specific embodiment, it may be desirable to administer the pharmaceutical compositions of the invention locally to the area in need of treatment. This may be achieved by, for example, and not in limitation of, local infusion during surgery, topical application, e.g., in conjuction with a wound dressing, by injection, by means of a catheter, by means of a suppository, or by means of an implant, said implant being of a porous, non-porous, or gelatinous material, including membranes, such as sialastic membranes or fibers.

A topical application of a VEGI-alpha and/or VEGI-beta polypeptide of the present invention for treatment of at least some of the eye disorders discussed herein consists of an effective amount of the VEGI-alpha and/or VEGI-beta polypeptide of the present invention in an ophthalmologically acceptable excipient such as buffered saline, mineral oil, vegetable oils (such as, for example, corn or arachis oils), petroleum jelly, Miglyol 182, alcohol solutions, or liposomes or iposome-like products. Any of these compositions may also include preservatives, antioxidants, antibiotics, immunosuppressants, and other biologically or pharmaceutically effective agents which do not exert a detrimental effect on the VEGI-alpha and/or VEGI-beta polypeptide of the present invention.

The VEGI polypeptides and agonists and antagonists which are polypeptides may also be employed in accordance with the present invention by expression of such polypeptides *in vivo,* which is often referred to as "gene therapy."

Thus, for example, cells from a patient may be engineered with a polynucleotide (DNA or RNA) encoding a polypeptide ex vivo, with the engineered cells then being provided to a patient to be treated with the polypeptide. Such methods are well-known in the art and are apparent from the teachings herein. For example, cells may be engineered by the use of a retroviral particle containing RNA encoding a polypeptide of the present invention.

Similarly, cells may be engineered in *vivo* for expression of a *polypeptide in vivo* by, for example, procedures known in the art. For example, a producer cell for producing a retroviral particle containing RNA encoding a polypeptide of the present invention may be administered to a patient for engineering cells *in vivo* and expression of the *polypeptide in vivo.* These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells *in vivo* after combination with a suitable delivery vehicle.

Retroviruses from which the retroviral plasmid vectors hereinabove mentioned may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

The vector includes one or more promoters. Suitable promoters which may be employed include, but are not limited to, the retroviral LTR; the SV40 promoter; and the human cytomegalovirus (CMV) promoter described by Miller and colleagues (Biotechniques 7:980-990 (1989)), or any other promoter (e.g., cellular promoters such as eukaryotic cellular promoters including, but not limited to, the histone, pol III, and b-actin promoters). Other viral promoters which may be employed include, but are not limited to, adenovirus promoters, thymidine kinase (TK) promoters, and B 19 parvovirus promoters. The selection of a suitable promoter will be apparent to those skilled in the art from the teachings contained herein.

The nucleic acid sequence encoding the polypeptide of the present invention is under the control of a suitable promoter. Suitable promoters which may be employed include, but are not limited to, adenoviral promoters, such as the adenoviral major late promoter; or hetorologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters; such as the Herpes Simplex thymidine kinase promoter; retroviral LTRs (including the modified retroviral LTRs hereinabove described); the b-actin promoter; and human growth hormone promoters. The promoter also may be the native promoter which controls the gene encoding the polypeptide.

The retroviral plasmid vector is employed to transduce packaging cell lines to form producer cell lines. Examples of packaging cells which may be transfected include, but are not limited to, the PE501, PA317, b-2, b-AM, PA12, T19-14X, VT-19-17-H2, CRE, β-CRIP, GP+E-86, GP+envAm12, and DAN cell lines as described by Miller (Human Gene. Therapy 1:5-14 (1990)), which is incorporated herein by reference in its entirety. The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line generates infectious retroviral vector particles which include the nucleic acid sequence(s) encoding the polypeptides. Such retroviral vector particles then may be employed, to transduce eukaryotic cells, either *in vitro* or *in vivo.* The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

This invention is also related to a method of screening compounds to identify those which mimic VEGI (agonists) or prevent the effect of VEGI (antagonists). An example of such a method takes advantage of the ability of VEGI polypeptides to significantly stimulate the proliferation of human endothelial cells in the presence of the comitogen Con A. Endothelial cells are obtained and cultured in 96-well flat-bottomed culture plates (Costar, Cambridge, MA) in RPMI 1640 supplemented with 10% heat-inactivated fetal bovine serum (Hyclone Labs, Logan, UT), 1% L-glutamine, 100 U/ml penicillin, 100 g/ml steptomycin, 0.1% gentamycin (Gibco Life Technologies, Grand Island, NY) in the presence of 2 g/ml of Con-A (Calbiochem, La Jolla, CA). Con-A, and the compound to be screened are added to a final volume of 0.2 ml. After 60 h at 37°C, cultures are pulsed with 1 Ci of [³H]thymidine (5 Ci/mmol; 1 Ci = 37 BGq; NEN) for 12-18 h and harvested onto glass fiber filters (PhD; Cambridge Technology, Watertown, MA). Mean [3H]thymidine incorporation (cpm) of triplicate cultures is determined using a liquid scintillation counter (Beckman Instruments, Irvine, CA). Significant [³H]-thymidine incorporation indicates stimulation of endothelial cell proliferation.

Alternatively, the response of a known second messenger system following interaction of VEGI polypeptides and receptor would be measured and compared in the presence or absence of the compound. Such second messenger systems include but are not limited to, cAMP guanylate cyclase, ion channels or phosphoinositide hydrolysis.

To assay for antagonists, the assay described above is performed, however, in this assay VEGI is added along with the compound to be screened and the ability of the compound to inhibit [³H]thymidine incorporation in the presence of VEGI polypeptides, indicates that the compund is an antagonist to VEGI. Alternatively, VEGI antagonists may be detected by combining VEGI and a potential antagonist with membrane-bound VEGI receptors or recombinant receptors under appropriate conditions for a competitive inhibition assay. VEGI can be labeled, such as by radioactivity, such that the number of VEGI molecules bound to the receptor can determine the effectiveness of the potential antagonist.

Alternatively, a mammalian cell or membrane preparation expressing the VEGI receptor is incubated with labeled VEGI in the presence of the compound. The ability of the compound to enhance or block this interaction could then be measured.

In another aspect of this embodiment the invention provides a method for identifying a receptor protein or other ligand-binding protein which binds specifically to a VEGI polypeptide (e.g. DR3). For example, a cellular compartment, such as a membrane or a preparation thereof, may be prepared from a cell that expresses a molecule that binds VEGI polypeptides. The preparation is incubated with labeled VEGI polypeptides and complexes of VEGI polypeptide bound to the receptor or other binding protein are isolated and characterized according to routine methods known in the art. Alternatively, the VEGI polypeptide may be bound to a solid support so that binding molecules solubilized from cells are bound to the column and then eluted and characterized according to routine methods.

In the assay of the invention for agonists or antagonists, a cellular compartment, such as a membrane or a preparation thereof, may be prepared from a cell that expresses a molecule that binds VEGI, such as a molecule of a signaling or regulatory pathway modulated by VEGI. The preparation is incubated with labeled VEGI polypeptide in the absence or the presence of a candidate molecule which may be a VEGI agonist or antagonist. The ability of the candidate molecule to bind the binding molecule is reflected in decreased binding of the labeled ligand. Molecules which bind gratuitously, i.e., without inducing the effects of VEGI on binding the VEGI binding molecule, are most likely to be good antagonists. Molecules that bind well and elicit effects that are the same as or closely related to VEGI are agonists.

VEGI-like effects of potential agonists and antagonists may by measured, for instance, by determining activity of a second messenger system following interaction of the candidate molecule with a cell or appropriate cell preparation, and comparing the effect with that of VEGI or molecules that elicit the same effects as VEGI. Second messenger systems that may be useful in this regard include but are not limited to AMP guanylate cyclase, ion channel or phosphoinositide hydrolysis second messenger systems.

Another example of an assay for VEGI antagonists is a competitive assay that combines VEGI polypeptide and a potential antagonist with membrane-bound VEGI receptor molecules or recombinant VEGI receptor molecules under appropriate conditions for a competitive inhibition assay. VEGI polypeptide can be labeled, such as by radioactivity, such that the number of VEGI molecules bound to a receptor molecule can be determined accurately to assess the effectiveness of the potential antagonist.

Potential antagonists include small organic molecules, peptides, polypeptides and antibodies that bind to a polypeptide of the invention and thereby inhibit or extinguish its activity. Potential antagonists also may be small organic molecules, a peptide, a polypeptide such as a closely related protein or antibody that binds the same sites on a binding molecule, such as a receptor molecule, without inducing VEGI-induced activities, thereby preventing the action of VEGI by excluding VEGI from binding.

Other potential antagonists include antisense molecules. Antisense technology can be used to control gene expression through antisense DNA or RNA or through triple-helix formation. Antisense techniques are discussed in a number of studies (for example, Okano, J. Neurochem. 56:560 (1991); "Oligodeoxynucleotides as Antisense Inhibitors of Gene Expression." CRC Press, Boca Raton, FL (1988)). Triple helix formation is discussed in a number of studies, as well (for instance, Lee, et al., Nucleic Acids Research 6:3073 (1979); Cooney, et al., Science 241:456 (1988); Dervan, et al., Science 251:1360 (1991)). The methods are based on binding of a polynucleotide to a complementary DNA or RNA. For example, the 5' coding portion of a polynucleotide that encodes the mature polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of VEGI. The antisense RNA oligonucleotide hybridizes to the mRNA *in vivo* and blocks translation of the mRNA molecule into VEGI polypeptide. The oligonucleotides described above can also be delivered to cells such that the antisense RNA or DNA may be expressed *in vivo* to inhibit production of VEGI polypeptide.

Antibodies specific to VEGI polypeptides may be used as antagonists by binding to VEGI and preventing it from binding to its receptor. Monoclonal antibodies are particularly effective in this regard. Antibodies specific to the VEGI receptor, however, may mediate distinct cellular responses which tend to agonize the effects of VEGI upon interaction with its receptor.

Potential VEGI antagonists also include VEGI mutants which bind to the VEGI receptor and elicit no second messenger response to effectively block the receptor from its natural ligand. Specifically designed oligonucleotides and small molecules may also bind to the VEGI receptor (e.g., DR3) and block it from VEGI. Examples of small molecules include but are not limited to small peptides or peptide-like molecules.

Another potential VEGI antagonist is a soluble form of the VEGI receptor which binds to VEGI and prevents it from interacting with membrane-bound VEGI receptors. In this way, the receptors are not stimulated by VEGI.

Another potential VEGI antagonist is an antisense construct prepared using antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of VEGI. The antisense RNA oligonucleotide hybridizes to the mRNA *in vivo* and blocks translation of the mRNA molecule into the VEGI polypeptide (Antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of VEGI.

VEGI-antagonists may also be employed to treat cachexia which is a lipid clearing defect resulting from a systemic deficiency of lipoprotein lipase which is suppressed by VEGI. The VEGI antagonists are also employed to treat cerebral malaria in which VEGI appears to play a pathogenic role.

The VEGI antagonists may also be employed to prevent graft rejection by preventing the stimulation of the immune system in the presence of a graft by VEGI.

The VEGI antagonists may also be employed to treat osteoporosis since VEGI may induce bone resorption.

Antagonists to VEGI may also be employed as anti-inflammation agents since VEGI mediates an enhanced inflammatory response.

The antagonists may also be used to treat endotoxic shock, also referred to as septic shock. This critical condition results from an exaggerated response to bacterial or other types of infection. This response leads to elevated levels of VEGI which causes shock and tissue injury.

The present invention also relates to a diagnostic assay for detecting altered levels of VEGI polypeptide in various tissues since an over-expression of the proteins compared to normal control tissue samples may detect the presence of a disease or susceptibility to a disease, for example, cerebral malaria. Assays used to detect levels of VEGI polypeptide in a sample derived from a host are well-known to those of skill in the art and include radioimmunoassays, competitive-binding assays, Western Blot analysis, ELISA assays and "sandwich" assay. An ELISA assay (Coligan, et al., Current Protocols in Immunology, 1 (2), Chapter 6, (1991)) initially comprises preparing an antibody specific to the VEGI antigen, preferably a monoclonal antibody. In addition a reporter antibody is prepared against the monoclonal antibody. To the reporter antibody is attached a detectable reagent such as radioactivity, flourescence or in this example a horseradish peroxidase enzyme. A sample is removed from a host and incubated on a solid support, e.g. a polystyrene dish, that binds the proteins in the sample. Any free protein binding sites on the dish are then covered by incubating with a non-specific protein like BSA. Next, the monoclonal antibody is incubated in the dish during which time the monoclonal antibodies attach to any VEGI polypeptides attached to the polystyrene dish. All unbound monoclonal antibody is washed out with buffer. The reporter antibody linked to horseradish perosxidase is now placed in the dish resulting in binding of the reporter antibody to any monoclonal antibody bound to VEGI. Unattached reporter antibody is then washed out. Peroxidase substrates are then added to the dish and the amount of color developed in a given time preiod is a measurement of the amount of VEGI polypeptide present in a given volume of patient sample when compared against a standard curve.

A competition assay may be employed wherein antibodies specific to VEGI polypeptides are attached to a solid support and labeled VEGI and a sample derived from the host are passed over the solid support and the amount of label detected, for example by liquid scintillation chromotagraphy, can be correlated to a quantity of VEGI in the sample.

A "sandwich" assay is similar to an ELISA assay. In a "sandwich" assay VEGI is passed over a solid support and binds to antibody attached to a solid support. A second antibody is then bound to the VEGI. A third antibody which is labeled and specific to the second antibody is then passed over the solid support and binds to the second antibody and an amount can then be quantitated.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region of the sequence is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA as short as 50 or 60 bases. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

Utilizing the techniques described above, the chromosomal location of VEGI was determined with very high confidence to be 9q32. Previous chromosomal mapping studies have linked several developmental defects to loci in this area of chromosome 9.

### Examples

Described below are examples of the present invention which are provided only for illustrative purposes, and not to limit the scope of the present invention. In light of the present disclosure, numerous embodiments within the scope of the claims will be apparent to those of ordinary skill in the art.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures, unless otherwise stated. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37 C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. et al., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1: Bacterial Expression and Purification of VEGI Polypeptides.

An expression vector containing a polynucleotide insert which encodes a 38 amino acid deletion from the N-terminal sequence of the predicted full length VEGI-alpha polypeptide was constructed. The DNA sequence encoding the N-terminally deleted VEGI-alpha polynucleotide was amplified using PCR oligonucleotide primers specific to the coding sequence of the VEGI-alpha coding sequence. Additional nucleotides generating restriction sites to facilitate cloning were added to the 5' and 3' sequences, respectively. The 5' oligonucleotide primers had the 5'-GCG CCA TGG CTC TGC ACT GGG AAC AT-3' (SEQ ID NO:3) containing a *Nco* I restriction site, followed by 18 nucleotides of coding sequence. The 3' primer has the sequence 5'-CGC AAG CTT CTA TAG TAA GAA GGC TCC-3' (SEQ ID NO:4) contains a *Hin* dIII restriction site followed by 18 nucleotides complementary to the last 15 nucleotides of the coding sequence and the stop codon. The amplified VEGI-alpha DNA product was cloned into vector pQE60 (Qiagen) after digestion with the appropriate restriction enzymes and subsequent ligation. The ligation mixture was transformed into competent *E. coli* cells (strain M15/rep4). Clones containing the desired constructs were grown to an optical density at 600 nm of between 0.4 and 0.6. Isopropyl-B-D-thiogalactopyranoside (IPTG) was then added to a final concentration of 1 mM to induce the expression of protein. After 3 hours, cells were harvested and inclusion bodies were purified from the disrupted cells. and protein was solubilized from the inclusion bodies into 8M urea. The solubilized protein was passed over a PD-10 column in 2X phosphate buffered saline (PBS), thereby removing the urea exchanging the buffer and tefolding the protein. The protein was purified by a further step of chromatography to remove endotoxin. The resulting VEGI-alpha polypeptide preparation was found to be more than 90% pure by SDS-PAGE and contained <10 EU endotoxin/mg.

In addition, the DNA sequence encoding the full-length VEGI-alpha ORF, ATCC Deposit No. 75927, was initially amplified using PCR oligonucleotide primers corresponding to the 5' and 3' sequences of the VEGI-alpha protein. Additional nucleotides corresponding to VEGI-alpha were added to the 5' and 3' sequences respectively. The 5' oligonucleotide primer is shown as SEQ ID NO:13 and has the sequence 5'-GCG CGG ATC CAC CAT GAG ACG CTT TTT AAG CAA AGT C-3' which contains a *Bam* HI restriction enzyme site followed by the first 24 nucleotides of VEGI-alpha coding sequence starting from the initiating methionine codon. The 3' sequence 5'-CGC GTC TAG ACT ATA GTA AGA AGG CTC CAA AGA AGG-3' (SEQ ID NO:14) contains sequences complementary to an *Xba* I site and 22 nucleotides of VEGI-alpha. The restriction enzyme sites correspond to the restriction enzyme sites in the bacterial expression vector pQE-9 (Qiagen). pQE-9 was then digested with Bam HI and *Xba* I. The amplified sequences were ligated into pQE-9 and were inserted in frame with the sequence encoding for the histidine tag and the RBS. The ligation mixture was then used to transform an *E. coli* strain available from Qiagen under the trademark M 15/rep 4 by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan^{r}). Transformants were identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture was used to inoculate a large culture at a ratio of 1:100 to 1:250. The cells were grown to an optical density 600 (O.D.₆₀₀) of between 0.4 and 0.6. IPTG ("Isopropyl-B-D-thiogalactopyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation. The cell pellet was solubilized in the chaotropic agent 6 M Guanidine HCl (Guanidine HCl concentrations of greater than or equal to 2.5 M were empirically found to resulat in a higher level of purity of recovered recombinant protein). After clarification, solubilized VEGI-alpha polypeptide was purified from this solution by chromatography on a Nickel-Chelate column under conditions that allow for tight binding by proteins containing the 6-His tag (Hochuli, E. et al., J. Chromatography 411:177-184 (1984)). VEGI-alpha polypeptide was further purified by a second run on the Nickel-chelate column. VEGI-alpha polypeptide (90% pure) was eluted from the column in 6 M guanidine HCl pH 5.0 and for the purpose of renaturation was dialyzed in PBS buffer. The expression product was electrophoresed by SDS-PAGE.

One of ordinary skill in the art will recognize that bacterial expression vectors other than pQE-9 may also be used to express VEGI polypeptides. One such preferred bacterial expression vector is pHE4-5. pHE4-5 may be obtained as pHE4-5/MPIFD23 plasmid DNA (this construct contains an unrelated insert which encodes an unrelaed ORF). The pHE4-5/MPIFD23 plasmid was deposited with the American Type Culture Collection, 12301 Park Lawn Drive, Rockville, Maryland 20852, on September 30, 1997 (Accession No. 209311). Using the *Nde I* and *Asp* 718 restriction sites flanking the unrelated MPIF ORF insert, one of ordinary skill in the art could easily use current molecular biological techniques to replace the unrelated ORF in the pHE4-5/MPIFD23 plasmid with the VEGI ORF, or variations thereof, of the present invention.

### Example 2: Cloning and expression of VEGI Polypeptides using the baculovirus expression system.

The DNA sequence encoding the full length VEGI-alpha polypeptide, ATCC No. 75927, was amplified using PCR oligonucleotide primers corresponding to the 5' and 3' sequences of the gene: The 5' primer has the sequence 5'-GCG C**GG ATC** CAC CAT GAG ACG CTT TTT AAG CAA AGT C-3' (SEQ ID NO:15) and contains a *Bam* HI restriction enzyme site (in bold) followed by 24 nucleotides of the VEGI-alpha coding sequence (the initiation codon for translation "ATG" is underlined). The 3' primer has the sequence 5'-CGC GTC TAG ACT ATA GTA AGA AGG CTC CAA AGA AGG-3' (SEQ ID NO:16) and contains the cleavage site for the restriction endonuclease *Xba* I and 22 nucleotides complementary to the 3' non-translated sequence of the VEGI-alpha coding sequence. The amplified sequences were isolated from a 1% agarose gel using a commercially available kit ("Geneclean," BIO 101 Inc., La Jolla, Ca.). The fragment was then digested with the endonucleases *Bam* HI and *Xba* I and then purified again on a 1% agarose gel. This fragment was designated F2.

The vector pA2 (modification of pVL941 vector, discussed below) was used for the expression of the VEGI-alpha polypeptide using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the *Autographa californica* nuclear polyhedrosis virus (AcMNPV) followed by the recognition sites for the restriction endonucleases *Bam* HI and *Xba* I. The polyadenylation site of the simian virus SV40 is used for efficient polyadenylation. For an easy selection of recombinant virus the beta-galactosidase gene from *E.coli* was inserted in the same orientation as the polyhedrin promoter followed by the polyadenylation signal of the polyhedrin gene. The polyhedrin sequences were flanked at both sides by viral sequences for the cell-mediated homologous recombination of cotransfected wild-type viral DNA. Many other baculovirus vectors could have been used in place of pA2, such as pRG1, pAc373, pVL941 and pAcIM1 (Luckow, V.A. and Summers, M.D., Virology, 170:31-39).

The plasmid was digested with the restriction enzymes *Bam* HI and *Xba* I and then dephosphorylated using calf intestinal phosphatase by procedures known in the art. The DNA was then isolated from a 1% agarose gel using the commercially available kit ("Geneclean" BIO 101 Inc., La Jolla, Ca.). This vector DNA was designated V2.

Fragment F2 and the dephosphorylated plasmid V2 were ligated with T4 DNA ligase. *E. coli* XL1 blue cells were then transformed. The sequence of the cloned fragment was confirmed by DNA sequencing.

5 µg of the plasmid pBac VEGI-alpha was cotransfected with 1.0 µg of a commercially available linearized baculovirus ("BaculoGold baculovirus DNA", Pharmingen, San Diego, CA.) using the lipofection method (Felgner et al. Proc. Natl. Acad. Sci. USA, 84:7413-7417 (1987)).

1 µg of BaculoGold virus DNA and 5 µg of the plasmid pBac VEGI-alpha were mixed in a sterile well of a microtiter plate containing 50 µl of serum free Grace's medium (Life Technologies Inc., Gaithersburg, MD). Afterwards, 10 µl Lipofectin plus 90 µl Grace's medium were added, mixed and incubated for 15 minutes at room temperature. Then the transfection mixture was added dropwise to the Sf9 insect cells (ATCC CRL 1711) seeded in a 35 mm tissue culture plate with 1ml Grace' medium without serum. The plate was rocked back and forth to mix the newly added solution. The plate was then incubated for 5 hours at 27°C. After 5 hours, the transfection solution was removed from the plate and 1 ml of Grace's insect medium supplemented with 10% fetal calf serum was added. The plate was put back into an incubator and cultivation continued at 27°C for four days.

After four days, the supernatant was collected and a plaque assay performed essentially as described by Summers and Smith *(supra).* As a modification, an agarose gel with "Blue Gal" (Life Technologies Inc., Gaithersburg) was used which allows an easy isolation of blue stained plaques. (A detailed description of a "plaque assay" can also be found in the user's guide for insect cell culture and baculovirology distributed by Life Technologies Inc., Gaithersburg, page 9-10).

Four days after the serial dilution, the virus was added to the cells, blue stained plaques were picked with the tip of an Eppendorf pipette. The agar containing the recombinant viruses was then resuspended in an Eppendorf tube containing 200 µl of Grace's medium. The agar was removed by a brief centrifugation and the supernatant containing the recombinant baculovirus was used to infect Sf9 cells seeded in 35 mm dishes. Four days later the supernatants of these culture dishes were harvested and then stored at 4°C.

Sf9 cells were grown in Grace's medium supplemented with 10% heat-inactivated FBS. The cells were infected with the recombinant baculovirus V-VEGI-alpha at a multiplicity of infection (MOI) of 2. Six hours later the medium was removed and replaced with SF900 II medium minus methionine and cysteine (Life Technologies Inc., Gaithersburg). 42 hours later 5 µCi of [³⁵S]-methionine and 5 µCi [³⁵S]-cysteine (Amersham) were added. The cells were further incubated for 16 hours before they were harvested by centrifugation and the labeled proteins visualized by SDS-PAGE and autoradiography.

### Example 3: Expression of Recombinant VEGI-alpha in Mammalian Cells.

The chinese hamster ovary (CHO) dihydrofolate reductase-negative (DHFR-) cell line was purchased from the American Type Culture Collection. Cloning of the VEGI-alpha cDNA into the pCl Chinese Hamster Ovarian Cell expression vector was carried out essentially as follows. The human cDNA sequence encoding the full length VEGI-alpha polypeptide was amplified using primers specific to the 5- and 3'-end of the VEGI-alpha coding sequence (5-Primer: GCG gga tcc GCC ACC ATG AAC TCC TTC TCC ACA AGC GCC TTC GGT CCA GTT GCC TTC TCC CTG GGG CTG CTC CTG GTG TTG CCT GCT GCC TTC CCT GCC CCA GTT GTG AGA C (SEQ ID NO:5), containing a *Bam* HI restriction site followed by a "Kozak" sequence (lowercase letters above) and the first 84 bases of the IL6 coding sequence and 13 bases of the VEGI-alpha coding sequence; 3'-primer: CGC gga tcc GAT ATT TGC TCT CCT CCT CA (SEQ ID NO:6), containing a *Bam* HI restriction site) followed by 17 nucleotides of the untranslated area and a stop codon. The amplified fragment was gel purified and digested with *Bam* HI. The CHO cell expression vector (pCl) was digested with *Bam* HI followed by agarose gel purification. The amplified VEGI-alpha coding sequence was ligated into the purified pCl vector using T4 DNA ligase. HB101 cells were subsequently transformed. A positive clone (pC1VEGI) was identified by PCR screening/enzyme digestion and the DNA sequence of the insert was confirmed using automatic DNA sequencing. The selection and amplification of stable CHO cell clones was performed as previously described by R. Gentz, et al, Eur. LT. Biochemistry 210. 545 (1992). CHO stable transfectants CHO-VEGI-alpha and CHO-Vector were maintained in MEM-a medium containing 10% FCS (dialyzed).

The expression of plasmid, VEGI-alpha-HA is derived from a vector pcDNAI/Amp (Invitrogen) containing: 1) SV40 origin of replication, 2) ampicillin resistance gene, 3) E.coli replication origin, 4) CMV promoter followed by a polylinker region, an SV40 intron, and a polyadenylation site. A DNA fragment encoding the entire VEGI-alpha precursor and a hemagglutinin antigen (HA) tag fused in frame to its 3' end was cloned into the polylinker region of the vector. Therefore, the recombinant protein expression is under the direction of the CMV promoter. The HA tag corresponds to an epitope derived from the influenza hemagglutinin protein as previously described (I. Wilson, H. Niman, R. Heighten, A Cherenson, M. Connolly, and R. Lemer, 1984, Cell 37, 767). The fusion of HA tag to our target protein allows easy detection of the recombinant protein with an antibody that recognizes the HA epitope.

The plasmid construction strategy is described as follows: The DNA sequence encoding VEGI-alpha, ATCC # 75927, was constructed by PCR on the original EST cloned using two primers: the 5' primer (SEQ ID NO:15) contains a *Bam* HI site followed by 24 nucleotides of VEGI-alpha coding sequence starting from the initiation codon; the 3' sequence 5'-CGC TCT AGA TCA AGC GTA GTC TGG GAC GTC GTA TGG ATA GTA AGA AGG CTC CAA AG-3' (SEQ ID NO:17) contains complementary sequences to *Xba* I site, translation stop codon, HA tag and the last 18 nucleotides of the VEGI-alpha coding sequence (not including the stop codon). Therefore; the PCR product contained a *Bam* HI site, VEGI-alpha coding sequence followed by HA tag fused in frame, a translation termination stop codon next to the HA tag, and an *Xba* I site. The PCR amplified DNA fragment and the vector, pcDNAI/Amp, were digested with Bam HI and *Xba* I restriction enzymes and ligated together. The ligation mixture was transformed into E. *coli* strain SURE (available from Stratagene Cloning Systems, 11099 North Torrey Pines Road, La Jolla, CA 92037) the transformed culture was plated on ampicillin media plates and resistant colonies were selected. Plasmid DNA was isolated from transformants and examined by restriction analysis for the presence of the correct fragment. For expression of the recombinant VEGI-alpha polypeptides, COS cells were transfected with the expression vector by DEAE-DEXTRAN method. (J. Sambrook, E. Fritsch, T. Maniatis, Molecular Cloning: A Laboratory Manual, Cold Spring Laboratory Press, (1989)). The expression of the VEGI-alpha-HA protein was detected by radiolabeling and immunoprecipitation method. (E. Harlow, D. Lane, Antibodies: A Laboratory Manual, Cold Spring Harbor Laboratory Press, (1988)). Cells were labeled for 8 hours with [³⁵S]-S-cysteine two days post transfection. Culture media were then collected and cells were lysed with detergent (RIPA buffer (150 mM NaCl, 1% NP-40, 0.1 % SDS, 1% NP-40, 0.5% DOC, 50mM Tris, pH 7.5; Wilson, I. et al., *Id.* 37:767 (1984)). Both cell lysate and culture media were precipitated with an HA-specific monoclonal antibody. Precipitated proteins were then analyzed on 15% SDS-PAGE gels.

### Example 4: Expression Pattern of VEGI in a Variety of Cells and Cell Types.

RNA blot analysis was carried out to examine the levels of expression of VEGI in human tissues. Total cellular RNA samples were isolated with RNAzol™ B system (Biotecx Laboratories, Inc. 6023 South Loop East, Houston, TX 77033). About 2 µg of total RNA isolated from each human tissue specified was separated on 1% agarose-formaldehyde gel and blotted onto a nylon filter (Sambrook, Fritsch, and Maniatis, Molecular Cloning, Cold Spring Harbor Press, (1989)). The labeling reaction was done according to the Stratagene Prime-It kit with 50 ng VEGI-alpha cDNA, to produce [³²P]-labeled VEGI-alpha cDNA. The labeled DNA was purified with a Select-G-50 column (5 Prime - 3 Prime, Inc. 5603 Arapahoe Road, Boulder, CO 80303). The filter was then hybridized with radioactive labeled full-length VEGI-alpha probe at 1,000,000 cpm/ml in 0.5 M NaPO₄, pH 7.4 and 7% SDS overnight at 65°C. After being washed twice at room temperature and twice at 60°C with 0.5 x SSC, 0.1% SDS, the X-ray film was then exposed to the blot at -70°C overnight with an intensifying screen. The message RNA for VEGI-alpha is abundant in kidney.

VEGI is specifically expressed in endothelial cells, as confirmed by Northern blotting analysis of total RNA preparations from 22 different types of cultured cells of various lineages (Figure lA). VEGI message was only detected in primary cultures of two types of endothelial cells: HUVE cells and human venous endothelial cells of an early passage. The mRNA was not detected in human venous endothelial cells of a later passage, nor was it seen in human artery cells. In sharp contrast, the TNF family members are mostly expressed in immune cells (B.B. Aggarwal and K. Natarajan, supra). For instance. TNF-α is produced by macrophages, monocytes, neutrophils, and T cells, while TNF-β is predominantly produced by mitogenstimulated T lymphocytes and leukocytes. Similarly, the ligands for Fas, CD27, CD30, CD40, OX40, and 4-1 BB are all expressed in cell types in the immune system.

Using multiple tissue Northern blots, the VEGI transcript was expressed in placenta, lung, kidney, skeletal muscle, pancreas, spleen, prostate, small intestine, and colon. Minimal amounts of VEGI signal were detected in heart, brain, liver, thymus, testis, ovary and peripheral blood lymphocytes (Figure 1B). The expression of VEGI in most major organs suggests that the gene product may play a role in the function of the normal vasculature.

Northern blot analyses were also performed to determine the relative expression level of the VEGI RNA with respect to the proliferation state of HUVEC cell cultures. In these experiments, identical amounts of total RNA isolated from HUVEC cells (15 µg) were electrophoretically separated and blotted as described above. RNA was isolated from cultures 1, 2, 3, 4, 6, and 7 days post-seeding. VEGI RNA was only seen at low levels in newly seeded cultures (days 1, 2, and 3). However, expression of VEGI RNA was apparent as the HUVEC cells in the cultures began to reach confluence (days 4, 6, and 7). These experiments indicate that VEGI expression increases as cells in a culture or tissue begin to reach the quiescent state of non- or reduced- proliferation.

### Example 5: VEGI-Mediated Inhibition of ABAE Cell Proliferation.

Cells were seeded at appropriate densities (ABAE cells, 8,000 cells/well; MDA-MB-231. 2,000 cells/well; MDA-MB-435, 2,000 cells/well cells in triplicate in 24-well plates. The culture media contained IMEM (Gibco) and 10% FCS. FGF-2 (1 ng/ml) was present in the culture media for ABAE cells. The cultures were maintained at 37°C, 5% C0₂, for 6 days. The cells were trypsinized, and the number of cells determined by using a Coulter counter. One-fifth of the total number of recovered ABAE cells were used to normalize the comparison with the cancer cells.

A truncated version of VEGI-alpha corresponding to the putative extracellular domain consisting of residues 38-174 was expressed in *E. coli,* as described above, purified, and examined in a variety of cellular assays. The truncated polypeptide was found to specifically inhibit the proliferation of endothelial cells (Figure 2). Nearly complete inhibition of the growth of adult bovine aortic endothelial (ABAE) cells was achieved at 10 mg/ml, with a half-maximum inhibition concentration value (IC 50) of about 1 mg/ml (about 70 nM). The activity of the recombinant polypeptide is comparable with that of angiostatin, endostatin, and platelet factor 4. In contrast, the polypeptide did not inhibit the growth of human breast cancer cells (MDA-MB-231 or MDA-MB-435) under similar experimental conditions. The VEGI-alpha deletion mutant was also found to have little effect on the proliferation of human T-cell leukemia cells (jurkat). human Burkitt's lymphoma cells (Raji), h um an monocytic leukemia cells (THP-1), or human promyelocytic leukemia cells (HL60). The mutant appears to bind to a distinct receptor, as no interaction was noted with any members of the TNF-receptor superfamily examined, including TNFR1, TNFR2, Fas, DR3, and DR4 (G.J. Mazzei et al. (19951 J. Biol. Chem. 270: 7205). It has been shown that several TNF family members, including TNF-α and the Fas ligand. are activated by metalloproteases from membrane-bound precursors (M. Tanaka et al.(1996] Nat. Med 2:317; R. A. Black et: al. [1997] Nature 385:729).

### Example 6: VEGI-Mediated Inhibition of Capillary-Like Tube Formation.

This assay was used to determine the relative ability of the truncated VEGI-alpha polypeptide to inhibit the FGF-2-induced formation of capillary-like tubular structures in cultures of adult bovine aortic endothelial (ABAE) cells. Three-dimensional collagen gel plates (24-well) were prepared by addition of 0.5 ml chilled solution of 0.7 mg/ml of rat tail type I collagen (Becton Dickinson Labwares, Bedford, MA) to each well containing 1 x DMEM and adjusting to neutral pH with NaHCO₃. After formation of collagen gel (about 1-2 mm thickness), ABAE cells were seeded at 5 x 10⁴ cells/well. The cultures were maintained in a humidified 5% CO₂ incubator at 37°C in DMEM containing 10% calf serum (HyClone, Logan, UT) supplemented with L-glutamine (2 mM) until the cultures reached confluence. The medium was then replaced with fresh medium containing 20 ng/ml of FGF-2. The effect of VEGI-alpha₁₂₋₁₄₇ as an inhibitor of FGF-2-induced formation of capillary-like tubular structures in ABAE cultures was analyzed by supplementing the culture medium with 0.1, 0.3, 1, 3, or 10 µg/ml of VEGI-alpha₁₂₋₁₄₇. All cultures were then maintained at 37°C for an additional 48 hours and then discontinued by fixation with cold methanol (-20°C).

The abundance of capillary-like structures formed by ABAE cells was analyzed by using a Kotron IBAS Image Analyzer assisted with a Hamamatsu C2400 video camera and a Zeiss Axioshop microscope. The abundance of the capillary-like structures were measured as percentages of the white areas over the total areas measured. As a control, the EC₅₀ value for the angiogenic factor FGF-2 to stimulate *in vitro* angiogenesis was about 5 ng/ml. As a further control, a maximum stimulatory effect was observed at 10 ng/ml of FGF-2.

### Example 7: VEGI-Mediated Inhibition of Tumor Growth.

An *in vivo* analysis of the potential effect of VEGI polypeptides on angiogenesis was performed using a xenograft tumor model. In this experimental approach, one million human breast carcinoma cells (MDA-MB-231 or MDA-MB-435) were injected into the mammary fat pad of female nude mice either alone or mixed with chinese hamster ovary (CHO) cells transfected with a VEGI-alpha expressing mammalian expression vector or CHO cells transfected only with the CHO-vector (5 x 10⁶ cells per mouse). The VEGI polypeptide expressed in these experiments consisted of the polypeptide shown as SEQ ID NO:2 excluding the N-terminal 22 amino acids. The N-terminal 22 amino acids of this VEGI mutein were replaced by the secretory signal peptide of human interleukin-6 (Hirano, T., et al., Nature 324:73-76 (1986)).

The anti-angiogenic activity of the VEGI polypeptide observed in this Example indicates that VEGI polypeptides may exhibit an inhibition of tumor growth. The potential anticancer activity of VEGI was examined using the xenograft tumor model described above, using breast cancer cells that are highly tumorigenic when implanted into the mammary fat pads of female athymic nude mice. Transcription of the construct in the transfectants was confirmed by Northern blotting analysis. The conditioned medium of the CHO cells overexpressing the secreted form of VEGI-alpha was concentrated and partially purified, and found to be able to inhibit ABAE cell growth, whereas that of the vector-transfected or the full-length VEGI transfected CHO cells did not have any effect (data not shown). The potential anticancer activity of VEGI-alpha was examined in a human breast cancer xenograft model. The soluble VEGI-alpha-transfected CHO cells and human breast cancer cells (MDA-MB-231 or MDA-MB-435) were co-injected into the mammary fat pads, and the growth of the xenograft tumors were monitored following injection.

CHO cells overexpressing the soluble VEGI-alpha mutein exerted a marked inhibitory effect on the growth of the human breast cancer xenograft tumors formed by MDA-MB-231 cells (Figure 4A), whereas the vector-transfected CHO cells showed no effect on tumor growth. Similarly, significant suppression of MDA-MB-435 tumor growth in nude mice was observed (Figure 4B). In either case, CHO cells overexpressing the full length VEGI-alpha had no effect on the growth of the xenograft tumors under similar experimental conditions. The anti-tumor effect of the soluble VEGI observed in the xenograft model may be ascribed to an anti-angiogenic effect on the endothelial cells of tumor vasculature.

Mice which were coinjected with human breast carcinoma cells and either VEGI-alpha-expressing CHO cells or vector-transfected CHO cells were then randomized and tumors were measured twice weekly. The tumor size was assessed by measuring perpendicular diameters with a caliper and calculated by multiplying the measurements of diameters in two dimensions. Tumors were measured beginning on day zero and approximately at 5 day intervals through approximately the twenty-eighth day. In each case, tumors which resulted from breast carcinoma cells coinjected with VEGI-alpha-expressing CHO cells remained significantly smaller in size than those which resulted from breast carcinoma cells coinjected with vector-only CHO cells.

Based on these findings, we suggest that VEGI is an endothelial cell-specific negative regulator of angiogenesis that may function in the maintenance of mature vasculature or the termination of an angiogenesis process. The endothelium under physiological conditions is a quiescent tissue, with about % of the endothelial cells undergoing proliferation (J. Denekamp [1990] Cancer Metas. Rev. 9:267). The mechanism underlying the maintenance of the quiescence of the endothelium is not yet clear. It is conceivable that a self-restriction mechanism is in place, in which the endothelial cells produce a growth inhibitor that acts upon the endothelial cells themselves through an autocrine pathway. VEGI may play a role in this important mechanism.

### Example 8: Ability of Recombinant VEGI-alpha to inhibit WEHI 164, ABAE, and L929 cell growth, and to induce cell adhesion in HL-60 cells.

The adherent target cells were prepared from confluent cultures by trypsinization in PBS, and non-adherent target cells were harvested from stationary cultures and washed once with medium. Target cells were suspended at 3 x 10⁵ cells/ml in medium containing 10% FCS. 0.1 ml aliquots were dispensed into 96-well flat-bottomed microtiter plates containing 0.1 ml serially diluted test samples of cells (WEHI 164 and L929). Incubation was continued for 70 hours. TNF-α, TNF-β and bacterially-produced VEGI-alpha were added at a 0.5 µg/ml concentration. The cytotoxicity and proliferation activity was quantified using an MTS assay performed by the addition of 20 µl of MTS and phenazine methosulfate (PMS) solution to each well. After a three hour incubation, the OD at 492 nm was measured by an ELISA plate reader. The OD₄₉₂ is proportional to the number of viable cells in the wells. The percent of cytotoxicity was calculated as follows: % cytotoxicity = (100 - ODₑₓₚₑᵣᵢₘₑₙₜₐₗ/OD_{control}) X 100. VEGI-alpha induced a morphology change which appeared as dark round cells (indicating killed cells).

A truncated form of the VEGI-alpha polypeptide consisting of amino acids 12-147 of the complete VEGI-alpha amino acid sequence shown as SEQ ID NO:2 (designated VEGI-alpha₁₂₋₁₄₇) was also used to examine the effect of VEGI-alpha on endothelial cell growth. Treatment of adult bovine aortic endothelial (ABAE) cells with VEGI-alpha₁₂₋₁₄₇ resulted in nearly complete inhibition of the growth of cells in the ABAE culture, but not of cells in the breast cancer cell lines MDA-MB-435 or MDA-MB-231. Nearly complete inhibition of the growth of the endothelial cells was achieved at 10 µg/ml VEGI-alpha₃₉₋₁₇₄, with a half-maximum inhibitory concentration value (IC₅₀) of approximately 1 µg/ml (approximately 70 nM).

To test adhesion ability of VEGI-alpha, HL-60 cells were used and cell adhesion and cell-cell contact were measured by observation under the microscope and scored subjectively by two independent investigators. In these experiments, VEGI-alpha appeared to induce cell adhesion. Cultures which were not treated with VEGI-alpha contained cells which had spread throughout the culture dish. However, cultures which were treated with VEGI-alpha, contained cells which were clearly aggregated together.

### Example 9: Expression via Gene Therapy

Fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask. At this time, fresh media is added (e.g., Ham's F12 media, supplemented with 10% FBS, penicillin, and streptomycin). The culture is then incubated at 37°C for approximately one week. At this time, fresh media is added and subsequently changed every 2-3 days. After an additional two weeks in culture, a monolayer of fibroblasts will have emerged. The monolayer is trypsinized and scaled into larger flasks.

pMV-7 (Kirschmeier, P.T. et al, DNA, 7:219-25 (1988)), which is flanked by the long terminal repeats of the Moloney murine sarcoma virus, is digested with *Eco* RI and *Hind* III, and, subsequently, treated with calf intestinal phosphatase. The linear vector is fractionated on agarose gel and purified using glass beads.

The cDNA encoding a polypeptide of the present invention is amplified using PCR primers which correspond to the 5' and 3' end sequences respectively. The 5' primer containing an *Eco* RI site and the 3' primer includes a *Hind* III site. Equal quantities of the Moloney murine sarcoma virus linear backbone and the amplified Eco RI and *Hind* III fragment are added together, in the presence of T4 DNA ligase. The resulting mixture is maintained under conditions appropriate for ligation of the two fragments. The ligation mixture is used to transform bacteria HB101, which are then plated onto agar-containing kanamycin for the purpose of confirming that the vector had the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells are transduced with the vector. The packaging cells now produce infectious viral particles containing the gene (the packaging cells are now referred to as producer cells).

Fresh media is added to the transduced producer cells, and, subsequently, the media is harvested from a 10 cm plate of confluent producer cells. The spent media, containing the infectious viral particles, is filtered through a millipore filter to remove detached producer cells. This media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it may be necessary to use a retroviral vector that has a selectable marker, such as *neo* or *his.*

The engineered fibroblasts are then injected into the host, either alone or after having been grown to confluence on cytodex 3 microcarrier beads. The fibroblasts now produce the protein product.

### Example 10: Chicken Embryonic Chorioallantoic Membrane (CAM) Angiogenesis Assay

The CAM assay was carried out essentially as described by Nguyen and colleagues (Microvasc. Res. 47:31-40 (1994)) and Iruela-Arispe and Dvorak (Thromb. Haemost. 78:672-677 (1997)). The method is based on the growth of new capillary vessels into a collagen gel pellet placed directly on the chorioallantoic membrane (CAM). The angiogenic factors FGF-2 (100 ng) or VEGF (250 ng) were embedded in collagen gel pellets and placed in contact with the CAM. Quantification of angiogenesis in the gels was carried out 24 hours after the placement of the gel pellets by using a Nikon fluorescence microscope. The images were transferred to a Power PC 100 AV, using a CCD Sony camera. Fluorescence intensity was evaluated with NH Image 1.61 software. Fluorescence intensity for the positive controls (which contained an angiogenic factor alone) was considered as the maximum angiogenic response, and set, arbitrarily, at 100. Due to the variability of the assay, inhibition greater than 20% was considered significant.

As an experimental determination of the effect of VEGI-alpha on the FGF-2- or VEGF-induced angiogenesis, bacterially-produced VEGI-alpha (250 ng) was mixed with either FGF-2 (100 ng) or VEGF (250 ng) and embedded in collagen gel pellets. The pellets were then placed in contact with the CAM as described above. VEGI-alpha markedly inhibited new capillary growth into collagen gels.

Numerous modifications and variations of the present invention are possible in light of the above teachings and, therefore, within the scope of the appended claims, the invention may be practiced otherwise than as particularly described.

## Claims

1. An isolated nucleic acid molecule comprising a polynucleotide selected from the group consisting of:
(a) a polynucleotide encoding a polypeptide comprising amino acids 1 to 174 of SEQ ID NO:2;
(b) a polynucleotide encoding a polypeptide comprising amino acids 23 to 174 of SEQ ID NO:2;
(c) a polynucleotide encoding a polypeptide comprising amino acids 39 to 174 of SEQ ID NO:2;
(d) a polynucleotide encoding a fragment of the polypeptide of (a) having angiogenesis inhibiting activity;
(e) a polynucleotide which hybridizes with the complement of SEQ ID NO: 1 wherein said polynucleotide encodes a polypeptide having angiogenesis inhibiting activity;
(f) a polynucleotide encoding an allelic form of the polynucleotide of (a); and
(g) the complementary strand of (a), (b), (c), (d), (e), or (f).

2. An isolated polypeptide comprising an amino acid sequence selected from the group consisting of:
(a) amino acids 1 to 174 of SEQ ID NO:2;
(b) amino acids 23 to 174 of SEQ ID NO:2;
(c) amino acids 39 to 174 of SEQ ID NO:2;
(d) a fragment of the amino acid sequence of (a) having angiogenesis inhibiting activity; and
(e) a polypeptide having angiogenesis inhibiting activity wherein said polypeptide comprises an amino acid sequence encoded by a polynucleotide which hybridizes with the complement of SEQ ID NO:1.

3. An isolated polypeptide according to claim 2, wherein said polypeptide comprises a secretion signal.

4. An angiogenesis inhibitor comprising a protease capable of releasing the polypeptide of claim 2 from a cell membrane.

5. A promoter of angiogenesis, said promoter comprising an antibody, drug or agent which reduces or eliminates VEGI function in a pharmaceutically acceptable diluent, in a pharmaceutically acceptable amount.

6. A promoter of angiogenesis according to claim 5, wherein said drug or agent is a ribozyme capable of digesting VEGI RNA.

7. A promoter of angiogenesis according to claim 5, wherein said drug or agent reduces or eliminates VEGI transcription or translation.

8. A promoter of angiogenesis according to claim 5, wherein said drug or agent is an antisense oligonucleotide.

9. A therapeutic method for inhibiting angiogenesis, comprising administering to a human or animal a composition comprising the nucleic acid molecule of claim 1 in a dosage sufficient to inhibit angiogenesis.

10. A therapeutic method for inhibiting angiogenesis according to claim 9, wherein the nucleic acid molecule comprises a VEGI polynucleotide operably associated with a regulatory sequence that controls gene expression.

11. A therapeutic method for promoting angiogenesis comprising administering to a human or animal the promoter of angiogenesis of claim 5 in a dosage sufficient to promote angiogenesis.

12. A method for the diagnosis of pathological angiogenesis comprising the steps of:
(i) contacting a sample from a human or animal suspected of having pathological angiogenesis with antibodies which recognize the polypeptide of claim 2; and
(ii) detecting the presence or absence of a complex formed between the polypeptide and the antibodies.

13. A diagnostic or prognostic kit comprising antibodies against VEGI polypeptide and ancillary reagents suitable for use in detecting the presence of VEGI polypeptide in a sample according to claim 12.

14. A method for the diagnosis of pathological angiogenesis comprising the steps of:
(i) contacting a sample from a human or animal suspected of having pathological angiogenesis with an oligonucleotide which binds the polynucleotide of claim 1; and
(ii) detecting the presence or absence of a duplex formed between the oligonucleotide and the polynucleotide.

15. A diagnostic or prognostic kit comprising an oligonucleotide complementary to VEGI RNA and ancillary reagents suitable for use in detecting the presence of VEGI polynucleotides in a sample according to claim 14.

16. A method for the diagnosis of pathological angiogenesis comprising the steps of detecting the presence of a polymorphism in VEGI polynucleotide in a sample from a human or animal suspected of having pathological angiogenesis.

17. A method for the diagnosis of pathological angiogenesis according to claim 16, wherein said detection is by Southern hybridization.

18. A method for the diagnosis of pathological angiogenesis according to claim 16, wherein said detection is by sequencing VEGI polynucleotide.

19. A therapeutic method for the treatment or amelioration of diseases and processes that are mediated by uncontrolled angiogenesis, comprising the step of administering to a human or animal in need of such treatment or amelioration the nucleic acid molecule of claim 1 in a pharmaceutically acceptable amount.

20. A therapeutic method for the treatment or amelioration of diseases and processes that are mediated by uncontrolled angiogenesis according to claim 19, wherein the nucleic acid molecule comprises a VEGI polynucleotide operably associated with a regulatory sequence that controls gene expression.

21. A therapeutic method for the treatment or amelioration of diseases and processes that are mediated by inhibited angiogenesis comprising the step of administering to a human or animal in need of such treatment or amelioration the promoter of angiogenesis of claim 5.

22. A therapeutic method for the treatment or amelioration of diseases and processes mediated by inhibited angiogenesis according to claim 21, wherein said agent is an antisense oligonucleotide.

23. A therapeutic method for the treatment or amelioration of diseases and processes mediated by inhibited angiogenesis according to claim 21, wherein said process is wound healing.

24. A therapeutic method for the treatment or amelioration of diseases and processes mediated by inhibited angiogenesis according to claim 21, wherein said agent is a ribozyme specific for VEGI RNA.

25. A method for detection or prognosis of cancer said method comprising:
(i) contacting a sample from a human or animal suspected of having cancer with an antibody which recognizes the polypeptide of claim 2; and
(ii) detecting the presence or absence of a complex formed between the polypeptide and the antibody.

26. An *in vitro* method for testing possible agents or drugs for angiogenic inhibitory activity said method comprising measuring the ability of said agent or drug to increase the anti-angiogenic activity of the polypeptide of claim 2 in an *in vitro* assay.

27. *An in vitro* method for testing agents or drugs for angiogenic inhibitory activity according to claim 26, wherein said drug or agent is an antitumour drug or agent.

28. An *in vitro* method for testing the ability of a drug or agent to promote angiogenesis said method comprising measuring ability of said drug or agent to reduce or eliminate function of the polypeptide of claim 2 in an *in vitro* assay.

29. A therapeutic method for inhibiting angiogenesis, comprising administering to a human or animal a composition comprising the polypeptide of claim 2 in a dosage sufficient to inhibit angiogenesis.

30. A therapeutic method for the treatment or amelioration of diseases and processes that are mediated by uncontrolled angiogenesis, comprising the step of administering to a human or animal in need of such treatment or amelioration the polypeptide of claim 2 in a pharmaceutically acceptable amount.

31. A therapeutic method for treating cancer comprising administering to a human or animal a composition comprising the nucleic acid molecule of claim 2 in a dosage sufficient to inhibit cancer.

32. A therapeutic method for treating cancer comprising administering to a human or animal a composition comprising the polypeptide of claim 2 in a dosage sufficient to inhibit cancer.

33. A therapeutic method according to claim 30 said disease and process is a member of the group consisting of:
(a) telangiectasia;
(b) psoriasis scleroderma;
(c) myocardial angiogenesis;
(d) plague neovascularization;
(e) ischemic limb angiogenesis;
(f) rubeosis;
(g) neovascular glaucoma;
(h) diabetic retinopathy;
(i) retrolental fibroplasia;
(j) diabetic neovascularization;
(k) neovascular glaucoma;
(l) retrolental fibroplasia;
(m) uveitis;
(n) retinopathy of prematurity;
(o) macular degeneration;
(p) corneal graft neovascularization;
(q) graft versus host disease;
(r) inflammatory bowel disease;
(s) myelosuppression; and
(t) restenosis.


SEQUENCE LISTING

<110> Human Genome Sciences, Inc.

<120> VEGI, An Inhibitor of Angiogenesis and Tumor Growth

<130> PF141PCT2

<140> Unassigned
<141> 1998-11-02

<150> 08/963,272
<151> 1997-11-03

<160> 31

<170> PatentIn Ver. 2.0

<210> 1
<211> 2683
<212> DNA
<213> Homo sapiens

<220>
<221> CDS
<222> (1022)..(1543)

<220>
<221> sig_peptide
<222> (1022)..(1096)

<220>
<221> mat_peptide
<222> (1097)..(1543)

<400> 1
   ttctctcctg tctctctctt tcattcatac actgagtcat tcagagatgg cttctctcca 60
   actcggagct gcaagtaatt ctggatctgg tcacacacac aaagtcccca gagttgccaa 120
   tttatctagt tcatctgtgc ctgttcaaga tgatgtaact aaacatttac cttcagggag 180
   gtgtttccaa agaattttca tcgatatata gaaatcaaga gaaaatccat actatcacca 240
   aatcaagaga aattccatac tatcaccagt tggccaactt tccaagtcta gtgcagaaat 300
   ccaaggcacc tcacacctag agttcctata cctctgagac tccagaggaa agaacaagac 360
   agtgcagaag gatatgttag aacccactga aaacctagaa ggttaaaaag gaagcatacc 420
   ctcctgacct ataagaaaat tttcagtctg cagggggata tccttgtggc ccaagacatt 480
   ggtgttatca tttgactaag aggaaattat ttgtggtgag ctctgagtga ggattaggac 540
   cagggagatg ccaagtttct atcacttacc tcatgcctgt aagacaagtg ttttgttcca 600
   attgatgaat ggggataaaa cagttcagcc aatcacttat ggggcaaaga atgggaattt 660
   gaagggtctg gtgcctggcc ttgtcatacg taaacaagag aggcatcgat gagttttatc 720
   tgagtcattt gggaaaggat aattcttgca gcaagccatt ttcctaaaca cagaagaata 780
   gggggattcc ttaaccttca ttgttctcca ggatcatagg tctcaggtaa aattaaaaat 840
   tttcaggtca gaccactcag tctcagaaag gcaaagtaat ttgccccagg tcactagtcc 900
   aagatgttat tctctttgaa caaatgtgta tgtccagtca catattcttc attcattcct 960
   ccccaaagca gtttttagct gttaggtata ttcgatcact ttagtctatt ttgaaaatga 1020
   t atg aga cgc ttt tta agc aaa gtc tac agt ttc cca atg aga aaa tta 1069
   Met Arg Arg Phe Leu Ser Lys Val Tyr Ser Phe Pro Met Arg Lys Leu
   -25 -20 -15 -10
   atc ctc ttt ctt gtc ttt cca gtt gtg aga caa act ccc aca cag cac 1117
   Ile Leu Phe Leu Val Phe Pro Val Val Arg Gln Thr Pro Thr Gln His
   -5 -1 1 5
   ttt aaa aat cag ttc cca gct ctg cac tgg gaa cat gaa cta ggc ctg 1165
   Phe Lys Asn Gln Phe Pro Ala Leu His Trp Glu His Glu Leu Gly Leu
   10 15 20
   gcc ttc acc aag aac cga atg aac tat acc aac aaa ttc ctg ctg atc 1213
   Ala Phe Thr Lys Asn Arg Met Asn Tyr Thr Asn Lys Phe Leu Leu Ile
   25 30 35
   cca gag tcg gga gac tac ttc att tac tcc cag gtc aca ttc cgt ggg 1261
   Pro Glu Ser Gly Asp Tyr Phe Ile Tyr Ser Gln Val Thr Phe Arg Gly
   40 45 50 55
   atg acc tct gag tgc agt gaa atc aga caa gca ggc cga cca aac aag 1309
   Met Thr Ser Glu Cys Ser Glu Ile Arg Gln Ala Gly Arg Pro Asn Lys
   60 65 70
   cca gac tcc atc act gtg gtc atc acc aag gta aca gac agc tac cct 1357
   Pro Asp Ser Ile Thr Val Val Ile Thr Lys Val Thr Asp Ser Tyr Pro
   75 80 85
   gag cca acc cag ctc ctc atg ggg acc aag tct gta tgc gaa gta ggt 1405
   Glu Pro Thr Gln Leu Leu Met Gly Thr Lys Ser Val Cys Glu Val Gly
   90 95 100
   agc aac tgg ttc cag ccc atc tac ctc gga gcc atg ttc tcc ttg caa 1453
   Ser Asn Trp Phe Gln Pro Ile Tyr Leu Gly Ala Met Phe Ser Leu Gln
   105 110 115
   gaa ggg gac aag cta atg gtg aac gtc agt gac atc tct ttg gtg gat 1501
   Glu Gly Asp Lys Leu Met Val Asn Val Ser Asp Ile Ser Leu Val Asp
   120 125 130 135
   tac aca aaa gaa gat aaa acc ttc ttt gga gcc ttc tta cta 1543
   Tyr Thr Lys Glu Asp Lys Thr Phe Phe Gly Ala Phe Leu Leu
   140 145
   taggaggaga gcaaatatca ttatatgaaa gtcctctgcc accgagttcc taattttctt 1603
   tgttcaaatg taattataac caggggtttt cttggggccg ggagtagggg gcattccaca 1663
   gggacaacgg tttagctatg aaatttgggg ccaaaatttc acacttcatg tgccttactg 1723
   atgagagtac taactggaaa aaggctgaag agagcaaata tattattaag atgggttgga 1783
   ggattggcga gtttctaaat attaagacac tgatcactaa atgaatggat gatctactcg 1843
   ggtcaggatt gaaagagaaa tatttcaaca cctcctgcta tacaatggtc accagtggtc 1903
   cagttattgt tcaatttgat cataaatttg cttcaattca ggagctttga aggaagtcca 1963
   aggaaagctc tagaaaacag tataaacttt cagaggcaaa atccttcacc aatttttcca 2023
   catactttca tgccttgcct aaaaaaaatg aaaagagagt tggtatgtct catgaatgtt 2083
   cacacagaag gagttggttt tcatgtcatc tacagcatat gagaaaagct acctttcttt 2143
   tgattatgta cacagatatc taaataagga agtatgagtt tcacatgtat atcaaaaata 2203
   caacagttgc ttgtattcag tagagttttc ttgcccacct attttgtgct gggttctacc 2263
   ttaacccaga agacactatg aaaaacaaga cagactccac tcaaaattta tatgaacacc 2323
   actagatact tcctgatcaa acatcagtca acatactcta aagaataact ccaagtcttg 2383
   gccaggcgca gtggctcaca cctgtaatcc caacactttg ggaggccaag gtgggtggat 2443
   catctaaggc cgggagttca agaccagcct gaccaacgtg gagaaacccc atctctacta 2503
   aaaatacaaa attagccggg cgtggtagcg catggctgta atcctggcta ctcaggaggc 2563
   cgaggcagaa gaattgcttg aactggggag gcagaggttg cggtgagccc agatcgcgcc 2623
   attgcactcc agcctgggta acaagagcaa aactctgtcc aaaaaaaaaa aaaaaaaaaa 2683

<210> 2
<211> 174
<212> PRT
<213> Homo sapiens

<400> 2
Met Arg Arg Phe Leu Ser Lys Val Tyr Ser Phe Pro Met Arg Lys Leu
-25                 -20                 -15                 -10

Ile Leu Phe Leu Val Phe Pro Val Val Arg Gln Thr Pro Thr Gln His
                -5                  -1  1               5

Phe Lys Asn Gln Phe Pro Ala Leu His Trp Glu His Glu Leu Gly Leu
        10                  15                  20

Ala Phe Thr Lys Asn Arg Met Asn Tyr Thr Asn Lys Phe Leu Leu Ile
    25                  30                  35

Pro Glu Ser Gly Asp Tyr Phe Ile Tyr Ser Gln Val Thr Phe Arg Gly
40                  45                  50                  55

Met Thr Ser Glu Cys Ser Glu Ile Arg Gln Ala Gly Arg Pro Asn Lys
                60                  65                  70

Pro Asp Ser Ile Thr Val Val Ile Thr Lys Val Thr Asp Ser Tyr Pro
            75                  80                  85

Glu Pro Thr Gln Leu Leu Met Gly Thr Lys Ser Val Cys Glu Val Gly
        90                  95                  100

Ser Asn Trp Phe Gln Pro Ile Tyr Leu Gly Ala Met Phe Ser Leu Gln
    105                 110                 115

Glu Gly Asp Lys Leu Met Val Asn Val Ser Asp Ile Ser Leu Val Asp
120                 125                 130                 135

Tyr Thr Lys Glu Asp Lys Thr Phe Phe Gly Ala Phe Leu Lèu
140 145

<210> 3
<211> 26
<212> DNA
<213> Homo sapiens

<400> 3
gcgccatggc tctgcactgg gaacat 26

<210> 4
<211> 27
<212> DNA
<213> Homo sapiens

<400> 4
cgcaagcttc tatagtaaga aggctcc 27

<210> 5
<211> 112
<212> DNA
<213> Homo sapiens

<400> 5
gcgggatccg ccaccatgaa ctccttctcc acaagcgcct tcggtccagt tgccttctcc 60

ctggggctgc tcctggtgtt gcctgctgcc ttccctgccc cagttgtgag ac 112

<210> 6
<211> 29
<212> DNA
<213> Homo sapiens

<400> 6
cgcggatccg atatttgctc tcctcctca 29

<210> 7
<211> 28
<212> PRT
<213> Homo sapiens

<400> 7
Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala
            20                  25

<210> 8
<211> 2442
<212> DNA
<213> Homo sapiens

<220>
<221> CDS
<222> (783)..(1304)

<220>
<221> sig_peptide
<222> (783)..(863)

<220>
<221> mat_peptide
<222> (864)..(1304)

<220>
<221> misc_feature
<222> (2265)
<223> n equals a, t, g or c

<220>
<221> misc_feature
<222> (2273)
<223> n equals a, t, g or c

<220>
<221> misc_feature
<222> (2307)
<223> n equals a, t, g or c

<220>
<221> misc_feature
<222> (2336)
<223> n equals a, t, g or c

<220>
<221> misc_feature
<222> (2341)
<223> n equals a, t, g or c

<220>
<221> misc_feature
<222> (2379)
<223> n equals a, t, g or c

<400> 8
   cccaatcaag agaaattcca tactatcacc agttggccga ctttccaagt ctagtgcaga 60
   aatccaaggc acctcacacc tagagttcct atacctctga gactccagag gaaagaacaa 120
   gacagtgcag aaggatatgt tagaacccac tgaaaaccta gaaggttgaa aaggaagcat 180
   accctcctga cctataagaa aattttcagt ctgcaggggg atatccttgt ggcccaagac 240
   attggtgtta tcatttgact aagaggaaat tatttgtggt gagctctgag tgaggattag 300
   gaccagggag atgccaagtt tctatcactt acctcatgcc tgtaagacaa gtgttttgtt 360
   ccaattgatg aatggggaga aaacagttca gccaatcact tatgggcaca gaatggaatt 420
   tgaagggtct ggtgcctgcc cttgtcatac gtaaacaaga gaggcatcga tgagttttat 480
   ctgagtcatt tgggaaagga taattcttgc accaagccat tttcctaaac acagaagaat 540
   agggggattc cttaaccttc attgttctcc aggatcatag gtctcaggat aaattaaaaa 600
   ttttcaggtc agaccactca gtctcagaaa ggcaaagtaa tttgccccag gtcactagtc 660
   caagatgtta ttctctttga acaaatgtgt atgtccagtc acatattctt cattcattcc 720
   tccccaaagc agtttttagc tgttaggtat attcgatcac tttagtctat tttgaaaatg 780
   at atg aga cgc ttt tta agc aaa gtc tac agt ttc cca atg aga aaa 827
   Met Arg Arg Phe Leu Ser Lys Val Tyr Ser Phe Pro Met Arg Lys
   -25 -20 -15
   tta atc ctc ttt ctt gtc ttt cca gtt gtg aga caa act ccc aca cag 875
   Leu Ile Leu Phe Leu Val Phe Pro Val Val Arg Gln Thr Pro Thr Gln
   -10 -5 -1 1
   cac ttt aaa aat cag ttc cca gct ctg cac tgg gaa cat gaa cta ggc 923
   His Phe Lys Asn Gln Phe Pro Ala Leu His Trp Glu His Glu Leu Gly
   5 10 15 20
   ctg gcc ttc acc aag aac cga atg aac tat acc aac aaa ttc ctg ctg 971
   Leu Ala Phe Thr Lys Asn Arg Met Asn Tyr Thr Asn Lys Phe Leu Leu
   25 30 35
   atc cca gag tcg gga gac tac ttc att tac tcc cag gtc aca ttc cgt 1019
   Ile Pro Glu Ser Gly Asp Tyr Phe Ile Tyr Ser Gln Val Thr Phe Arg
   40 45 50
   ggg atg acc tct gag tgc agt gaa atc aga caa gca ggc cga cca aac 1067
   Gly Met Thr Ser Glu Cys Ser Glu Ile Arg Gln Ala Gly Arg Pro Asn
   55 60 65
   aag cca gac tcc atc act gtg gtc atc acc aag gta aca gac agc tac 1115
   Lys Pro Asp Ser Ile Thr Val Val Ile Thr Lys Val Thr Asp Ser Tyr
   70 75 80
   cct gag cca acc cag ctc ctc atg ggg acc aag tct gta tgc gaa gta 1163
   Pro Glu Pro Thr Gln Leu Leu Met Gly Thr Lys Ser Val Cys Glu Val
   85 90 95 100
   ggt agc aac tgg ttc cag ccc atc tac ctc gga gcc atg ttc tcc ttg 1211
   Gly Ser Asn Trp Phe Gln Pro Ile Tyr Leu Gly Ala Met Phe Ser Leu
   105 110 115
   caa gaa ggg gac aag cta atg gtg aac gtc agt gac atc tct ttg gtg 1259
   Gln Glu Gly Asp Lys Leu Met Val Asn Val Ser Asp Ile Ser Leu Val
   120 125 130
   gat tac aca aaa gaa gat aaa acc ttc ttt gga gcc ttc tta cta 1304
   Asp Tyr Thr Lys Glu Asp Lys Thr Phe Phe Gly Ala Phe Leu Leu
   135 140 145
   taggaggaga gcaaatatca ttatatgaaa gtcctctgcc accgagttcc taattttctt 1364
   tgttcaaatg taattataac caggggtttt cttggggccg ggagtagggg gcattccaca 1424
   gggacaacgg tttagctatg aaatttgggg ccaaaatttc acacttcatg tgccttactg 1484
   atgagagtac taactggaaa aaggctgaag agagcaaata tattattaag atgggttgga 1544
   ggattggcga gtttctaaat attaagacac tgatcactaa atgaatggat gatctactcg 1604
   ggtcaggatt gaaagagaaa tatttcaaca cctccctgct atacaatggt caccagtggt 1664
   ccagttattg ttcaatttga tcataaattt gcttcaattc aggagctttg aaggaagtcc 1724
   aaggaaagct ctagaaaaca gtataaactt tcagaggcaa aatccttcac caatttttcc 1784
   acatactttc atgccttgcc taaaaaaaat gaaaagagag ttggtatgtc tcatgaatgt 1844
   tcacacagaa ggagttggtt ttcatgtcat ctacagcata tgagaaaagc tacctttctt 1904
   ttgattatgt acacagatat ctaaataagg aagtttgagt ttcacatgta tatcccaaat 1964
   acaacagttg cttgtattca gtagagtttt cttgcccacc tattttgtgc tgggttctac 2024
   cttaacccag aagacactat gaaaaacaag acagactcca ctcaaaattt atatgaacac 2084
   cactagatac ttcctgatca aacatcagtc aacatactct aaagaataac tccaagtctt 2144
   ggccaggcgc agtggctcac acctgtaatc ccaacacttt gggaggccaa ggtgggtgga 2204
   tcatctaagg ccgggagttc aagaccagcc tgaccaacgt ggagaaaccc catctctact 2264
   naaaatacna aattagccgg gcgtggtagc gcatggctgt aancctggct actcaggagg 2324
   ccgaggcaga anaattnctt gaactgggga ggcagaggtt gcggtgagcc cagancgcgc 2384
   cattgcactc cagcctgggt aacaagagca aaactctgtc caaaaaaaaa aaaaaaaa 2442

<210> 9
<211> 174
<212> PRT
<213> Homo sapiens

<400> 9
Met Arg Arg Phe Leu Ser Lys Val Tyr Ser Phe Pro Met Arg Lys Leu
-25 -20 -15

Ile Leu Phe Leu Val Phe Pro Val Val Arg Gln Thr Pro Thr Gln His
-10 -5 -1 1 5

Phe Lys Asn Gln Phe Pro Ala Leu His Trp Glu His Glu Leu Gly Leu
10 15 20

Ala Phe Thr Lys Asn Arg Met Asn Tyr Thr Asn Lys Phe Leu Leu Ile
25 30 35

Pro Glu Ser Gly Asp Tyr Phe Ile Tyr Ser Gln Val Thr Phe Arg Gly
40 45 50

Met Thr Ser Glu Cys Ser Glu Ile Arg Gln Ala Gly Arg Pro Asn Lys
55 60 65

Pro Asp Ser Ile Thr Val Val Ile Thr Lys Val Thr Asp Ser Tyr Pro
70 75 80 85

Glu Pro Thr Gln Leu Leu Met Gly Thr Lys Ser Val Cys Glu Val Gly
90 95 100

Ser Asn Trp Phe Gln Pro Ile Tyr Leu Gly Ala Met Phe Ser Leu Gln
105 110 115

Glu Gly Asp Lys Leu Met Val Asn Val Ser Asp Ile Ser Leu Val Asp
120 125 130

Tyr Thr Lys Glu Asp Lys Thr Phe Phe Gly Ala Phe Leu Leu
135 140 145

<210> 10
<211> 233
<212> PRT
<213> Homo sapiens

<400> 10
Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230


<210> 11
<211> 205
<212> PRT
<213> Homo sapiens

<400> 11
Met Thr Pro Pro Glu Arg Leu Phe Leu Pro Arg Val Arg Gly Thr Thr
1               5                   10                  15

Leu His Leu Leu Leu Leu Gly Leu Leu Leu Val Leu Leu Pro Gly Ala
            20                  25                  30

Gln Gly Leu Pro Gly Val Gly Leu Thr Pro Ser Ala Ala Gln Thr Ala
        35                  40                  45

Arg Gln His Pro Lys Met His Leu Ala His Ser Thr Leu Lys Pro Ala
    50                  55                  60

Ala His Leu Ile Gly Asp Pro Ser Lys Gln Asn Ser Leu Leu Trp Arg
65                  70                  75                  80

Ala Asn Thr Asp Arg Ala Phe Leu Gln Asp Gly Phe Ser Leu Ser Asn
                85                  90                  95

Asn Ser Leu Leu Val Pro Thr Ser Gly Ile Tyr Phe Val Tyr Ser Gln
            100                 105                 110

Val Val Phe Ser Gly Lys Ala Tyr Ser Pro Lys Ala Thr Ser Ser Pro
        115                 120                 125

Leu Tyr Leu Ala His Glu Val Gln Leu Phe Ser Ser Gln Tyr Pro Phe
    130                 135                 140

His Val Pro Leu Leu Ser Ser Gln Lys Met Val Tyr Pro Gly Leu Gln
145                 150                 155                 160

Glu Pro Trp Leu His Ser Met Tyr His Gly Ala Ala Phe Gln Leu Thr
                165                 170                 175

Gln Gly Asp Gln Leu Ser Thr His Thr Asp Gly Ile Pro His Leu Val
            180                 185                 190

Leu Ser Pro Ser Thr Val Phe Phe Gly Ala Phe Ala Leu
        195                 200                 205


<210> 12
<211> 244
<212> PRT
<213> Homo sapiens

<400> 12
Met Gly Ala Leu Gly Leu Glu Gly Arg Gly Gly Arg Leu Gln Gly Arg
1               5                   10                  15

Gly Ser Leu Leu Leu Ala Val Ala Gly Ala Thr Ser Leu Val Thr Leu
            20                  25                  30

Leu Leu Ala Val Pro Ile Thr Val Leu Ala Val Leu Ala Leu Val Pro
        35                  40                  45

Gln Asp Gln Gly Gly Leu Val Thr Glu Thr Ala Asp Pro Gly Ala Gln
    50                  55                  60

Ala Gln Gln Gly Leu Gly Phe Gln Lys Leu Pro Glu Glu Glu Pro Glu
65                  70                  75                  80

Thr Asp Leu Ser Pro Gly Leu Pro Ala Ala His Leu Ile Gly Ala Pro
                85                  90                  95

Leu Lys Gly Gln Gly Leu Gly Trp Glu Thr Thr Lys Glu Gln Ala Phe
            100                 105                 110

Leu Thr Ser Gly Thr Gln Phe Ser Asp Ala Glu Gly Leu Ala Leu Pro

115                 120                 125

Gln Asp Gly Leu Tyr Tyr Leu Tyr Cys Leu Val Gly Tyr Arg Gly Arg
    130                 135                 140

Ala Pro Pro Gly Gly Gly Asp Pro Gln Gly Arg Ser Val Thr Leu Arg
145                 150                 155                 160

Ser Ser Leu Tyr Arg Ala Gly Gly Ala Tyr Gly Pro Gly Thr Pro Glu
                165                 170                 175

Leu Leu Leu Glu Gly Ala Glu Thr Val Thr Pro Val Leu Asp Pro Ala
            180                 185                 190

Arg Arg Gln Gly Tyr Gly Pro Leu Trp Tyr Thr Ser Val Gly Phe Gly
        195                 200                 205

Gly Leu Val Gln Leu Arg Arg Gly Glu Arg Val Tyr Val Asn Ile Ser
    210                 215                 220

His Pro Asp Met Val Asp Phe Ala Arg Gly Lys Thr Phe Phe Gly Ala
225                 230                 235                 240

Val Met Val Gly

<210> 13
<211> 281
<212> PRT
<213> Homo sapiens

<400> 13
Met Gln Gln Pro Phe Asn Tyr Pro Tyr Pro Gln Ile Tyr Trp Val Asp
1               5                   10                  15

Ser Ser Ala Ser Ser Pro Trp Ala Pro Pro Gly Thr Val Leu Pro Cys
            20                  25                  30

Pro Thr Ser Val Pro Arg Arg Pro Gly Gln Arg Arg Pro Pro Pro Pro
        35                  40                  45

Pro Pro Pro Pro Pro Leu Pro Pro Pro Pro Pro Pro Pro Pro Leu Pro
    50                  55                  60

Pro Leu Pro Leu Pro Pro Leu Lys Lys Arg Gly Asn His Ser Thr Gly
65                  70                  75                  80

Leu Cys Leu Leu Val Met Phe Phe Met Val Leu Val Ala Leu Val Gly
                85                  90                  95

Leu Gly Leu Gly Met Phe Gln Leu Phe His Leu Gln Lys Glu Leu Ala
            100                 105                 110

Glu Leu Arg Glu Ser Thr Ser Gln Met His Thr Ala Ser Ser Leu Glu
        115                 120                 125

Lys Gln Ile Gly His Pro Ser Pro Pro Pro Glu Lys Lys Glu Leu Arg
    130                 135                 140

Lys Val Ala His Leu Thr Gly Lys Ser Asn Ser Arg Ser Met Pro Leu
145                 150                 155                 160

Glu Trp Glu Asp Thr Tyr Gly Ile Val Leu Leu Ser Gly Val Lys Tyr
                165                 170                 175

Lys Lys Gly Gly Leu Val Ile Asn Glu Thr Gly Leu Tyr Phe Val Tyr
            180                 185                 190

Ser Lys Val Tyr Phe Arg Gly Gln Ser Cys Asn Asn Leu Pro Leu Ser
        195                 200                 205

His Lys Val Tyr Met Arg Asn Ser Lys Tyr Pro Gln Asp Leu Val Met
    210                 215                 220

Met Glu Gly Lys Met Met Ser Tyr Cys Thr Thr Gly Gln Met Trp Ala
225                 230                 235                 240

Arg Ser Ser Tyr Leu Gly Ala Val Phe Asn Leu Thr Ser Ala Asp His
                245                 250                 255

Leu Tyr Val Asn Val Ser Glu Leu Ser Leu Val Asn Phe Glu Glu Ser
            260                 265                 270

Gln Thr Phe Phe Gly Leu Tyr Lys Leu
        275                 280


<210> 14
<211> 235
<212> PRT
<213> Oryctolagus cuniculus

<400> 14
Met Ser Thr Glu Ser Met Ile Arg Asp Val Glu Leu Ala Glu Gly Pro
  1               5                  10                  15

Leu Pro Lys Lys Ala Gly Gly Pro Gln Gly Ser Lys Arg Cys Leu Cys
             20                  25                  30

Leu Ser Leu Phe Ser Phe Leu Leu Val Ala Gly Ala Thr Thr Leu Phe
         35                  40                  45

Cys Leu Leu His Phe Arg Val Ile Gly Pro Gln Glu Glu Glu Gln Ser
     50                  55                  60

Pro Asn Asn Leu His Leu Val Asn Pro Val Ala Gln Met Val Thr Leu
 65                  70                  75                  80

Arg Ser Ala Ser Arg Ala Leu Ser Asp Lys Pro Leu Ala His Val Val
                 85                  90                  95

Ala Asn Pro Gln Val Glu Gly Gln Leu Gln Trp Leu Ser Gln Arg Ala
            100                 105                 110

Asn Ala Leu Leu Ala Asn Gly Met Lys Leu Thr Asp Asn Gln Leu Val
        115                 120                 125

Val Pro Ala Asp Gly Leu Tyr Leu Ile Tyr Ser Gln Val Leu Phe Ser
    130                 135                 140

Gly Gln Gly Cys Arg Ser Tyr Val Leu Leu Thr His Thr Val Ser Arg
145                 150                 155                 160

Phe Ala Val Ser Tyr Pro Asn Lys Val Asn Leu Leu Ser Ala Ile Lys
                165                 170                 175

Ser Pro Cys His Arg Glu Thr Pro Glu Glu Ala Glu Pro Met Ala Trp
            180                 185                 190

Tyr Glu Pro Ile Tyr Leu Gly Gly Val Phe Gln Leu Glu Lys Gly Asp
        195                 200                 205

Arg Leu Ser Thr Glu Val Asn Gln Pro Glu Tyr Leu Asp Leu Ala Glu
    210                 215                 220

Ser Gly Gln Val Tyr Phe Gly Ile Ile Ala Leu
225                 230                 235

<210> 15
<211> 434
<212> DNA
<213> Homo sapiens

<220>
<221> misc_feature
<222> (15)
<223> n equals a, t, g or c

<220>
<221> misc_feature
<222> (19)
<223> n equals a, t, g or c

<220>
<221> misc_feature
<222> (133)
<223> n equals a, t, g or c

<220>
<221> misc_feature
<222> (388)
<223> n equals a, t, g or c

<220>
<221> misc_feature
<222> (424)
<223> n equals a, t, g or c

<400> 15
tctacacaag gtacngacng ctaccctgag ccaacccagc tcctcatggg gaccaagtct 60

gtatgcgaag taggtagcaa ctggttccag cccatctacc tcggagccat gttctccttg 120

caagaagggg acnagctaat ggtgaacgtc agtgacatct ctttggtgga ttacacaaaa 180

gaagataaaa ccttctttgg agccttctta ctataggagg agagcaaata tcattatatg 240

aaagtcctct gccaccgagt tcctaatttt ctttgttcaa atgtaattat aaccaggggt 300

tttcttgggg ccgggagtag ggggcattcc cacagggaca acggtttagc tatgaaattt 360

gggggcccca aaatttcaca acttcatngt tgcccttact tgatgagaag tacttaactt 420

gganaaaagg cttg                                                   434

<210> 16

<211> 493
<212> DNA
<213> Homo sapiens

<220>
<221> misc_feature
<222> (288)
<223> n equals a, t, g or c

<220>
<221> misc_feature
<222> (296)
<223> n equals a, t, g or c

<220>
<221> misc_feature
<222> (309)
<223> n equals a, t, g or c

<220>
<221> misc_feature
<222> (314)
<223> n equals a, t, g or c

<220>
<221> misc_feature
<222> (340)
<223> n equals a, t, g or c

<220>
<221> misc_feature
<222> (343)
<223> n equals a, t, g or c

<220>
<221> misc_feature
<222> (348)
<223> n equals a, t, g or c

<220>
<221> misc_feature
<222> (369)
<223> n equals a, t, g or c

<220>
<221> misc_feature
<222> (385)
<223> n equals a, t, g or c

<220>
<221> misc_feature
<222> (410)
<223> n equals a, t, g or c

<220>
<221> misc_feature
<222> (417)
<223> n equals a, t, g or c

<220>
<221> misc_feature
<222> (423)
<223> n equals a, t, g or c

<220>
<221> misc_feature
<222> (431)
<223> n equals a, t, g or c

<220>
<221> misc_feature
<222> (434)
<223> n equals a, t, g or c

<220>
<221> misc_feature
<222> (437)
<223> n equals a, t, g or c

<220>
<221> misc_feature
<222> (444)
<223> n equals a, t, g or c

<220>
<221> misc_feature
<222> (459)
<223> n equals a, t, g or c

<220>
<221> misc_feature
<222> (486)
<223> n equals a, t, g or c

<400> 16
aattcggcag agaaattcca tactatcacc agttggccaa ctttccaagt ctagtgcaga 60

aatccaaggc acctcacacc tagagttcct atacctctga gactccagag gaaagaacaa 120

gacagtgcag aaggatatgt tagaacccac tgaaaaccta gaaggttaaa aaggaagcat 180

accctcctga cctataagaa aattttcagt ctgcaggggg atatccttgt ggcccaagac 240

attggtgtta tcatttgact aagaggaaat tatttgtggt gagctccnag tgaggnttag 300

ggaccaggng gtgnccaagt ttctatcact tacctcatgn ctntaagnca agtgttttgt 360

tcccattgnt gatggggtta aaacnttcag ccatcacttt tggggcaagn atggggnttt 420

gangggttgg ngcnggnctt gtcntcgtaa acagggggnt tggtgggttt ttctgggtcc 480

ttgggnagga ctt 493

<210> 17
<211> 380
<212> DNA
<213> Homo sapiens

<220>
<221> misc_feature
<222> (53)
<223> n equals a, t, g or c

<220>
<221> misc_feature

<222> (258)
<223> n equals a, t, g or c

<220>
<221> misc_feature
<222> (316)
<223> n equals a, t, g or c

<220>
<221> misc_feature
<222> (324)
<223> n equals a, t, g or c

<220>
<221> misc_feature
<222> (346)
<223> n equals a, t, g or c

<220>
<221> misc_feature
<222> (367)
<223> n equals a, t, g or c

<220>
<221> misc_feature
<222> (378)
<223> n equals a, t, g or c

<400> 17
ggcagaggtt caatttgatc ataaatttgc ttcaattcag gagctttgaa ggnngtccaa 60

ggaaagctct agaaaacagt ataaactttc agaggcaaaa tccttcacca atttttccac 120

atactttcat gccttgccta aaaaaaatga aaagagagtt ggtatgtctc atggaatgtt 180

cacacagaag gagttggttt tcatgtcatc tacagcatat gagaaaagct acctttcttt 240

tgattatgta cacaggtntc taaataagga agtatgagtt tcacatgtat attcaaaaat 300

acaacagttg cttgtnttca gttngggttt ttcttggccc acccantttt ggtgctgggg 360

gttctanctt taaccccnga                                              380


<210> 18
<211> 458
<212> DNA
<213> Homo sapiens

<220>
<221> misc_feature
<222> (9)
<223> n equals a, t, g or c

<220>
<221> misc_feature
<222> (12)
<223> n equals a, t, g or c

<220>
<221> misc_feature
<222> (119)
<223> n equals a, t, g or c

<220>
<221> misc_feature
<222> (303)
<223> n equals a, t, g or c

<220>
<221> misc_feature
<222> (311)
<223> n equals a, t, g or c

<220>
<221> misc_feature
<222> (387)
<223> n equals a, t, g or c

<220>
<221> misc_feature
<222> (409)
<223> n equals a, t, g or c

<220>
<221> misc_feature
<222> (425)
<223> n equals a, t, g or c

<220>
<221> misc_feature
<222> (427)
<223> n equals a, t, g or c

<220>
<221> misc_feature
<222> (453)
<223> n equals a, t, g or c

<400> 18
ggcacagcng gnagtagggg gcattccaca gggacaacgg tttagctatg aaatttgggg 60

cccaaaattt cacacttcat gtgccttact gatgagagta ctaactggaa aaaggctgna 120

agagagcaaa tatattatta agatgggttg gaggattggc gagtttctaa atattaagac 180

actggatcac tgaaatgaat ggatgatcta ctcgggtcca ggattgaaag agaaatattt 240

caacaccttc ctgctataca atggtcacca gtggtccagt tattgttcca atttggatcc 300

atnaatttgc nttcaattcc aggagctttg gaaggaattc caaggaaagc tccaggaaaa 360

ccgtattaaa ctttccaggg gccaaantcc ttcaccaatt ttttccacna actttccagg 420

cctgncncaa aaaaatggaa agggagttgg tangtccc 458


<210> 19
<211> 388
<212> DNA
<213> Homo sapiens

<220>
<221> misc_feature
<222> (11)
<223> n equals a, t, g or c

<220>
<221> misc_feature
<222> (46)
<223> n equals a, t, g or c

<220>
<221> misc_feature
<222> (50)
<223> n equals a, t, g or c

<220>
<221> misc_feature
<222> (81)
<223> n equals a, t, g or c

<220>
<221> misc_feature
<222> (138)
<223> n equals a, t, g or c

<220>
<221> misc_feature
<222> (155)
<223> n equals a, t, g or c

<220>
<221> misc_feature
<222> (182)
<223> n equals a, t, g or c

<220>
<221> misc_feature
<222> (188)
<223> n equals a, t, g or c

<220>
<221> misc_feature
<222> (269)
<223> n equals a, t, g or c

<220>
<221> misc_feature
<222> (317)
<223> n equals a, t, g or c

<220>
<221> misc_feature
<222> (322)
<223> n equals a, t, g or c

<220>
<221> misc_feature
<222> (358)
<223> n equals a, t, g or c

<220>
<221> misc_feature
<222> (363)
<223> n equals a, t, g or c

<220>
<221> misc_feature

<222> (375)
<223> n equals a, t, g or c

<400> 19
   ctgcactggg nncatgaact aggcctggcc ttcaccaaga accgantgan ctataccaac 60
   aaattcctgc tgatcccaga ntcgggagac tacttcattt actcccaggt cacattccgt 120
   gggaatgaac ctctgaantg ccagtgaaaa tcagncaagc aggccgacca aacaagccag 180
   antccatnca ctgtggtcat caccaaggta acagacagct accctgagcc aacccagctc 240
   cttcatgggg accaagtttg tttgcgaant aggttagcaa ctggttccag cccattttac 300
   cttgggggcc agttctnctt gncaagaagg ggacaagctt atggtggaac gttcatanca 360
   tcnttttggg gtggntttac acaaaagg 388

<210> 20
<211> 37
<212> DNA
<213> Homo sapiens

<400> 20
gcgcggatcc accatgagac gctttttaag caaagtc 37

<210> 21
<211> 36
<212> DNA
<213> Homo sapiens

<400> 21
cgcgtctaga ctatagtaag aaggctccaa agaagg 36

<210> 22
<211> 37
<212> DNA
<213> Homo sapiens

<400> 22
gcgcggatcc accatgagac gctttttaag caaagtc 37

<210> 23
<211> 36
<212> DNA
<213> Homo sapiens

<400> 23
cgcgtctaga ctatagtaag aaggctccaa agaagg 36

<210> 24
<211> 56
<212> DNA
<213> Homo sapiens

<400> 24
cgctctagat caagcgtagt ctgggacgtc gtatggatag taagaaggct ccaaag 56

<210> 25
<211> 733
<212> DNA
<213> Homo sapiens

<400> 25
gggatccgga gcccaaatct tctgacaaaa ctcacacatg cccaccgtgc ccagcacctg 60

aattcgaggg tgcaccgtca gtcttcctct tccccccaaa acccaaggac accctcatga 120

tctcccggac tcctgaggtc acatgcgtgg tggtggacgt aagccacgaa gaccctgagg 180

tcaagttcaa ctggtacgtg gacggcgtgg aggtgcataa tgccaagaca aagccgcggg 240

aggagcagta caacagcacg taccgtgtgg tcagcgtcct caccgtcctg caccaggact 300

ggctgaatgg caaggagtac aagtgcaagg tctccaacaa agccctccca acccccatcg 360

agaaaaccat ctccaaagcc aaagggcagc cccgagaacc acaggtgtac accctgcccc 420

catcccggga tgagctgacc aagaaccagg tcagcctgac ctgcctggtc aaaggcttct 480

atccaagcga catcgccgtg gagtgggaga gcaatgggca gccggagaac aactacaaga 540

ccacgcctcc cgtgctggac tccgacggct ccttcttcct ctacagcaag ctcaccgtgg 600

acaagagcag gtggcagcag gggaacgtct tctcatgctc cgtgatgcat gaggctctgc 660

acaaccacta cacgcagaag agcctctccc tgtctccggg taaatgagtg cgacggccgc 720

gactctagag gat 733


<210> 26
<211> 1116
<212> DNA
<213> Homo sapiens

<220>
<221> CDS
<222> (1)..(756)

<400> 26
atg gcc gag gat ctg gga ctg agc ttt ggg gaa aca gcc agt gtg gaa 48
Met Ala Glu Asp Leu Gly Leu Ser Phe Gly Glu Thr Ala Ser Val Glu
1               5                   10                  15

atg ctg cca gag cac ggc agc tgc agg ccc aag gcc agg agc agc agc 96
Met Leu Pro Glu His Gly Ser Cys Arg Pro Lys Ala Arg Ser Ser Ser
            20                  25                  30

gca cgc tgg gct ctc acc tgc tgc ctg gtg ttg ctc ccc ttc ctt gca 144
Ala Arg Trp Ala Leu Thr Cys Cys Leu Val Leu Leu Pro Phe Leu Ala
        35                  40                  45

gga ctc acc aca tac ctg ctt gtc agc cag ctc cgg gcc cag gga gag 192
Gly Leu Thr Thr Tyr Leu Leu Val Ser Gln Leu Arg Ala Gln Gly Glu
    50                  55                  60

gcc tgt gtg cag ttc cag gct cta aaa gga cag gag ttt gca cct tca 240
Ala Cys Val Gln Phe Gln Ala Leu Lys Gly Gln Glu Phe Ala Pro Ser
65                  70                  75                  80

cat cag caa gtt tat gca cct ctt aga gca gac gga gat aag cca agg    288
His Gln Gln Val Tyr Ala Pro Leu Arg Ala Asp Gly Asp Lys Pro Arg
                85                  90                  95

gca cac ctg aca gtt gtg aga caa act ccc aca cag cac ttt aaa aat    336
Ala His Leu Thr Val Val Arg Gln Thr Pro Thr Gln His Phe Lys Asn
            100                 105                 110

cag ttc cca gct ctg cac tgg gaa cat gaa cta ggc ctg gcc ttc acc    384
Gln Phe Pro Ala Leu His Trp Glu His Glu Leu Gly Leu Ala Phe Thr
        115                 120                 125

aag aac cga atg aac tat acc aac aaa ttc ctg ctg atc cca gag tcg    432
Lys Asn Arg Met Asn Tyr Thr Asn Lys Phe Leu Leu Ile Pro Glu Ser
    130                 135                 140

gga gac tac ttc att tac tcc cag gtc aca ttc cgt ggg atg acc tct    480
Gly Asp Tyr Phe Ile Tyr Ser Gln Val Thr Phe Arg Gly Met Thr Ser
145                 150                 155                 160

gag tgc agt gaa atc aga caa gca ggc cga cca aac aag cca gac tcc    528
Glu Cys Ser Glu Ile Arg Gln Ala Gly Arg Pro Asn Lys Pro Asp Ser
                165                 170                 175

atc act gtg gtc atc acc aag gta aca gac agc tac cct gag cca acc    576
Ile Thr Val Val Ile Thr Lys Val Thr Asp Ser Tyr Pro Glu Pro Thr
            180                 185                 190

cag ctc ctc atg ggg acc aag tct gta tgc gaa gta ggt agc aac tgg    624
Gln Leu Leu Met Gly Thr Lys Ser Val Cys Glu Val Gly Ser Asn Trp
        195                 200                 205

ttc cag ccc atc tac ctc gga gcc atg ttc tcc ttg caa gaa ggg gac    672
Phe Gln Pro Ile Tyr Leu Gly Ala Met Phe Ser Leu Gln Glu Gly Asp
    210                 215                 220

aag cta atg gtg aac gtc agt gac atc tct ttg gtg gat tac aca aaa    720
Lys Leu Met Val Asn Val Ser Asp Ile Ser Leu Val Asp Tyr Thr Lys
225                 230                 235                 240

gaa gat aaa acc ttc ttt gga gcc ttc tta cta tag gaggagagca         766
Glu Asp Lys Thr Phe Phe Gly Ala Phe Leu Leu
                245                 250

aatatcatta tatgaaagtc ctctgccacc gagttcctaa ttttctttgt tcaaatgtaa 826

ttataaccag gggttttctt ggggccggga gtaggggcat tccacaggga caacggttta 886

gctatgaaat ttggggccca aaatttcaca cttcatgtgc cttactgatg agagtactaa 946

ctggaaaaag gctgaagaga gcaaatatat tattaagatg ggttggagga ttggcgagtt 1006

tctaaatatt aagacactga tcactaaatg aatggatgat ctactcgggt caggattgaa 1066

agagaaatat ttcaacacct tcctgctata caatggtcac cagtggtcca            1116

<210> 27
<211> 251
<212> PRT
<213> Homo sapiens

<400> 27
Met Ala Glu Asp Leu Gly Leu Ser Phe Gly Glu Thr Ala Ser Val Glu
1               5                   10                  15

Met Leu Pro Glu His Gly Ser Cys Arg Pro Lys Ala Arg Ser Ser Ser
            20                  25                  30

Ala Arg Trp Ala Leu Thr Cys Cys Leu Val Leu Leu Pro Phe Leu Ala
        35                  40                  45

Gly Leu Thr Thr Tyr Leu Leu Val Ser Gln Leu Arg Ala Gln Gly Glu
    50                  55                  60

Ala Cys Val Gln Phe Gln Ala Leu Lys Gly Gln Glu Phe Ala Pro Ser
65                  70                  75                  80

His Gln Gln Val Tyr Ala Pro Leu Arg Ala Asp Gly Asp Lys Pro Arg
                85                  90                  95

Ala His Leu Thr Val Val Arg Gln Thr Pro Thr Gln His Phe Lys Asn
            100                 105                 110

Gln Phe Pro Ala Leu His Trp Glu His Glu Leu Gly Leu Ala Phe Thr
        115                 120                 125

Lys Asn Arg Met Asn Tyr Thr Asn Lys Phe Leu Leu Ile Pro Glu Ser
    130                 135                 140

Gly Asp Tyr Phe Ile Tyr Ser Gln Val Thr Phe Arg Gly Met Thr Ser
145                 150                 155                 160

Glu Cys Ser Glu Ile Arg Gln Ala Gly Arg Pro Asn Lys Pro Asp Ser
                165                 170                 175

Ile Thr Val Val Ile Thr Lys Val Thr Asp Ser Tyr Pro Glu Pro Thr
            180                 185                 190

Gln Leu Leu Met Gly Thr Lys Ser Val Cys Glu Val Gly Ser Asn Trp
        195                 200                 205

Phe Gln Pro Ile Tyr Leu Gly Ala Met Phe Ser Leu Gln Glu Gly Asp
    210                 215                 220

Lys Leu Met Val Asn Val Ser Asp Ile Ser Leu Val Asp Tyr Thr Lys
225                 230                 235                 240

Glu Asp Lys Thr Phe Phe Gly Ala Phe Leu Leu
                245                 250


<210> 28
<211> 434
<212> DNA
<213> Homo sapiens

<220>
<221> misc_feature
<222> (15)
<223> n equals a, t, g or c

<220>
<221> misc_feature
<222> (19)

<223> n equals a, t, g or c

<220>
<221> misc_feature
<222> (133)
<223> n equals a, t, g or c

<220>
<221> misc_feature
<222> (388)
<223> n equals a, t, g or c

<220>
<221> misc_feature
<222> (424)
<223> n equals a, t, g or c

<400> 28
tctacacaag gtacngacng ctaccctgag ccaacccagc tcctcatggg gaccaagtct 60

gtatgcgaag taggtagcaa ctggttccag cccatctacc tcggagccat gttctccttg 120

caagaagggg acnagctaat ggtgaacgtc agtgacatct ctttggtgga ttacacaaaa 180

gaagataaaa ccttctttgg agccttctta ctataggagg agagcaaata tcattatatg 240

aaagtcctct gccaccgagt tcctaatttt ctttgttcaa atgtaattat aaccaggggt 300

tttcttgggg ccgggagtag ggggcattcc cacagggaca acggtttagc tatgaaattt 360

ggggggccca aaatttcaca acttcatngt tgcccttact tgatgagaag tacttaactt 420

gganaaaagg cttg 434


<210> 29
<211> 417
<212> DNA
<213> Homo sapiens

<220>
<221> misc_feature
<222> (4)
<223> n equals a, t, g or c

<220>
<221> misc_feature
<222> (8)
<223> n equals a, t, g or c

<220>
<221> misc_feature
<222> (17)
<223> n equals a, t, g or c

<220>
<221> misc_feature
<222> (24)
<223> n equals a, t, g or c

<220>
<221> misc_feature
<222> (28)

<223> n equals a, t, g or c

<220>
<221> misc_feature
<222> (31)
<223> n equals a, t, g or c

<220>
<221> misc_feature
<222> (35)
<223> n equals a, t, g or c

<220>
<221> misc_feature
<222> (41)
<223> n equals a, t, g or c

<220>
<221> misc_feature
<222> (43)
<223> n equals a, t, g or c

<220>
<221> misc_feature
<222> (46)
<223> n equals a, t, g or c

<220>
<221> misc_feature
<222> (48)
<223> n equals a, t, g or c

<220>
<221> misc_feature
<222> (50)
<223> n equals a, t, g or c

<220>
<221> misc_feature
<222> (53)
<223> n equals a, t, g or c

<220>
<221> misc_feature
<222> (55)
<223> n equals a, t, g or c

<220>
<221> misc_feature
<222> (61)
<223> n equals a, t, g or c

<220>
<221> misc_feature
<222> (63)
<223> n equals a, t, g or c

<220>
<221> misc_feature
<222> (66)
<223> n equals a, t, g or c

<220>

<221> misc_feature
<222> (202)
<223> n equals a, t, g or c

<220>
<221> misc_difference
<222> (209)
<223> n equals a, t, g or c

<220>
<221> misc_feature
<222> (282)
<223> n equals a, t, g or c

<220>
<221> misc_feature
<222> (306)
<223> n equals a, t, g or c

<220>
<221> misc_feature
<222> (321)
<223> n equals a, t, g or c

<220>
<221> misc_feature
<222> (344)
<223> n equals a, t, g or c

<220>
<221> misc_feature
<222> (346)
<223> n equals a, t, g or c

<220>
<221> misc_feature
<222> (380)
<223> n equals a, t, g or c

<220>
<221> misc_feature
<222> (395)
<223> n equals a, t, g or c

<220>
<221> misc_feature
<222> (405)
<223> n equals a, t, g or c

<400> 29
attncggnac gagcagnggc atgnccgngg nnctnggact nnnctntngn gananagcca 60

nnnttnnaat gctgccagag cacggcagct gcaggcccaa ggccaggagc agcagcgcac 120

gctgggctct cacctgctgc ctggtgttgc tccccttcct tgcaggactc accacatacc 180

tgcttgtcag ccagcttcgg gnccagggng aggcctgtgt gcagttccag ggtctaaaag 240

gacaggagtt tgcaccttca catcagcaag tttatgcacc tnttagagca gacggagata 300

agccangggg acaactgaca nttgtgagac aaattccaca cagnanttta aaatcagttt 360

ccagttttga atggggacan nattaggctg gcttnacaag accgntggat tttacag 417

<210> 30
<211> 388
<212> DNA
<213> Homo sapiens

<220>
<221> misc_feature
<222> (11)
<223> n equals a, t, g or c

<220>
<221> misc_feature
<222> (46)
<223> n equals a, t, g or c

<220>
<221> misc_feature
<222> (50)
<223> n equals a, t, g or c

<220>
<221> misc_feature
<222> (81)
<223> n equals a, t, g or c

<220>
<221> misc_feature
<222> (138)
<223> n equals a, t, g or c

<220>
<221> misc_feature
<222> (155)
<223> n equals a, t, g or c

<220>
<221> misc_feature
<222> (182)
<223> n equals a, t, g or c

<220>
<221> misc_feature
<222> (188)
<223> n equals a, t, g or c

<220>
<221> misc_feature
<222> (269)
<223> n equals a, t, g or c

<220>
<221> misc_feature
<222> (317)
<223> n equals a, t, g or c

<220>
<221> misc_feature
<222> (322)
<223> n equals a, t, g or c

<220>

<221> misc_feature
<222> (358)
<223> n equals a, t, g or c

<220>
<221> misc_feature
<222> (363)
<223> n equals a, t, g or c

<220>
<221> misc_feature
<222> (375)
<223> n equals a, t, g or c

<400> 30
   ctgcactggg nncatgaact aggcctggcc ttcaccaaga accgantgan ctataccaac 60
   aaattcctgc tgatcccaga ntcgggagac tacttcattt actcccaggt cacattccgt 120
   gggaatgaac ctctgaantg ccagtgaaaa tcagncaagc aggccgacca aacaagccag 180
   antccatnca ctgtggtcat caccaaggta acagacagct accctgagcc aacccagctc 240
   cttcatgggg accaagtttg tttgcgaant aggttagcaa ctggttccag cccattttac 300
   cttgggggcc agttctnctt gncaagaagg ggacaagctt atggtggaac gttcatanca 360
   tcnttttggg gtggntttac acaaaagg 388

<210> 31
<211> 458
<212> DNA
<213> Homo sapiens

<220>
<221> misc_feature
<222> (9)
<223> n equals a, t, g or c

<220>
<221> misc_feature
<222> (12)
<223> n equals a, t, g or c

<220>
<221> misc_feature
<222> (119)
<223> n equals a, t, g or c

<220>
<221> misc_feature
<222> (303)
<223> n equals a, t, g or c

<220>
<221> misc_feature
<222> (311)
<223> n equals a, t, g or c

<220>
<221> misc_feature
<222> (387)

<223> n equals a, t, g or c

<220>
<221> misc_feature
<222> (409)
<223> n equals a, t, g or c

<220>
<221> misc_feature
<222> (425)
<223> n equals a, t, g or c

<220>
<221> misc_feature
<222> (427)
<223> n equals a, t, g or c

<220>
<221> misc_feature
<222> (453)
<223> n equals a, t, g or c

<400> 31
ggcacagcng gnagtagggg gcattccaca gggacaacgg tttagctatg aaatttgggg 60

cccaaaattt cacacttcat gtgccttact gatgagagta ctaactggaa aaaggctgna 120

agagagcaaa tatattatta agatgggttg gaggattggc gagtttctaa atattaagac 180

actggatcac tgaaatgaat ggatgatcta ctcgggtcca ggattgaaag agaaatattt 240

caacaccttc ctgctataca atggtcacca gtggtccagt tattgttcca atttggatcc 300

atnaatttgc nttcaattcc aggagctttg gaaggaattc caaggaaagc tccaggaaaa 360

ccgtattaaa ctttccaggg gccaaantcc ttcaccaatt ttttccacna actttccagg 420

cctgncncaa aaaaatggaa agggagttgg tangtccc 458